# EUROPEAN PATENT SPECIFICATION

(11) **EP 4 136 080 B1**
(45) Date of publication and mention of the grant of the patent: **24.04.2024**
(21) Application number: 21718845.7
(22) Date of filing: 12.04.2021
(51) Int. Cl.: C07D 257/06, C07D 271/113, C07D 401/12, C07D 403/12, C07D 409/12, C07D 413/12, C07D 417/12, A01N 43/713, A01N 43/78, A01N 43/80, A01N 43/82

(54) **HERBICIDAL COMPOUNDS**
HERBIZIDE VERBINDUNGEN
COMPOSÉS HERBICIDES

(30) Priority: 17.04.2020 IN 202011016632
(43) Date of publication of application: 22.02.2023
(73) Proprietor: Syngenta Crop Protection AG, 4058 Basel (CH)
(72) Inventor: BURTON, Paul, Matthew, Bracknell Berkshire RG42 6EY (GB); MITCHELL, Glynn, Bracknell Berkshire RG42 6EY (GB); RAJAN, Ramya, Corlim IIhas Goa 403 110 (IN); EMERY, Katie, Bracknell Berkshire RG42 6EY (GB); TAYLOR, Nicholas, John, Bracknell Berkshire RG42 6EY (GB)
(74) Representative: SYNGENTA IP
(86) International application number: PCT/EP2021/059431
(87) International publication number: WO 2021/209383

(56) References cited:
- WO-A1-2012/028579
- WO-A1-2012/126932
- WO-A1-2012/130685
- WO-A1-2013/087577
- WO-A1-2013/092834
- WO-A1-2014/184015
- WO-A1-2015/052153
- WO-A1-2018/234371
- WO-A2-2013/083859
- US-A1- 2014 080 705
- US-A1- 2016 235 067
- DATABASE CAPLUS [Online] 10 February 2006 (2006-02-10), ---: "List of compounds falling within the scope of claims 16 and 17", XP55800268, Database accession no. CAS registry numbers 873984-65-7, 873980-66-6, 873

## Description

The present invention relates to novel herbicidal compounds, to processes for their preparation, to herbicidal compositions which comprise the novel compounds, and to their use for controlling weeds, in particular in crops of useful plants, or for inhibiting plant growth.

N-(tetrazol-5-yl)- and N-(1,3,4-oxadiazol-2-yl) arylcarboxamides are disclosed in, for example, WO2012/028579 and WO2012/126932 respectively. The present invention relates to novel arylcarboxamides.

Thus, according to the present invention there is provided a compound of Formula (I): 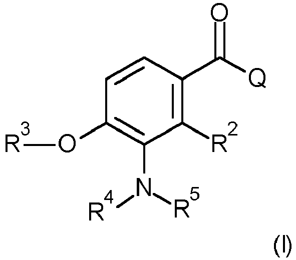
or an agronomically acceptable salt thereof,
wherein: -
Q is Q¹ or Q²; 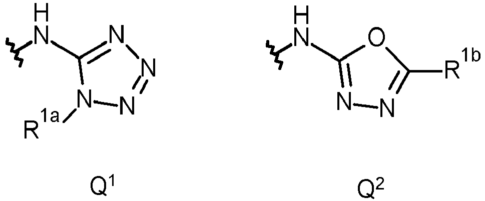
R^{1a} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R^{1b} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is C₁-C₆haloalkyl or C₁-C₆alkyl;
R⁴ is selected from the group consisting of C₁-C₆alkyl-, C₁-C₆ haloalkyl-, C₁-C₆alkyl-C(O)-, C₁-C₆haloalkyl-C(O)-, C₃-C₆cycloalkyl-, C₃-C₆cycloalkyl-C₁-C₃alkyl-, C₃-C₆cycloalkyl-C(O)-, C₁-C₃alkoxy-C₁-C₃alkyl-, C₁-C₃alkoxy-C₁-C₃alkyl-C(O)-, -C(O)-phenyl and -C(O)-heteroaryl wherein the phenyl, heteroaryl or C₃-C₆cycloalkyl is optionally substituted by 1, 2 or 3 substituents selected from the group consisting of halogen, C₁-C₆ alkyl (e.g methyl), C₁-C₆ haloalkyl and C₁-C₆ alkoxy;
R⁵ is selected from the group consisting of hydrogen, C₁-C₆alkyl-, C₁-C₆haloalkyl and C₁-C₆cycloalkyl; or
R⁴ and R⁵ together with the nitrogen atom to which they are attached form a 5- or 6-membered saturated heterocycle which is optionally oxo substituted; and
p is 0, 1 or 2.
C₁-C₆alkyl and C₁-C₄alkyl groups include, for example, methyl (Me, CH₃), ethyl (Et, C₂H₅), *n*-propyl (*n*-Pr), isopropyl (*i*-Pr), *n*-butyl (*n*-Bu), isobutyl (*i*-Bu), *sec*-butyl and *tert-*butyl (*t*-Bu).
C₃-C₆cycloalkyl- includes cyclopropyl (*c*-propyl (*c*-Pr)), cyclobutyl (*c*-butyl (*c*-Bu)), cyclopentyl (*c*-pentyl) and cyclohexyl (*c*-hexyl).

Halogen (or halo) encompasses fluorine, chlorine, bromine or iodine. The same correspondingly applies to halogen in the context of other definitions, such as haloalkyl.

C₁-C₆haloalkyl includes, for example, fluoromethyl-, difluoromethyl-, trifluoromethyl-, chloromethyl-, dichloromethyl-, trichloromethyl-, 2,2,2-trifluoroethyl-, 2,2-difluoroethyl, 1,1-difluoroethyl, 1,1,2,2-tetrafluoroethyl, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl-, heptafluoro-n-propyl and perfluoro-n-hexyl. C₁-C₄haloalkyl includes, for example, fluoromethyl-, difluoromethyl-, trifluoromethyl-, chloromethyl-, dichloromethyl-, trichloromethyl-, 2,2,2-trifluoroethyl-, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl- and heptafluoro-n-propyl-.

C₁-C₆alkyl-S- (alkylthio) is, for example, methylthio, ethylthio, propylthio, isopropylthio, n-butylthio, isobutylthio, sec-butylthio or tert-butylthio, preferably methylthio or ethylthio.

C₁-C₆alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₆alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In a preferred embodiment of the present invention, R^{1a} and R^{1b} are selected from the group consisting of methyl, ethyl and *n*-propyl.

In another embodiment of the present invention, Q is Q¹. Thus, in this embodiment the compound of Formula (I) is a compound of Formula (Ia): 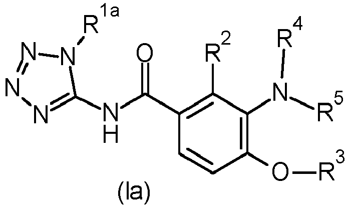 wherein R^{1a}, R², R³, R⁴ and R⁵ are as defined with regard to a compound of Formula (I).

In another embodiment of the present invention, Q is Q². Thus, in this particular embodiment of the present invention there is provided a compound of Formula (Ib) 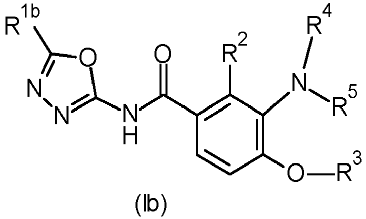 wherein R^{1b}, R², R³, R⁴ and R⁵ are as defined with regard to a compound of Formula (I).

In a preferred embodiment of the present invention, R² is selected from the group consisting of methyl, Cl, -CF₃ and -SO₂methyl, more preferably Cl.

In another preferred embodiment of the present invention, R³ is selected from the group consisting of -CH₃, -CF₃, -CHF₂ and -CF₂CF₂H, more preferably -CF₃ or -CHF₂.

In one embodiment of the present invention, R⁴ is -C(O)-heteroaryl wherein the heteroaryl optionally substituted as previously described and is selected from the group consisting of R^{4a}, R^{4b}, R^{4c}, R^{4d}, R^{4e}, R^{4f}, R^{4g} and R^{4h}:

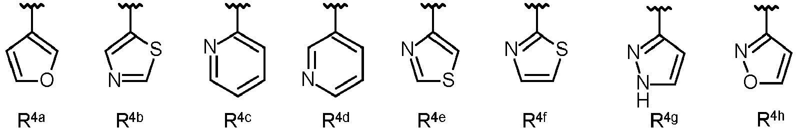

In a preferred embodiment of the present invention, the heteroaryl is R^{4c} which is optionally substituted by 1, 2 or 3 substituents selected from the group consisting of halogen, C₁-C₆ alkyl (e.g methyl), C₁-C₆ haloalkyl and C₁-C₆ alkoxy. In still more preferred embodiment of the present invention, the heteroaryl is R^{4c} which is optionally substituted by one halogen, preferably fluorine.

In another embodiment of the present invention R⁴ is selected from the group consisting of C₁-C₆alkyl- (preferably methyl), C₁-C₆alkyl-C(O)- (preferably CH₃CH₂C(O)-) and C₃-C₆cycloalkyl-C(O)- (preferably *c*Pr-C(O)-).

In another embodiment of the present invention R⁵ is hydrogen or C₁-C₆alkyl- (preferably methyl), most preferably hydrogen.

In one embodiment of the present invention, R⁴ is methyl or CH₃CH₂C(O)- and R⁵ is hydrogen. In another embodiment of the present invention, R⁴ is -C(O)-heteroaryl wherein the heteroaryl is is R^{4c} which is optionally substituted by one halogen, preferably fluorine and R⁵ is hydrogen.

In another embodiment of the present invention R⁴ and R⁵ taken together form a 5- or 6-membered saturated heterocycle which is optionally oxo substituted selected from the group consisting of -C(O)-CH₂CH₂CH₂CH₂-, -CH₂CH₂OCH₂CH₂-, -C(O)CH₂CH₂CH₂-, - CH₂CH₂CH₂CH₂CH₂- and -CH₂CH₂CH₂CH₂-, preferably -C(O)-CH₂CH₂CH₂CH₂-.

Compounds of Formula (I) (and certain intermediate compounds used to synthesise compound of Formula (I)) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The present invention also includes all possible geometric and tautomeric forms of a compound of formula (I).

The present invention also includes agronomically acceptable salts that the compounds of Formula (I) may form with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used as salt formers, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used.

The compounds of Formula (I) according to the invention can be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound of the present invention and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

The herbicidal compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, bensulfuron (including bensulfuron-methyl), bentazone, bicyclopyrone, bilanafos, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, diquat dibromide, diuron, epyrifenacil, ethalfluralin, ethofumesate, fenoxaprop (including fenoxaprop-P-ethyl), fenoxasulfone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including the ammonium salt thereof), glyphosate (including the diammonium, isopropylammonium and potassium salts thereof), halauxifen (including halauxifen-methyl), haloxyfop (including haloxyfop-methyl), hexazinone, hydantocidin, imazamox, imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 3-[2-(3,4-dimethoxyphenyl)-6-methyl-3-oxo-pyridazine-4-carbonyl]bicyclo[3.2.1]octane-2,4-dione, 2-[2-(3,4-dimethoxyphenyl)-6-methyl-3-oxo-pyridazine-4-carbonyl]-5-methyl-cyclohexane-1,3-dione, 2-[2-(3,4-dimethoxyphenyl)-6-methyl-3-oxo-pyridazine-4-carbonyl]cyclohexane-1,3-dione, 2-[2-(3,4-dimethoxyphenyl)-6-methyl-3-oxo-pyridazine-4-carbonyl]-5,5-dimethyl-cyclohexane-1,3-dione, 6-[2-(3,4-dimethoxyphenyl)-6-methyl-3-oxo-pyridazine-4-carbonyl]-2,2,4,4-tetramethyl-cyclohexane-1,3,5-trione, 2-[2-(3,4-dimethoxyphenyl)-6-methyl-3-oxo-pyridazine-4-carbonyl]-5-ethyl-cyclohexane-1,3-dione, 2-[2-(3,4-dimethoxyphenyl)-6-methyl-3-oxo-pyridazine-4-carbonyl]-4,4,6,6-tetramethyl-cyclohexane-1,3-dione, 2-[6-cyclopropyl-2-(3,4-dimethoxyphenyl)-3-oxo-pyridazine-4-carbonyl]-5-methyl-cyclohexane-1,3-dione, 3-[6-cyclopropyl-2-(3,4-dimethoxyphenyl)-3-oxo-pyridazine-4-carbonyl]bicyclo[3.2.1]octane-2,4-dione, 2-[6-cyclopropyl-2-(3,4-dimethoxyphenyl)-3-oxo-pyridazine-4-carbonyl]-5,5-dimethyl-cyclohexane-1,3-dione, 6-[6-cyclopropyl-2-(3,4-dimethoxyphenyl)-3-oxo-pyridazine-4-carbonyl]-2,2,4,4-tetramethyl-cyclohexane-1,3,5-trione, 2-[6-cyclopropyl-2-(3,4-dimethoxyphenyl)-3-oxo-pyridazine-4-carbonyl]cyclohexane-1,3-dione, 4-[2-(3,4-dimethoxyphenyl)-6-methyl-3-oxo-pyridazine-4-carbonyl]-2,2,6,6-tetramethyl-tetrahydropyran-3,5-dione, 4-[6-cyclopropyl-2-(3,4-dimethoxyphenyl)-3-oxo-pyridazine-4-carbonyl]-2,2,6,6-tetramethyl-tetrahydropyran-3,5-dione, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide and ethyl 2-[[3-[[3-chloro-5-fluoro-6-[3-methyl-2,6-dioxo-4-(trifluoromethyl)pyrimidin-1-yl]-2-pyridyl]oxy]acetate.

The mixing partners of the compound of Formula I may also be in the form of esters or salts, as mentioned e.g. in The Pesticide Manual, Sixteenth Edition, British Crop Protection Council, 2012.

The compound of Formula I can also be used in mixtures with other agrochemicals such as fungicides, nematicides or insecticides, examples of which are given in The Pesticide Manual.

The mixing ratio of the compound of Formula I to the mixing partner is preferably from 1: 100 to 1000: 1.

The mixtures can advantageously be used in the above-mentioned formulations (in which case "active ingredient" relates to the respective mixture of compound of Formula I with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil. Particularly preferred are mixtures of a compound of Formula I with cyprosulfamide, isoxadifen-ethyl, cloquintocet-mexyl and/or metcamifen.

The safeners of the compound of Formula I may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of Formula I to safener is from 100:1 to 1: 10, especially from 20:1 to 1:1.

The mixtures can advantageously be used in the above-mentioned formulations (in which case "active ingredient" relates to the respective mixture of compound of Formula I with the safener).

The present invention still further provides a method of controlling weeds at a locus said method comprising application to the locus of a weed controlling amount of a composition comprising a compound of Formula (I). Moreover, the present invention further provides a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. Generally, the plants to be controlled are unwanted plants (weeds). 'Locus' means the area in which the plants are growing or will grow. Some crop plants may be inherently tolerant to herbicidal effects of compounds of Formula (I). However, in some instances tolerance may need to be engineered into the crop plant, for example by way of genetic engineering. Thus, it is possible that the crop plant is rendered tolerant to HPPD-inhibitors via genetic engineering. Methods of rending crop plants tolerant to HPPD-inhibitors are known, for example from WO0246387. Thus in an even more preferred embodiment the crop plant is transgenic in respect of a polynucleotide comprising a DNA sequence which encodes an HPPD-inhibitor resistant HPPD enzyme derived from a bacterium, more particularly from *Pseudomonas fluorescens* or *Shewanella colwelliana,* or from a plant, more particularly, derived from a monocot plant or, yet more particularly, from a barley, maize, wheat, rice, *Brachiaria, Cenchrus, Lolium, Festuca, Setaria, Eleusine, Sorghum* or *Avena* species. Several HPPD-tolerant soybean transgenic "events" are known and include for example SYHT04R (WO2012/082542), SYHT0H2 (WO2012/082548) and FG72. Other polynucleotide sequences that can be used to provide plants which are tolerant to the compounds of the present invention are disclosed in, for example, WO2010/085705 and WO2011/068567. Crop plants in which the composition according to the invention can be used thus include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to herbicides or classes of herbicides (e.g. ALS-, GS-, EPSPS-, PPO-, ACCase-and HPPD-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} andProtexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled may be both monocotyledonous species, for example Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum, and dicotyledonous species, for example Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium. Weeds can also include plants which may be considered crop plants but which are growing outside a crop area ('escapes'), or which grow from seed left over from a previous planting of a different crop (`volunteers'). Such volunteers or escapes may be tolerant to certain other herbicides.

The compounds of the present invention can be prepared according to the following schemes.

Compounds of formula (I) may be prepared from compounds of formula (II).

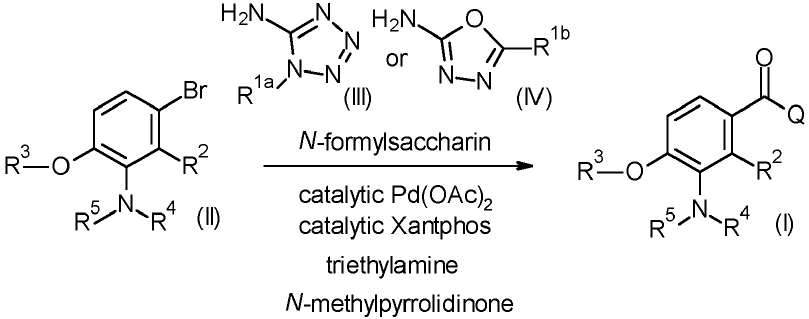

The compound of formula (II) is treated with an amine of formula (III) (for embodiments of the invention where Q=Q1) or an amine of formula (IV) (for embodiments of the invention where Q=Q2) and N-formylsaccharin and triethylamine with N-methylpyrrolidinone as a solvent with a palladium (II) acetate catalyst and a Xantphos ligand. The reaction may be performed using a continuous flow reactor.

Alternatively, compounds of formula (I) maybe prepared from benzoic acids of formula (V).

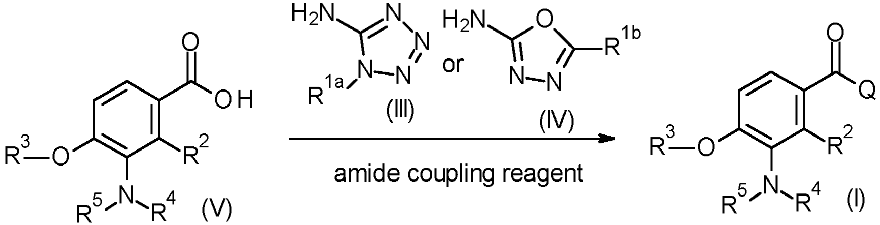

The benzoic acid of formula (V) and an amine of formula (III) (for embodiments of the invention where Q=Q1) or an amine of formula (IV) (for embodiments of the invention where Q=Q²) are treated with an amide coupling reagent, for example thionyl chloride and *N*-methylimidazole, in a suitable solvent, for example pyridine.

Compounds of formula (V) may be prepared by hydrolysis of esters of formula (VI).

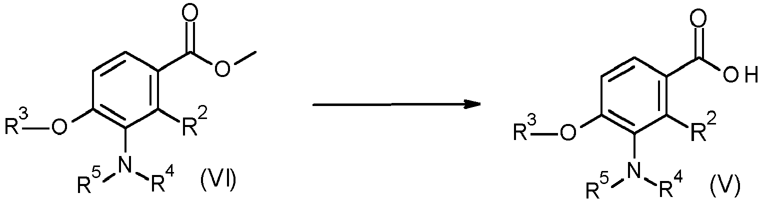

The ester of formula (VI) is treated with sodium hydroxide in a suitable solvent, for example a 3:1 mixture of ethanol : water, to give the compound of formula (V).

Where R² is not chloro, compounds of formula (VI) may be prepared from compounds of formula (VI) where R² is chloro. 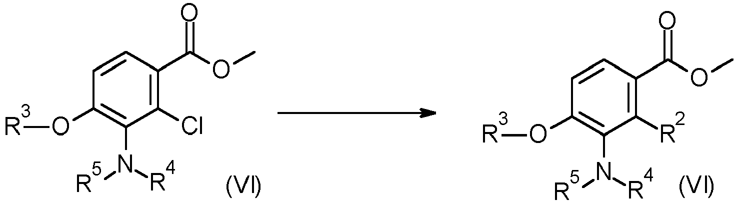 where R2 = Cl

The method of the transformation will depend on the identity of R². The skilled person will be familiar with methods to convert the chloro group to the R² group. For example, where R² is methyl, a Suzuki reaction is performed where the compound of R²=Cl is treated with trimethylboroxine and a base, for example potassium carbonate, with a suitable catalyst, for example [1,3-Bis(2,6-Diisopropylphenyl)imidazol-2-ylidene](3-chloropyridyl)palladium(II) dichloride.

Compounds of formula (VI) where R²=Cl may be prepared from compounds of formula (II) where R²=Cl.

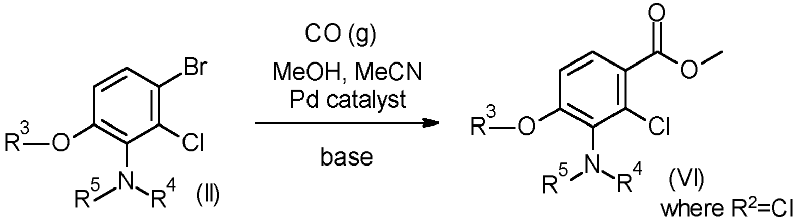

The compound of formula (II) is reacted in an autoclave with a carbon monoxide atmosphere with a base, for example triethylamine, and a palladium catalyst, for example [1,1'-Bis(diphenylphosphino)ferrocene]palladium(II) dichloride, in a mixed solvent of methanol and acetonitrile.

Where R⁵ is not hydrogen, compounds of formula (II) where R²=Cl may be prepared from compounds of formula (VII) and compounds of formula (VII).

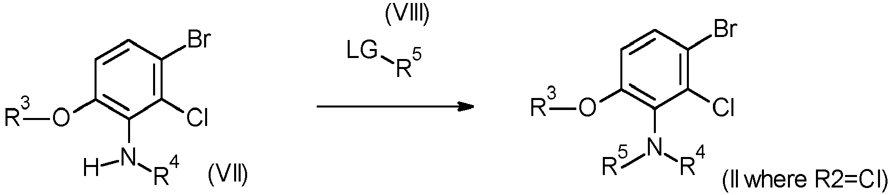

The compound of formula (VII) is treated with a compound of formula (VIII), where LG is defined as a leaving group, in a suitable solvent. A base may optionally be required for this reaction. The requirement and choice of base will be familiar to the skilled person. For example, where R⁵ is acetyl, the compound of formula (VIII) is acetyl chloride. For example, where R⁵ is methyl, the compound of formula (VIII) is iodomethane and base is sodium hydride.

Compounds of formula (VII) may be prepared from compounds of formula (IX) and a compound of formula (X).

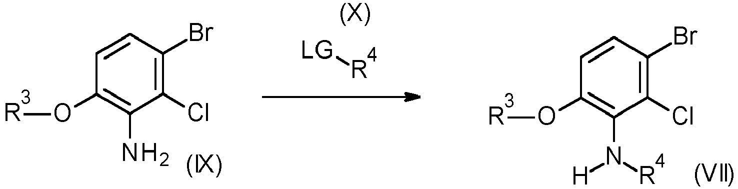

The compound of formula (IX) is treated with a compound of formula (X), where LG is defined as a leaving group, in a suitable solvent. A base may optionally be required for this reaction. The requirement and choice of base will be familiar to the skilled person. For example, where R4 is acetyl, the compound of formula (X) is acetyl chloride. For example, where R⁵ is methyl, the compound of formula (X) is iodomethane and base is sodium hydride.

Compounds of formula (IX) may be prepared from compounds of formula (XI).

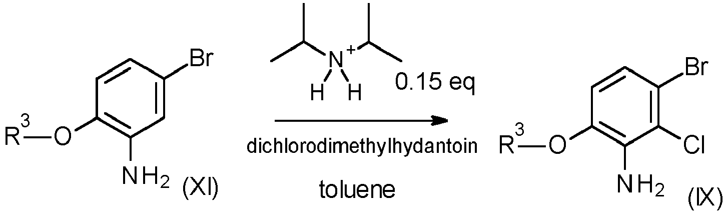

The compound of formula (XI) is treated with dichlorodimethylhydantoin with a catalytic amount of isopropylammonium chloride with toluene as a solvent to give the compound of formula (IX). Compounds of formula (XI) may be commercially available. Alternatively, they may be prepared from 2-amino-4-bromo-phenol.

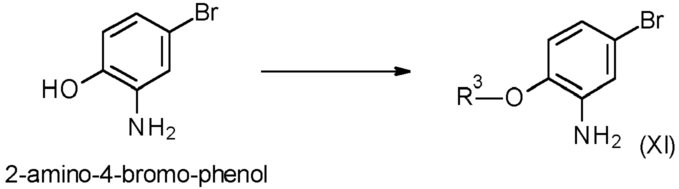

The method of transformation will depend on the nature of R³. For example, where R³ is -CH₂CF₃, 2-amino-4-bromo-phenol is treated with potassium carbonate and 2,2,2-trifluoroethyltriflate. For example, where R³ is -CF₂H, 2-amino-4-bromo-phenol is treated with sodium bromodifluoroacetate and a base, for example caesium carbonate.

Thus, according to the present invention there is further provided a compound of Formula (II) 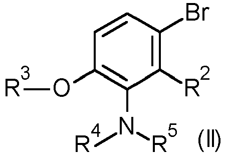 wherein R², R⁴ and R⁵ are as defined in the compound of Formula (I) above and R³ is C₁-C₆haloalkyl. In a preferred embodiment R² is Cl and R³ is -CF₃ or -CHF₂.

The present invention further provides a compound of Formula (V) 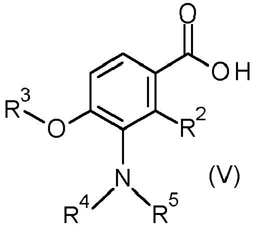 wherein R², R⁴ and R⁵ are as defined in the compound of Formula (I) above and R³ is C₁-C₆haloalkyl. In a preferred embodiment R² is Cl and R³ is -CF₃ or -CHF₂.

The present invention still further provides a compound of Formula (VIa) 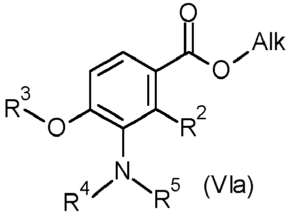 wherein "Alk" is C₁-C₆ alkyl (preferably methyl) and wherein R², R³, R⁴ and R⁵ are as defined in the compound of Formula (I) above. In a preferred embodiment R² is Cl and R³ is -CF₃ or -CHF₂.

The following non-limiting examples provide specific synthesis methods for representative compounds of the present invention, as referred to the Tables provided herein.

### Preparative Example 1: Compound 1.004

### Step 1.

To a flask containing 5-bromo-2-(trifluoromethoxy)aniline (10 g, 39.1 mmol) was added toluene (100 mL) and diisopropylammonium chloride (1.08 g, 7.81 mmol). The reaction mixture was covered in foil to remove light. At 0 °C, 1,3-dichloro-5,5-dimethyl-imidazolidine-2,4-dione (7.70 g, 39.1 mmol) was added and the reaction mixture was allowed to warm to room temperature and was stirred for 3 hours. The reaction mixture was quenched by the addition of saturated aqueous sodium bisulfite, then diluted with water and ethyl acetate, and the phases were separated. The organic phase was dried and concentrated *in vacuo.* The crude material was purified by normal phase flash chromatography (0 to 5 % ethyl acetate in cyclohexane) to give 3-bromo-2-chloro-6-(trifluoromethoxy)aniline (7.07 g, 21.9 mmol, 56%) as a pale yellow oil. ¹H NMR (Methanol): 7.05 (m, 1H), 6.99 (d, 1H).

### Step 2.

To a flask containing 3-bromo-2-chloro-6-(trifluoromethoxy)aniline (1.00 g, 3.40 mmol) was added acetonitrile (20 mL) and the reaction mixture was put under a nitrogen atmosphere. 5-Bromopentanoyl chloride (1.10 g, 0.79 mL, 5.90 mmol) was added. The reaction mixture was stirred at 50 °C for 6 hours. The reaction mixture was quenched by the addition of water and was concentrated *in vacuo* to remove the acetonitrile solvent. The reaction residue was taken up in ethyl acetate and water and the phases were separated. The aqueous phase was further extracted with ethyl acetate. The organic phases were combined, dried and concentrated *in vacuo.* The crude material was purified by normal phase flash chromatography (0 to 30 % ethyl acetate in cyclohexane) to give impure product as a pale yellow solid. The crude material was taken up in ethyl acetate and washed with aqueous 2M sodium hydroxide solution. The organic phase was dried and concentrated *in vacuo* to give 5-bromo-N-[3-bromo-2-chloro-6-(trifluoromethoxy)phenyl]pentanamide (1.44 g, 2.92 mmol, 85%) as a pale yellow solid. ¹H NMR (chloroform): 7.62 (d, 1H), 7.16 (m, 1H), 6.90 (br s, 1H), 3.45 (t, 2H), 2.47 (br s, 2H), 2.02 - 1.87 (m, 4H).

### Step 3.

To a flask containing N-[3-bromo-2-chloro-6-(trifluoromethoxy)phenyl]acetamide (1.44 g, 2.92 mmol) was added tetrahydrofuran (14 mL). The reaction mixture was stirred at 0 °C under nitrogen atmosphere for 10 min. To the reaction mixture was added sodium hydride in paraffin oil (60 mass%, 0.129 g, 3.21 mmol). The reaction mixture was stirred at room temperature for 4.5 hours. To the reaction mixture was added more sodium hydride in paraffin oil (60 mass%, 0.0818 g, 2.04 mmol). The reaction mixture was stirred at room temperature for 2 hours. The reaction mixture was quenched by the addition of water and was concentrated *in vacuo* to remove the tetrahydrofuran solvent. The residue was taken up in ethyl acetate and water, and the phases were separated. The aqueous phase was further extracted with ethyl acetate. The organic phases were combined, dried and concentrated *in vacuo.* The crude material was purified by normal phase flash chromatography (0 to 25 % ethyl acetate in cyclohexane) to give 1-[3-bromo-2-chloro-6-(trifluoromethoxy)phenyl]piperidin-2-one (1.03 g, 2.43 mmol, 83%) as a colourless oil. ¹H NMR (chloroform): 7.64 (d, 1H), 7.17 (dq, 1H), 3.64 - 3.50 (m, 1H), 3.49 - 3.38 (m, 1H), 2.58 (dt, 2H), 2.07 - 1.88 (m, 4H).

### Step 4.

To a vessel containing 1-[3-bromo-2-chloro-6-(trifluoromethoxy)phenyl]piperidin-2-one (0.500 g, 1.34 mmol) was added palladium(II) acetate (0.0301 g, 0.134 mmol), XantPhos (0.160 g, 0.268 mmol), N-formylsaccharine (0.638 g, 3.02 mmol), 1-methyltetrazol-5-amine (1.20 g, 12.1 mmol) and 1-methyl-2-pyrrolidinone was added so the total volume of the vessel was 20 mL. To a second vessel containing triethylamine (0.611 g, 6.04 mmol, 0.842 mL) was added 1-methyl-2-pyrrolidinone so that the total volume of the vessel was 20 mL. The two solutions were injected into the sample loops pumped through a T-piece and then round a 20 mL stainless steel coil heated to 170 °C. The flow rate was set so that the total residence time was 15 mins. The reaction mixture was concentrated *in vacuo* to remove the solvent 1-methyl-2-pyrrolidinone. The residue was taken up in dichloromethane and saturated aqueous sodium carbonate, and the phases were separated. The aqueous phase was further extracted with dichloromethane. The aqueous phase was acidified to pH 5 and was extracted with ethyl acetate. The organic phases were combined, dried and concentrated *in vacuo.* The crude material was purified by normal phase flash chromatography (0 to 10 % dichloromethane in methanol) to give impure product as a glassy solid. The crude material was taken up in ethyl acetate and washed dilute aqueous HCl. The organic phase was dried and concentrated *in vacuo* to give 3-acetamido-2-chloro-N-(5-methyl-1,3,4-oxadiazol-2-yl)-4-(trifluoromethoxy)benzamide (Compound 1.004) (0.0486 g, 0.116 mmol, 9%) as a foaming white solid. ¹H NMR (Methanol): 7.82 (d, 1H), 7.58 (m, 1H), 4.06 (s, 3H), 3.69 - 3.60 (m, 1H), 3.59 - 3.50 (m, 1H), 2.57 (m, 2H), 2.10 - 1.93 (dt, 4H)

### Preparative Example 2: Compound 1.006

### 3-bromo-2-chloro-6-(trifluoromethoxy)aniline was prepared as described previously.

### Step 1

An autoclave was charged with 3-bromo-2-chloro-6-(trifluoromethoxy)aniline (24 g, 76 mmol). Methanol (144 mL) was added. Triethylamine (23 g, 228 mmol) and then allylpalladium(II) chloride dimer (1.13 g, 3.10 mmol) and 2,2'-bis(diphenylphosphino)-1,1'-binaphthyl (2.44 g, 3.92 mmol) were added. The autoclave was flushed with nitrogen x 3 and then with carbon monoxide x 3. The reaction was pressurized to 20 bar CO and heated at 100°C for 4 h. It was then cooled to RT and the atmosphere replaced with N₂. The contents were discharged to a conical flask and the reaction mixture was filtered through celite and evaporated. Column chromatography gave methyl 3-amino-2-chloro-4-(trifluoromethoxy)benzoate (16 g, 59 mmol, 78%) as a white solid.

### Step 2

To a flask containing methyl 3-amino-2-chloro-4-(trifluoromethoxy)benzoate (2.08 g, 7.70 mmol) was added acetonitrile (60 mL) and cyclopropanecarbonyl chloride (2.41 g, 23.1 mmol). The reaction mixture was stirred at 60°C. After the addition a white solid was formed that required vigorous stirring. After stirring for 2 h, the reaction mixture was cooled and the solvent was evaporated. The solid obtained was stirred was stirred in cyclohexane (100 ml) and filtered, then washed with cyclohexane to give methyl 2-chloro-3-(cyclopropanecarbonylamino)-4-(trifluoromethoxy)benzoate (2.40 g, 7.12 mmol, 92%) as white solid.

### Step 3

To a solution of methyl 2-chloro-3-(cyclopropanecarbonylamino)-4-(trifluoromethoxy)benzoate (A, 0.35 g, 1.04 mmol) in THF (9 mL) and water (3 mL). Lithium hydroxide hydrate (87 mg, 2.1 mmol) was added and the reaction mixture was stirred for 16 h. The reaction was concentrated to remove THF. 2 M HCl was added to the solution until a white solid precipitated, which was isolated by filtration to give 2-chloro-3-(cyclopropanecarbonylamino)-4-(trifluoromethoxy)benzoic acid (0.296 g, 0.915 mmol, 88%) as a white solid. 1H NMR (400 MHz, methanol) δ ppm 7.87 (d, 1 H) 7.43 (d, 1H) 1.85 - 1.96 (m, 1 H) 0.84 - 1.02 (m, 4 H).

### Step 4

2-chloro-3-(cyclopropanecarbonylamino)-4-(trifluoromethoxy)benzoic acid (1.10 g, 3.4 mmol), 1-methyltetrazol-5-amine (400 mg, 4.1 mmol) in 2-methylpyridine (8 mL) was stirred for 10 min under a nitrogen atmosphere, then 1-methylimidazole (280 mg, 3.4 mmol) was added followed by triethylamine (520 mg, 5.1 mmol) and stirred at RT for 10 min. The reaction mass was then cooled to 0 °C and thionyl chloride (810 mg, 6.8 mmol) was added dropwise. The reaction mixture was stirred RT for 16 h. The reaction was diluted with 2N HCl and stirred for 30 min. A solid obtained was filtered, washed with ethanol and dried to obtain 2-chloro-3-(cyclopropanecarbonylamino)-N-(1-methyltetrazol-5-yl)-4-(trifluoromethoxy)benzamide as a white solid (820 mg, 2.08 mmol, 61%). 1H NMR (Methanol): 7.73 (d, 1H), 7.52 (br d, 1H), 4.06 (s, 3H), 1.91 (m, 1H), 1.01-0.88 (m, 4H).

### Preparative Example 3: Compound 1.007

Methyl 3-amino-2-chloro-4-(trifluoromethoxy)benzoate was prepared as described previously.

### Step 1

To a solution of methyl 3-amino-2-chloro-4-(trifluoromethoxy)benzoate (2.5 g, 9.3 mmol) in acetonitrile (60 mL) was added propanoyl chloride (2.6 g, 28 mmol). The reaction mixture was stirred at 60 °C for 2 h. After 2 h, the mixture was cooled to room temperature and the acetonitrile was removed under reduced pressure. The residue was taken up in ethyl acetate, then washed with sodium bicarbonate solution, dried (MgSO₄) and concentrated under reduced pressure. Flash chromatography (0-30% EtOAc and cyclohexane) gave methyl 2-chloro-3-(propanoylamino)-4-(trifluoromethoxy)benzoate (2.28 g, 7.00 mmol, 76%) as a white solid. 1H NMR (400 MHz, d4-methanol): 7.87 (d, 1 H) 7.45 (m, 1 H) 3.93 (s, 3 H) 2.46 (q, 2 H) 1.24 (t, 3 H).

### Step 2

To a stirred solution of methyl 2-chloro-3-(propanoylamino)-4-(trifluoromethoxy)benzoate (27.7 g, 85.1 mmol) in tetrahydrofuran (10 mL) and methanol (10 mL) was added lithium hydroxide (6.11 g, 255 mmol) in water (10 mL) and stirred for RT 12 hr. The reaction mass was concentrated under reduced pressure and partitioned between 1 N HCl and ethyl acetate. The organic layer was dried over sodium sulphate and concentrated to afford 2-chloro-3-(propanoylamino)-4-(trifluoromethoxy)benzoic acid (25.7 g, 78.3 mmol, 92%) as a white solid. 1H NMR (400 MHz, d6-DMSO): 13.70 (1H, s), 9.82 (1H, s), 7.79 (1H, d), 7.51 (1H, d), 2.35n (2H, q), 1.11 (H, t).

### Step 3

A flask was charged with pyridine (50 mL) and 2-chloro-3-(propanoylamino)-4-(trifluoromethoxy)benzoic acid (5.00 g, 16.0 mmol) under N2 atmosphere at RT. 1-Methyltetrazol-5-amine (1.79 g, 17.7 mmol) and 1-methylimidazole (1.33 g, 16.0 mmol) were then added and the reaction mixture was cooled to 0 °C. Thionyl chloride (3.94 g, 32.1 mmol) was added dropwise over 3 h maintaining the temperature at 0-10 °C using a syringe pump. After complete addition, the pH of the reaction mixture = 5.7 and a brown precipitate appeared. The ice bath was removed and reaction was allowed to stir at RT, After a further 2 h, the reaction mixture became brown solution. After stirring for a further 2 h, the reaction mixture was cooled to 0°C using an ice bath and then 25mL of water was added under stirring (pH = 5.8-5.9). The reaction mixture was acidified using 2 N HCl to pH=1.5-2. During acidification a sticky solid precipitated. The suspension was extracted with ethyl acetate (50 mL x 3). The combined organic layers were washed with cold water (25 mL), dried (Na2SO4) and concentrated under reduced pressure to get a light orange solid. This was purified by using chromatography (30-40% ethyl acetate in cyclohexane) to give 2-chloro-N-(1-methyltetrazol-5-yl)-3-(propanoylamino)-4-(trifluoromethoxy)benzamide (2.59 g, 6.61 mmol, 41%) as a white solid. 1H NMR (400 MHz, d6-DMSO): 11.92 (1H, s), 9.90 (1H, s), 7.80 (1H, d), 7.61 (1H, d), 4.00 (3H, s), 2.37 (2H, q), 1.12 (3H, t).

### Preparative Example 4: Compound 1.012

Methyl 3-amino-2-chloro-4-(trifluoromethoxy)benzoate was prepared as described previously.

### Step 1

Methyl 3-amino-2-chloro-4-(trifluoromethoxy)benzoate (2.0 g, 7.4 mmol) and pyridine-2-carboxylic acid (1.1 g, 8.9 mmol) was dissolved in pyridine (10 mL) The solution was cooled to 0°C by ice water. Phosphorous oxychloride (1.70 g, 11.1 mmol) was added dropwise over 4 min, and then the reaction was allowed to warm to RT and stirred for 3 h, whereupon a white precipitate appeared. The reaction mixture was quenched slowly into cold (0 °C) saturated aq sodium bicarbonate solution. After the addition, stirring was continued vigorously for 30 min. The white solid obtained was filtered and washed with water 3 times to give methyl 2-chloro-3-(pyridine-2-carbonylamino)-4-(trifluoromethoxy)benzoate (1.69 g, 4.51 mmol, 61%) as a white solid. 1H NMR (400 MHz, d6-DMSO): 10.69 (1H, s), 8.78 (1H, d), 8.15-8.06 (2H, m), 7.91 (1H, d), 7.73 (1H, ddd), 7.62 (1H, d), 3.90 (3H, s).

### Step 2

To a solution of methyl 2-chloro-3-(pyridine-2-carbonylamino)-4-(trifluoromethoxy)benzoate (1.6 g, 4.3 mmol) in tetrahydrofuran (32 mL), a solution of hydroxylithium hydrate (0.54 g, 13 mmol) in water (8.0 mL) was added. The reaction mixture was stirred at RT for 16 h. The reaction was concentrated to remove THF. The aqueous solution was washed with cyclohexane, then cooled to 0°C and adjusted pH to 3 with 10% aq. solution of Citric acid. The aqueous mixture was extracted with ethyl acetate x 3 and the combined organic layers were dried over sodium sulphate and concentrated under reduced pressure to give 2-chloro-3-(pyridine-2-carbonylamino)-4-(trifluoromethoxy)benzoic acid (1.6 g, 4.3 mmol, 100%) as a white solid. 1H NMR (400 MHz, d6-DMSO): 10.65 (1H, d), 8.77 (1H, d), 8.13 (1H, d), 8.05 (1H, t), 7.88 (1H, d), 7.69 (1H, m), 7.58 (1H, d).

### Step 3

2-chloro-3-(pyridine-2-carbonylamino)-4-(trifluoromethoxy)benzoic acid (1.5 g, 4.2 mmol), 1-methyltetrazol-5-amine (500 mg, 5.0 mmol) in 3-methylpyridine (15 mL) was stirred for 10 mins under a nitrogen atmosphere. Triethylamine (630 mg, 6.2 mmol) followed by 1-methylimidazole (340 mg, 4.2 mmol) was added and stirred at RT for 30 min. The reaction mass was then cooled to 0°C and thionyl chloride (990 mg, 8.3 mmol) was added dropwise. After stirring at RT for 16 h, the reaction mixture was quenched with 2N HCl to pH 1-2 at 0°C with vigorous stirring for 30 min. The aqueous layer was extracted with ethyl acetate x 3, then concentrated under reduced pressure. The solid was recrystallized from ethyl acetate / n-pentane to obtain N-[2-chloro-3-[(1-methyltetrazol-5-yl)carbamoyl]-6-(trifluoromethoxy)phenyl]pyridine-2-carboxamide (1.45 g, 3.17 mmol, 76%) as a white solid. 1H NMR (400 MHz, d6-DMSO): 12.00 (1H, brs), 10.77 (1H, s), 8.78 (1H, d), 8.16 - 8.05 (2H, m), 7.88 (1H, d), 7.72 (1H, ddd), 7.67 (1H, dd), 4.00 (3H, s).

### Preparative Example 5: Compound 1.014

### Step 1

To a 15 mL thick wall pressure vessel at room temperature, was added 2-amino-4-bromo-phenol (564 mg, 3.00 mmol), KOH (0.218 g, 3.90 mmol), DMSO (10 mL) and 1,2-dibromotetrafluoroethane (1.17 g, 4.50 mmol). The reaction flask was then closed and heated to 80°C for 18h. The reaction mixture was cooled to room temperature. The mixture is washed with H2O (200 mL) and extracted with ethyl acetate (100 mL x 3). The layers are separated and dried over sodium sulfate. After evaporation of the solvent, the residue was purified by chromatography (petroleum ether / ethyl acetate = 30:1) to afford 5-bromo-2-(2-bromo-1,1,2,2-tetrafluoro-ethoxy)aniline (0.215 g, 0.586 mmol, 19.5 %) as a brown oil and 5-bromo-2-(1,1,2,2-tetrafluoroethoxy)aniline (0.206 g, 0.715 mmol, yield: 23.8 %) as a brown oil. 1H NMR of 5-bromo-2-(1,1,2,2-tetrafluoroethoxy)aniline (400 MHz, d6-DMSO): 6.97 - 6.76 (3H, m), 6.65 - 6.62 (1H, m), 5.54 (2H, brs).

### Step 2

Diisopropylamine (0.973 g, 9.63 mmol) is added to a mixture of ammonium chloride (0.511 g, 9.63 mmol) and ethanol (25 mL). The mixture was heated to reflux for 4 h. The reaction mixture was cooled and then concentrated to give a white solid that was washed with further ethanol, then dried under vacuum. To a 3 necked flask under nitrogen, is added 5-bromo-2-(1,1,2,2-tetrafluoroethoxy)aniline (18.5 g, 64.2 mmol), toluene (300 mL) and diisopropylammonium;chloride (1.4 g). The flask was covered in foil, then cooled to 0 deg C. 1,3-dichloro-5,5-dimethyl-imidazolidine-2,4-dione (11.4 g, 57.8 mmol) was added portionwise and the mixture was stirred at 0 deg C for 2h. The reaction mixture was quenched by addition of saturated aqueous sodium bisulfite solution, then diluted with water and ethyl acetate. The residue was purified by chromatography (eluent: petroleum ether / ethyl acetate = 30:1) to afford 3-bromo-2-chloro-6-(1,1,2,2-tetrafluoroethoxy)aniline (9.00 g, 27.9 mmol, 43.5 %) as a yellow oil. 1H NMR (400 MHz, d6-DMSO): 7.03 (1H, d), 3.92 (1H, d), 6.85 (1H, tt), 5.83 (2H, brs).

### Step 3

Synthesis carried out in a Flow syn fitted with a 20ml stainless steel loop. To loop A was added: palladium(II) acetate (694 mg., 3.10 mmol), XantPhos (3.59 g, 6.20 mmol), N-formylsaccharin (14.7 g, 69.8 mmol) and 3-bromo-2-chloro-6-(1,1,2,2-tetrafluoroethoxy)aniline (10 g, 31.0 mmol) in 1-methyl-2-pyrrolidinone (190 mL). To loop B was added triethylamine (19.4 mL, 139.54 mmol) and 1-methyl-2-pyrrolidinone (190 mL) and water (20.1 mL, 1116.3 mmol). The temperature was set to 170 mins and the time set to 15 mins. In a fixed vessel, to the output reaction mixure is added water (1000ml) and 1M HCl (1000ml), followed by the addition of ethyl acetate (1000ml) was added and phases were separated. The organic layer was concentrated under reduced pressure. The crude material was purified by reversed phase chromatography: (50-70% MeCN in H2O gradient with 0.1% formic acid) to afford 3-amino-2-chloro-4-(1,1,2,2-tetrafluoroethoxy)benzoic acid (5.13 g, 17.8 mmol, 58%) as a white solid. 1H NMR (400 MHz, methanol) δ ppm 6.25 - 6.62 (m, 1 H) 7.01 - 7.26 (m, 2 H).

### Step 4

To a stirred suspension of 3-amino-2-chloro-4-(1,1,2,2-tetrafluoroethoxy)benzoic acid (5.13 g, 17.8 mmol) and 2,3,4,5,6-pentafluorophenol (3.61 g, 19.6 mmol) in dichloromethane (70 mL), at room temperature was added 3-(ethyliminomethyleneamino)-N,N-dimethyl-propan-1-amine hydrochloride (4.1 g, 21 mmol). The mixture was stirred at room temperature. Initially heterogeneous, within 5 minutes of adding EDC, the mixture was a homogeneous solution. The reaction mixture was stirred for 16 h at RT. The reaction was quenched by addition of sat. aq. NaHCO3 (100 mL). The mixture was stirred at room temperature for a further 5 min. The mixture filtered through a phase separation cartridge and the organics are collected. The filtrate was adsorbed onto silica and the crude product was purified by flash column chromatography (0-10% gradient of EtOAc in cyclohexane). Product-containing fractions were combined and concentrated in vacuo to afford (2,3,4,5,6-pentafluorophenyl) 3-amino-2-chloro-4-(1,1,2,2-tetrafluoroethoxy)benzoate (6.36 g, 14.0 mmol, 79%) as a colourless oil, which crystalised on standing. LCMS: M+H = 452.1 in ES-.

### Step 5

To a round bottom flask containing a solution of (2,3,4,5,6-pentafluorophenyl) 3-amino-2-chloro-4-(1,1,2,2-tetrafluoroethoxy)benzoate (3.18 g, 7.01 mmol) in acetonitrile (50 mL) at room temperature was added 1-methyltetrazol-5-amine (1.53 g, 15.4 mmol) followed by 2-tert-butylimino-N,N-diethyl-1,3-dimethyl-1,3,2λ⁵-diazaphosphinan-2-amine (4.4 g, 4.6 mL, 15 mmol). The mixture was stirred at room temperature overnight. The reaction was quenched by addition of 2 M aq. HCl (100 mL). The mixture was stirred at room temperature for a further 5 minutes. The mixture was transferred to a separating funnel and diluted with EtOAc (100 mL). The phases were separated. The aqueous phase was extracted with EtOAc (100 mL). The combined organic was adsorbed onto C18-silica and purified via reverse phase column chromatography (40-80% MeCN in H2O gradient with 0.1% formic acid) to give 3-amino-2-chloro-N-(1-methyltetrazol-5-yl)-4-(1,1,2,2-tetrafluoroethoxy)benzamide (1.88 g, 4.84 mmol, 69%) as a white solid. 1H NMR (400 MHz, methanol) δ ppm 4.05 (s, 3 H) 6.28 - 6.62 (m, 1 H) 6.91 - 6.99 (m, 1 H) 7.22 - 7.30 (m, 1 H).

### Step 6

To a stirred solution of 3-amino-2-chloro-N-(1-methyltetrazol-5-yl)-4-(1,1,2,2-tetrafluoroethoxy)benzamide (0.3 g, 0.81 mmol) in acetonitrile (6 mL) at room temperature was added propanoyl chloride ( 0.23 g, 0.22 mL, 2.5 mmol). The stirred mixture was heated to 60 °C overnight. The reaction was cooled to room temperature and quenched by slow addition of water (2 mL). The mixture was stirred at room temperature for a further 5 minutes. The mixture was transferred to a separating funnel and diluted with EtOAc (20 mL) and water (20 mL). The phases were separated. The aqueous phase was extracted with EtOAc (20 mL). The combined organic phases were dried (MgSO₄) and filtered. The filtrate was adsorbed onto C18-silica and purified via reverse phase column chromatography (30-60% MeCN in H₂O gradient with 0.1% formic acid) to give 2-chloro-N-(1-methyltetrazol-5-yl)-3-(propanoylamino)-4-(1,1,2,2-tetrafluoroethoxy)benzamide (280 mg, 0.626 mmol, 77%) as a white solid. 1H NMR (400 MHz, d4-methanol): 1.16 - 1.28 (m, 3H) 2.41 - 2.53 (m, 2H) 4.06 (s, 3H) 6.21 - 6.53 (m, 1H) 7.49 - 7.57 (m, 1H) 7.73 (d, 1H).

### Preparative Example 6: Compound 1.016

### Step 1.

To a flask containing 3-chloro-4-methyl-2-nitro-phenol (5.33 mmol, 1.00 g) was added acetone (20 mL), water (0.1 mL), potassium carbonate (10.7 mmol, 1.47 g) and 2,2,2-trifluoroethyl trifluoromethanesulfonate (8.00 mmol, 1.86 g). The reaction mixture was stirred at room temperature overnight. The reaction mixture was concentrated *in vacuo* to remove solvent. The residue was dissolved in water and ethyl acetate. The phases were separated then the aqueous phase was further extracted with ethyl acetate. The organic phases were combined, washed with water and concentrated *in vacuo* to give 2-chloro-1-methyl-3-nitro-4-(2,2,2-trifluoroethoxy)benzene (5.39 mmol, 1.450 g, quant%) as an orange solid, which required no further purification. ¹H NMR (chloroform): 7.32 (m, 1H), 6.91 (d, 1H), 4.42 (q, 2H), 2.39 (s, 3H).

### Step 2.

To a flask containing 2-chloro-1-methyl-3-nitro-4-(2,2,2-trifluoroethoxy)benzene (10.0 mmol, 2.70 g) was added water (41 mL) and pyridine (41 mL). The reaction mixture was stirred at 100 °C until the reaction mixture was a solution. Potassium permanganate (40.0 mmol, 6.33 g) was added in 4 portions, one hour apart. The reaction mixture was stirred at 100 °C for 1 hour more, then left standing at room temperature overnight. The reaction mixture was cooled to room temperature and the solids were filtered off. The solution was washed with ethyl acetate. The aqueous phase was acidified with aqueous 2M HCl, and the material was extracted with ethyl acetate. The organic phases were combined and concentrated *in vacuo* to give 2-chloro-3-nitro-4-(2,2,2-trifluoroethoxy)benzoic acid (4.37 mmol, 1.31 g) as a white solid, which required no further purification. ¹H NMR (Methanol): 8.13 (d, 1H), 7.40 (d, 1H), 4.84 (m, 2H)

### Step 3

To a flask containing 2-chloro-3-nitro-4-(2,2,2-trifluoroethoxy)benzoic acid (4.37 mmol, 1.31 g) was added triethyl orthoformate (60.1 mmol, 8.91 g, 10 mL). The reaction mixture was stirred at 140 °C for 1 hour. The reaction mixture was concentrated *in vacuo* to remove the triethyl orthoformate. The residue was triturated with ethyl acetate, and the solid was dried *in vacuo* to give ethyl 2-chloro-3-nitro-4-(2,2,2-trifluoroethoxy)benzoate (4.23 mmol, 1.39 g, 97%) as an orange solid, which required no further purification. ¹H NMR (chloroform): 8.05 (d, 1H), 7.02 (d, 1H), 4.52 (m, 2H), 4.42 (m, 2H), 1.41 (m, 3H)

### Step 4

To a flask containing ethyl 2-chloro-3-nitro-4-(2,2,2-trifluoroethoxy)benzoate (2.44 mmol, 0.800 g) was added ethanol (8 mL), ammonium chloride (14.7 mmol, 0.784 g), water (8 mL) and iron (7.33 mmol, 0.409 g). The reaction mixture was stirred at 95°C for 1 hour. The reaction mixture was cooled to room temperature and the solids were filtered through celite, then washed with water and ethyl acetate. The solution was further diluted with water and ethyl acetate. The phases were separated then the aqueous phase was further extracted with ethyl acetate. The organic phases were combined, washed with water, then brine, and concentrated *in vacuo* to give ethyl 3-amino-2-chloro-4-(2,2,2-trifluoroethoxy)benzoate (2.43 mmol, 0.723 g, 100%) as a pale orange solid, which required no further purification. ¹H NMR (chloroform): 7.27 (m, 1H), 6.70 (d, 1H), 4.46 - 4.40 (m, 4H), 4.37 (m, 2H), 1.39 (m, 3H)

### Step 5

To a flask ethyl 3-amino-2-chloro-4-(2,2,2-trifluoroethoxy)benzoate (2.43 mmol, 0.723 g) was added ethanol (15 mL), sodium hydroxide (7.29 mmol, 0.291 g) and water (5 mL). The reaction mixture was stirred at room temperature for 6.5 hours. The reaction mixture was concentrated *in vacuo* to remove the ethanol solvent. The aqueous phase was acidified with concentrated HCl to ~ pH 4, then concentrated *in vacuo* to remove the water. The residue was triturated with ethyl acetate and the resulting solid was dried *in vacuo* to give 3-amino-2-chloro-4-(2,2,2-trifluoroethoxy)benzoic acid (assumed 2.43 mmol, assumed quant%), which did not undergo further purification, and was taken on crude.

### Step 6

To a flask containing 3-amino-2-chloro-4-(2,2,2-trifluoroethoxy)benzoic acid (2.43 mmol, 0.655 g) was added 2,3,4,5,6-pentafluorophenol (2.79 mmol, 0.514 g), dichloromethane (33 mL) and 1-3-(dimethylaminopropyl)-3-ethylcarbodiimide hydrochloride (2.79 mmol, 0.564 g). The reaction mixture was stirred at room temperature for 1 hour. The reaction mixture was concentrated *in vacuo* to remove solvent. The reaction residue was dissolved in ethyl acetate and water. The phases were separated then the aqueous phase was further extracted with ethyl acetate. The organic phases were combined and concentrated *in vacuo* to give (2,3,4,5,6-pentafluorophenyl) 3-amino-2-chloro-4-(2,2,2-trifluoroethoxy)benzoate (assumed 2.43 mmol, assumed quant%) as a colourless oil, which did not undergo further purification and was taken on crude.

### Step 7

To a flask containing (2,3,4,5,6-pentafluorophenyl) 3-amino-2-chloro-4-(2,2,2-trifluoroethoxy)benzoate (2.43 mmol, 1.06 g) was added 1-methyltetrazol-5-amine (2.68 mmol, 0.265 g), acetonitrile (21 mL) and 2-tert-butylimino-2-diethylamino-1,3-dimethylperhydro-1,3,2-diazaphosphorine (5.35 mmol, 1.51 g, 1.60 mL) was added. The reaction mixture was stirred at room temperature for 1.5 hours. The reaction mixture was quenched by the addition of aqueous 2M HCl, then extracted with ethyl acetate. The organic phases were combined, washed with water and concentrated *in vacuo* to give an off white solid. The crude material was triturated with dichloromethane, and the resulting solid was dried *in vacuo* to give 3-amino-2-chloro-N-(1-methyltetrazol-5-yl)-4-(2,2,2-trifluoroethoxy)benzamide (1.87 mmol, 0.655 g, 77%) as a white solid. ¹H NMR (Methanol): 7.01 (d, 2H), 4.69 (m, 2H), 4.04 (s, 3H)

### Step 8

To a flask containing 3-amino-2-chloro-N-(1-methyltetrazol-5-yl)-4-(2,2,2-trifluoroethoxy)benzamide (0.570 mmol, 0.200 g) was added acetonitrile (4 mL) and 2-fluoropropanoyl chloride (1.43 mmol, 0.158 g). The reaction mixture was stirred at 60 °C for 1.5 hours. The reaction mixture was concentrated *in vacuo* to remove solvent. The crude material was purified by normal phase flash chromatography (0 to 5% methanol in dichloromethane) to give 2-chloro-3-(2-fluoropropanoylamino)-N-(1-methyltetrazol-5-yl)-4-(2,2,2-trifluoroethoxy)benzamide (Compound 1.016) (0.313 mmol, 0.133 g, 55%) as a white solid. 1H NMR (Methanol): 7.71 (d, 1H), 7.26 (d, 1H), 5.25 (m, 0.5H), 5.13 (m, 0.5H), 4.68 (m, 2H), 4.04 (s, 3H), 1.68 (d, 1.5H), 1.62 (d, 1.5H).

### Preparative Example 7: Compound 1.018

### Step 1

3-amino-2-chloro-N-(1-methyltetrazol-5-yl)-4-(2,2,2-trifluoroethoxy)benzamide is prepared as described above.

### Step 2

To a flask containing 3-amino-2-chloro-N-(1-methyltetrazol-5-yl)-4-(2,2,2-trifluoroethoxy)benzamide (0.428 mmol, 0.150 g) was added acetonitrile (6 mL) and acetyl chloride (1.28 mmol, 0.101 g, 0.092 mL). The reaction mixture was stirred at 60 °C for 1.5 hours. The reaction mixture was concentrated *in vacuo* to remove solvent. The crude material was purified by normal phase flash chromatography (0 to 5% methanol in dichloromethane) to give 3-acetamido-2-chloro-N-(1-methyltetrazol-5-yl)-4-(2,2,2-trifluoroethoxy)benzamide (Compound 1.018) (0.357 mmol, 0.140 g, 83%) as a white solid. ¹H NMR (methanol): 7.67 (d, 1H), 7.25 (d, 1H), 4.67 (m, 2H), 4.04 (s, 3H), 2.18 (s, 3H).

### Preparative Example 8: Compound 1.028

### Step 1

To a flask containing 3-chloro-4-methyl-2-nitro-phenol (5.33 mmol, 1.00 g) was added acetone (20 mL) and water (0.1 mL). Potassium carbonate (8.00 mmol, 1.11 g) and iodomethane (10.7 mmol, 1.51 g, 0.664 mL) were added. The reaction mixture was stirred at room temperature overnight. The reaction mixture was concentrated *in vacuo* to remove the acetone solvent. The residue was dissolved in water and ethyl acetate. The phases were separated then the aqueous phase was further extracted with ethyl acetate. The organic phases were combined and concentrated *in vacuo* to give 3-chloro-1-methoxy-4-methyl-2-nitro-benzene (4.98 mmol, 1.00 g, 93%) as a yellow solid, which required no further purification. ¹H NMR (chloroform): 7.28 (m, 1H), 6.87 (d, 1H), 3.88 (s, 3H), 2.36 (s, 3H).

### Step 2

To a flask containing 3-chloro-1-methoxy-4-methyl-2-nitro-benzene (3.97 mmol, 0.800 g) was added water (16 mL) and pyridine (16 mL). The reaction mixture was stirred at 100 °C until the reaction mixture was a solution. Potassium permanganate (11.9 mmol, 1.88 g) was added in 3 portions, one hour apart. The reaction mixture was stirred at 100 °C overnight. The reaction mixture was cooled to room temperature and the solids were filtered off. The solution was washed with TBME. The aqueous phase was acidified with aqueous 2M HCl, and the material was extracted with ethyl acetate. The organic phases were combined and concentrated *in vacuo* to give 2-chloro-4-methoxy-3-nitro-benzoic acid (2.20 mmol, 0.509 g, 55%) as a white solid, which required no further purification. ¹H NMR (methanol): 8.11 (d, 1H), 7.29 (d, 1H), 4.00 (s, 3H).

### Step 3

To a flask containing 2-chloro-4-methoxy-3-nitro-benzoic acid (8.77 mmol, 2.03 g) was added triethyl orthoformate (120 mmol, 17.8 g, 20 mL). The reaction mixture was stirred at 140°C for 3 hours. The reaction mixture was concentrated *in vacuo* to remove the triethyl orthoformate. The residue was triturated with ethyl acetate, and the solid was dried *in vacuo* to give ethyl 2-chloro-4-methoxy-3-nitro-benzoate (8.77 mmol, 2.28 g, quant%) as an orange solid, which required no further purification.

### Step 4

To a flask containing ethyl 2-chloro-4-methoxy-3-nitro-benzoate (3.85 mmol, 1.00 g) was added ethanol (10 mL), ammonium chloride (23.1 mmol, 1.24 g), water (10 mL) and iron (11.6 mmol, 0.645 g). The reaction mixture was stirred at 95 °C for 1 hour. The reaction mixture was cooled to room temperature and the solids were filtered through celite, then washed with water and ethyl acetate. The solution was further diluted with water and ethyl acetate. The phases were separated then the aqueous phase was further extracted with ethyl acetate. The organic phases were combined, washed with water, then brine, and concentrated *in vacuo* to give ethyl 3-amino-2-chloro-4-methoxy-benzoate (3.76 mmol, 0.864 g, 98%) as an off white solid, which required no further purification.

### Step 5

To a flask containing ethyl 3-amino-2-chloro-4-methoxy-benzoate (3.76 mmol, 0.864 g) was added ethanol (15 mL), sodium hydroxide (11.3 mmol, 0.451 g) and water (5 mL). The reaction mixture was stirred at room temperature overnight. The reaction mixture was concentrated *in vacuo* to remove the ethanol solvent. The aqueous phase was acidified with concentrated HCl to ~ pH 4, then concentrated *in vacuo* to remove the water. The residue was dissolved in water and ethyl acetate and the phases were separated. The organic phase was concentrated *in vacuo* to give 3-amino-2-chloro-4-methoxy-benzoic acid (3.67 mmol, 0.740 g, 98%) as an off white solid, which required no further purification.

### Step 6

To a flask containing 3-amino-2-chloro-4-methoxy-benzoic acid (3.70 mmol, 0.740 g) was added 2,3,4,5,6-pentafluorophenol (4.20 mmol, 0.780 g), dichloromethane (37 mL) and 1-3-(dimethylaminopropyl)-3-ethylcarbodiimide hydrochloride (4.20 mmol, 0.850 g). The reaction mixture was stirred at room temperature for 30 minutes. The reaction mixture was concentrated *in vacuo* to remove solvent. The reaction residue was dissolved in ethyl acetate and water. The phases were separated then the aqueous phase was further extracted with ethyl acetate. The organic phases were combined and concentrated *in vacuo* to give (2,3,4,5,6-pentafluorophenyl) 3-amino-2-chloro-4-methoxy-benzoate (assumed 3.70 mmol, assumed quant%) as an orange oil, which did not undergo further purification and was taken on crude.

### Step 7

To a flask containing (2,3,4,5,6-pentafluorophenyl) 3-amino-2-chloro-4-methoxy-benzoate (3.70 mmol, 1.36 g) was added 1-methyltetrazol-5-amine (4.07 mmol, 0.403 g), acetonitrile (27 mL), 2-tert-butylimino-2-diethylamino-1,3-dimethylperhydro-1,3,2-diazaphosphorine (8.14 mmol, 2.30 g, 2.43 mL). The reaction mixture was stirred at room temperature for 1 hour. The reaction mixture was quenched by the addition of aqueous 2M HCl, then extracted with ethyl acetate. The organic phases were combined, washed with water and concentrated *in vacuo* to give an orange oil. The crude material was purified by normal phase flash chromatography (0 to 10% methanol in dichloromethane) to give 3-amino-2-chloro-4-methoxy-N-(1-methyltetrazol-5-yl)benzamide (2.21 mmol, 0.624 g, 60%) as a pale orange solid. ¹H NMR (Methanol): 7.04 (d, 1H), 6.91 (d, 1H), 4.03 (s, 3H), 3.94 (s, 3H).

### Step 8

To a flask containing 2-fluoropropanoic acid (10.9 mmol, 1.00 g) was added thionyl chloride (23.5 mmol, 2.80 g, 1.68 mL). The reaction mixture was stirred at 60 °C for 4 hours. The product was distilled off to give 2-fluoropropanoyl chloride (6.81 mmol, 0.753 g, 63%) as a colourless oil. ¹H NMR (chloroform): 5.21 (m, 0.5H), 5.08 (m, 0.5H), 1.74 (d, 1.5H), 1.68 (d, 1.5H)

### Step 9

To a flask containing 3-amino-2-chloro-4-methoxy-N-(1-methyltetrazol-5-yl)benzamide (0.707 mmol, 0.200 g) was added acetonitrile (8 mL) and 2-fluoropropanoyl chloride (1.41 mmol, 0.209 g). The reaction mixture was heated to 60 °C for 4 hours. The reaction mixture was concentrated *in vacuo* to remove solvent. The crude material was purified by normal phase flash chromatography (0 to 10% methanol in dichloromethane) to give 2-chloro-3-(2-fluoropropanoylamino)-4-methoxy-N-(1-methyltetrazol-5-yl)benzamide (Compound 1.031) (0.555 mmol, 0.198 g, 79%) as yellow solid. ¹H NMR (Methanol): 7.69 (d, 1H), 7.17 (d, 1H), 5.25 (m, 0.5H), 5.13 (m, 0.5H), 4.03 (s, 3H), 3.92 (s, 3H), 1.68 (d, 1.5H), 1.61 (d, 1.5H).

### Preparative Example 10: Compound 1.030

### Step 1

3-amino-2-chloro-4-methoxy-N-(1-methyltetrazol-5-yl)benzamide is prepared as described above.

### Step 2

To a flask containing 3-amino-2-chloro-4-methoxy-N-(1-methyltetrazol-5-yl)benzamide (0.531 mmol, 0.150 g) was added acetonitrile (6 mL) and acetyl chloride (1.06 mmol, 0.0833 g). The reaction mixture was stirred at 60 °C for 2 hours. The reaction mixture was concentrated *in vacuo* to remove solvent. The crude material was triturated with ethyl acetate, and the solid was dried *in vacuo* to give 3-acetamido-2-chloro-4-methoxy-N-(1-methyltetrazol-5-yl)benzamide (Compound 1.033) (0.490 mmol, 0.159 g, 92%) as a white solid. ¹H NMR (Methanol): 7.66 (d, 1H), 7.16 (d, 1H), 4.04 (s, 3H), 3.92 (s, 3H), 2.17 (s, 3H).

### Preparative Example 11: Compound 2.001

### Step 1

3-bromo-2-chloro-6-(trifluoromethoxy)aniline is prepared as outlined previously.

### Step 2

To a flask containing 3-bromo-2-chloro-6-(trifluoromethoxy)aniline (2.00 g, 6.89 mmol) was added tetrahydrofuran (30 mL). The reaction mixture was put under a nitrogen atmosphere and stirred at -78 °C for 30 minutes. To the reaction mixture, *n*-butyllithium (2.5 M in hexane, 7.57 mmol, 3.00 mL) was added dropwise. The reaction mixture was stirred at -78 °C for 1 hour. To the reaction mixture was added iodomethane (2.93 g, 20.7 mmol, 1.29 mL). The reaction mixture was allowed to warm to 0 °C and stirred for 3 hours. The reaction mixture was carefully quenched by slow addition of the reaction mixture into saturated aqueous ammonium chloride solution. The mixture was stirred at room temperature for 15 minutes, and the aqueous phase was extracted with ethyl acetate. The organic phases were combined, dried and concentrated *in vacuo.* The crude material was purified by normal phase flash chromatography (0 to 5 % ethyl acetate in cyclohexane) to give impure product. The crude material was purified by reverse phase flash chromatography (60 to 100 % (acetonitrile + 0.1% formic acid) in (water + 0.1% formic acid)) to give 3-bromo-2-chloro-N-methyl-6-(trifluoromethoxy)aniline (0.587 g, 1.93 mmol, 28%) as a brown oil. ¹H NMR (chloroform): 7.03 - 6.99 (m, 1H), 6.99 - 6.94 (m, 1H), 3.08 (s, 3H).

### Step 3

To a flask containing 3-bromo-2-chloro-N-methyl-6-(trifluoromethoxy)aniline (1.36 g, 4.47 mmol) was added tetrahydrofuran (41 mL). The reaction mixture was put under a nitrogen atmosphere and stirred at -78 °C for 1 hour. To the reaction mixture, n-butyllithium (2.5 M in hexane, 4.91 mmol, 2.00 mL) was added dropwise. The reaction mixture was stirred at -78 °C for 45 minutes. To the reaction mixture was added iodomethane (1.90 g, 13.4 mmol, 0.834 mL). The reaction mixture was allowed to warm to 0 °C and stirred for 3.5 hours. The reaction mixture was carefully quenched by slow addition of the reaction mixture into ice cooled saturated aqueous ammonium chloride solution. The mixture was stirred at 0 °C for 15 minutes, and the aqueous phase was extracted with ethyl acetate. The organic phases were combined, dried and concentrated *in vacuo.* The crude material was purified by normal phase flash chromatography (0 to 1 % ethyl acetate in cyclohexane) to give 3-bromo-2-chloro-N,N-dimethyl-6-(trifluoromethoxy)aniline (0.520 g, 1.63 mmol, 37%). ¹H NMR (chloroform): 7.35 (d, *J* = 8.9 Hz, 1H), 7.01 (m, 1H), 2.87 (s, 6H).

### Step 4

To a vessel containing 3-bromo-2-chloro-N,N-dimethyl-6-(trifluoromethoxy)aniline (0.520 g, 1.63 mmol) was added palladium(II) acetate (0.0367 g, 0.163 mmol), XantPhos (0.195 g, 0.327 mmol), N-formylsaccharine (0.776 g, 3.67 mmol), 5-methyl-1,3,4-oxadiazol-2-amine (1.46 g, 14.7 mmol) and 1-methyl-2-pyrrolidinone was added so the total volume of the vessel was 20 mL. To a second vessel containing triethylamine (0.743 g, 7.35 mmol, 1.02 mL) was added 1-methyl-2-pyrrolidinone so that the total volume of the vessel was 20 mL. The reaction was carried out in a Uniqsis FlowSyn. The two solutions were injected into the sample loops pumped through a T-piece and then round a 20 mL stainless steel coil heated to 170 °C. The flow rate was set so that the total residence time was 20 mins. The reaction mixture was concentrated *in vacuo* to remove the solvent 1-methyl-2-pyrrolidinone. The residue was purified by reverse phase HPLC to give impure product. The crude material was purified by normal phase flash chromatography (10 to 80% ethyl acetate in cyclohexane) to give 2-chloro-3-(dimethylamino)-N-(5-methyl-1,3,4-oxadiazol-2-yl)-4-(trifluoromethoxy)benzamide (Compound 2.001) (0.174 g, 0.478 mmol, 29%) as a pale yellow solid. ¹H NMR (Methanol): 7.37 - 7.34 (m, 2H), 2.90 (s, 6H), 2.51 (s, 3H).

### TABLE 1 - Examples of herbicidal compounds of the present invention.

Compounds are characterised using NMR as indicated or LCMS using the following conditions. Waters Aquity UPLC-MS using a Sample Organizer with Sample Manager FTN, H-class QSM, Column Manager, 2 x Column Manager Aux, photodiode array, ELSD and a QDA SQD 2 equipped with a Waters HSS C18 column (column length 50 mm, internal diameter of column 2.3 mm, particle size 1.8 micron). The analysis was conducted using a four minute run time, according to the following gradient table:

| Time (mins) | Solvent A (%) | Solvent B (%) | Flow (ml / mn) |
|---|---|---|---|
| 0 | 95 | 5 | 0.6 |
| 3.3 | 0 | 100 | 0.6 |
| 3.5 | 0 | 100 | 0.6 |
| 3.55 | 95.5 | 5 | 0.6 |
| 4.1 | 95.5 | 5 | 0.6 |

| | | | |
|---|---|---|---|
| Solvent A: H₂O with 0.05% TFA Solvent B: CH₃CN with 0.05% TFA | | | |

| **Compound Number** | **Structure** | **1H-NMR or LCMS (M⁺H; RT(min))** | |
|---|---|---|---|
| 1.001 | 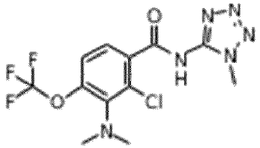 | 1H NMR (chloroform): 7.44 (d, 1H), 7.30-7.25 (m, 1H), 4.12 (s, 3H), 2.91 (s, 6H) | |
| 1.002 | 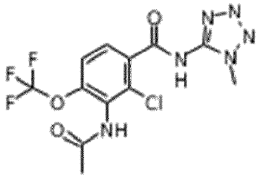 | 1H NMR (Methanol): 7.75 (d, 1H), 7.53 (m, 1H), 4.06 (s, 3H), 2.20 (s, 3H) | |
| 1.003 | 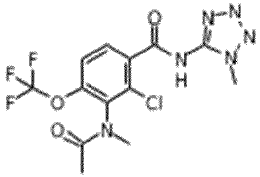 | 1H NMR (Methanol, rotameric): 7.89 (d, 0.8H), 7.81 (d, 0.2H), 7.67 (m, 0.8H), 7.57 (m, 0.2H), 4.11-4.02 (m, 3H), 3.36 (s, 0.6H), 3.17 (s, 2.4H), 2.32 (s, 0.6H), 1.84 (s, 2.4H) | |
| 1.004 | 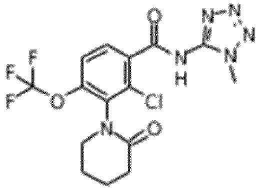 | 1H NMR (Methanol): 7.82 (d, 1H), 7.58 (m, 1H), 4.06 (s, 3H), 3.69-3.60 (m, 1H), 3.59-3.50 (m, 1H), 2.57 (m, 2H), 2.10-1.93 (m, 4H) | |
| 1.005 | 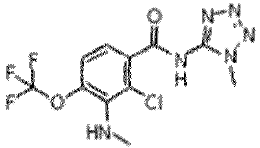 | 1H NMR (Methanol): 7.29 (m, 1H), 7.00 (d, 1H), 4.05 (s, 3H), 3.07 (s, 3H) | |
| 1.006 | 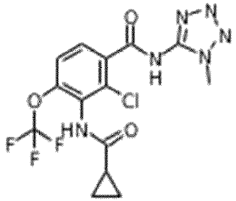 | 1H NMR (Methanol): 7.73 (d, 1H), 7.52 (br d, 1H), 4.06 (s, 3H), 1.91 (m, 1H), 1.01-0.88 (m, 4H) | |
| 1.007 | 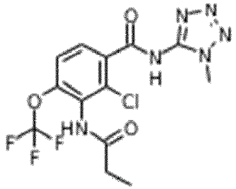 | 1H NMR (400 MHz, d6-DMSO): 11.92 (1H, s), 9.90 (1H, s), 7.80 (1H, d), 7.61 (1H, d), 4.00 (3H, s), 2.37 (2H, q), 1.12 (3H, t) | |
| 1.008 | 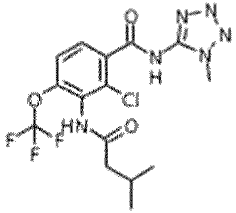 | 1H NMR (400 MHz, methanol) 7.73 (d, 1H), 7.52 (m, 1H), 4.04 (s, 3H), 2.33 (d, 2H), 2.19 (m, 1H), 1.06 (d, 6H) | |
| 1.009 | 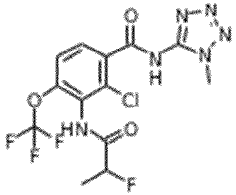 | 1H NMR (400 MHz, methanol) δ = 7.79 (d, 1H), 7.59-7.54 (m, 1H), 5.22 (qd, 1H), 4.06 (s, 3H), 1.65 (dd, 3H) | |
| 1.010 | 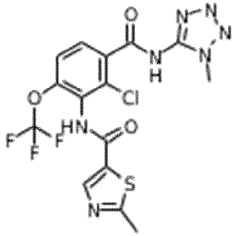 | 1H NMR (400 MHz, methanol) 8.39 (s, 1H), 7.84 (d, 1H), 7.62 (m, 1H), 4.09 (s, 3H), 2.80 (s, 3H) | |
| 1.011 | 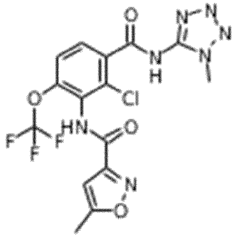 | 1H NMR (400 MHz, methanol) 7.85 (d, 1H), 7.62 (m, 1H), 6.59 (s, 1H), 4.09 (s, 3H), 2.56 (s, 3H) | |
| 1.012 | 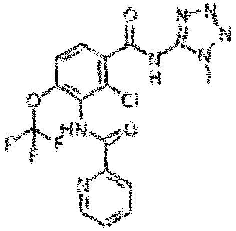 | 1HNMR(Methanol): 8.76(m,1H), 8.19(m,1H), 8.04(m,1H), 7.81(d,1H), 7.65(m,1H), 7.60(m,1H), 4.07(s,3H) | |
| 1.013 | 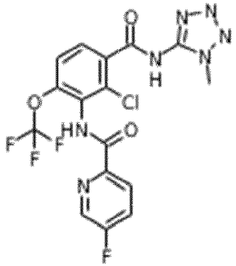 | 1H NMR (400 MHz, methanol) 8.67 (d, 1H), 8.29 (m, 1H), 7.89-7.82 (m, 2H), 7.63 (m, 1H), 4.10 (s, 3H) | |
| 1.014 | 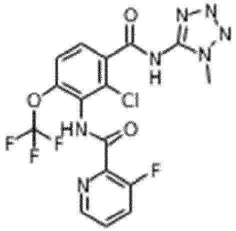 | 1H NMR (400 MHz, methanol) 8.61 (d, 1H), 7.89-7.81 (m, 2H), 7.80-7.72 (m, 1H), 7.63 (m, 1H), 4.10 (s, 3H) | |
| 1.015 | 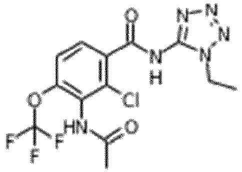 | 1HNMR(Methanol): 7.77(d,1H), 7.57(m,1H), 4.45(m,2H), 2.23(s,3H), 1.60(m,3H) | |
| 1.016 | 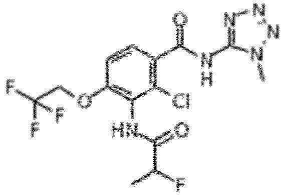 | 1H NMR (Methanol): 7.71 (d, 1H), 7.26 (d, 1H), 5.25-5.13 (m, 1H), 4.68 (m, 2H), 4.04 (s, 3H), 1.68-1.62 (m, 3H) | |
| 1.017 | 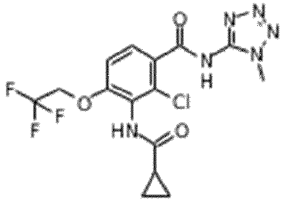 | 1H NMR (Methanol): 7.66 (d, 1H), 7.24 (d, 1H), 4.66 (m, 2H), 4.05 (s, 3H), 1.90 (m, 1H), 1.02-0.84 (m, 4H) | |
| 1.018 | 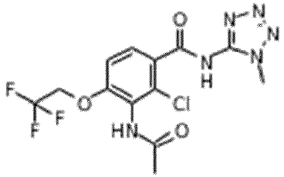 | 1H NMR (Methanol): 7.67 (d, 1H), 7.25 (d, 1H), 4.67 (m, 2H), 4.04 (s, 3H), 2.18 (s, 3H) | |
| 1.019 | 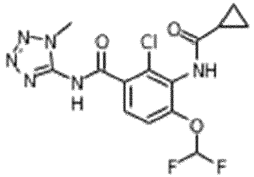 | 1H NMR (DMSO-d6) δ ppm 11.85 (br s, 1H) 10.00 (s, 1H) 7.72 - 7.74 (d, 1H) 6.99 - 7.38 (m, 2H) 3.98 (s, 3H) 1.90 (br s, 1H) 0.77-0.82 (m, 4H) | |
| 1.020 | 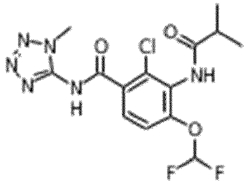 | 1H NMR (DMSO-d6) δ ppm 11.85 (br s, 1H) 9.67 (s, 1H) 7.74 (d, 1H) 7.35 (d, 1H) 7.15 (t, 1H) 3.98 (s, 3H) 2.68-2.72 (m, 1H) 1.14 (d, 6H) | |
| 1.021 | 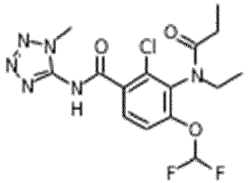 | | |
| 1.022 | 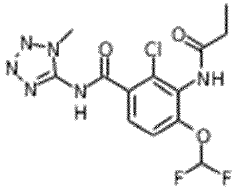 | 1H NMR (DMSO-d6) δ ppm 11.89 (br s, 1H) 9.72 (br s, 1H) 7.74 - 7.76 (d, 1H) 6.91-7.49 (m, 2H) 3.99 (s, 3H) 2.34 - 2.40 (m, 2H) 1.10 - 1.14 (t, 3H) | |
| 1.023 | 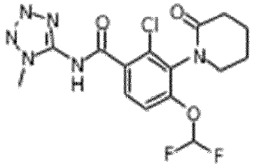 | 1H NMR (METHANOL-d4) δ ppm7.79 (d, 1H) 7.44 (d, 1H) 6.97 (t, 1H) 4.08 (s, 3H) 3.58 - 3.61 (m, 2H) 2.59 (br t, 2H) 1.98-2.09 (m, 4H) | |
| 1.024 | 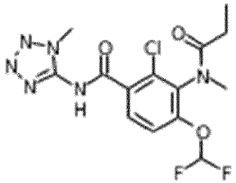 | 1H NMR (400 MHz, CHLOROFORM-d, 60°C) δ ppm 0.96 (t, 3H) 1.74 - 2.04 (m, 2 H) 3.05 (s, 3H) 4.01 (s, 3H) 7.45 (t, 1H) 7.53 (d, 1H) 7.86 (d, 1H) 11.76 (br s, 1 H) | |
| 1.025 | 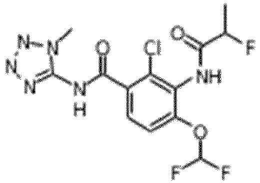 | 1H NMR (METHANOL-d4) δ ppm 7.76 (d, 1H), 7.42 (d, 1H) 6.92 (t, 1H) 5.15 - 5.33 (m, 1H) 4.08 (s, 3H) 1.62-1.74 (m, 3H) | |
| 1.026 | 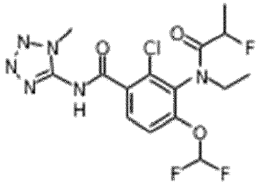 | 1H NMR (400 MHz, CHLOROFORM-d) δ ppm 1.16 - 1.29 (m, 3 H) 1.37 - 1.53 (m, 3 H) 3.57-3.93 (m, 2 H) 4.26 (s, 3 H) 4.85-5.01 (m, 1 H) 6.55 - 6.69 (m, 1 H) 7.35 - 7.41 (m, 1 H) 7.43 - 7.52 (m, 1 H) | |
| 1.027 | 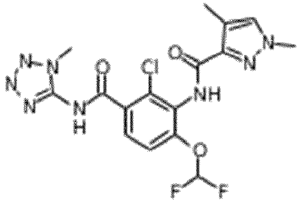 | | |
| 1.028 | 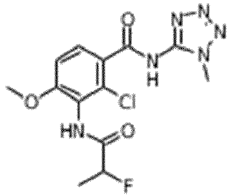 | 1H NMR (Methanol): 7.69 (d, 1H), 7.17 (d, 1H), 5.25-5.13 (m, 1H), 4.03 (s, 3H), 3.92 (s, 3H), 1.68-1.61 (m, 3H) | |
| 1.029 | 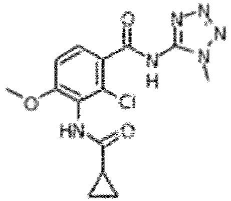 | 1H NMR (Methanol): 7.64 (d, 1H), 7.15 (d, 1H), 4.04 (s, 3H), 3.92 (s, 3H), 1.87 (m, 1H), 1.00-0.83 (m, 4H) | |
| 1.030 | 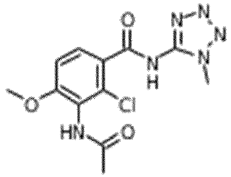 | 1H NMR (Methanol): 7.66 (d, 1H), 7.16 (d, 1H), 4.04 (s, 3H), 3.92 (s, 3H), 2.17 (s, 3H) | |
| 1.031 | 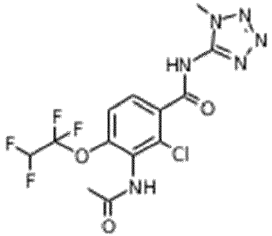 | 1H NMR (d6-DMSO): 11.87 (1H, s), 9.83 (1H, s), 7.76 (1H, d), 7.52 (1H, d), 6.78 (1H, t), 3.98 (3H, s), 2.04 (3H, s) | |
| 1.032 | 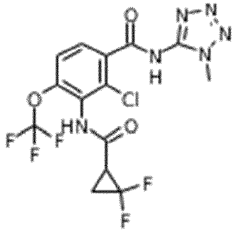 | 1H NMR (400 MHz, methanol) 7.76 (d, 1H), 7.55 (m, 1H), 4.06 (s, 3H), 2.83 (m, 1H), 2.10 (m, 1H), 1.98-1.83 (m,1H) | |
| 1.033 | 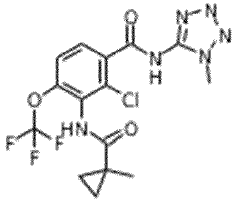 | 1H NMR (400 MHz, methanol) 7.75 (d, 1H), 7.52 (m, 1H), 4.06 (s, 3H), 1.49 (s, 3H), 1.23 (m, 2H), 0.77 (m, 2H) | |
| 1.034 | 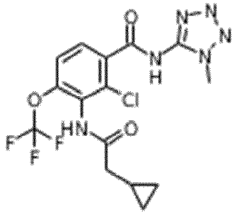 | 1H NMR (400 MHz, methanol) 7.76 (d, 1H), 7.55 (m, 1H), 4.06 (s, 3H), 2.35 (d, 2H), 1.16 (m, 1H), 0.61 (m, 2H), 0.30 (m, 2H) | |
| 1.035 | 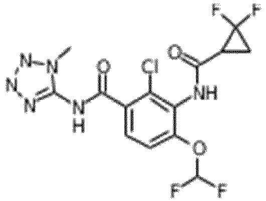 | 1H NMR (METHANOL-d4) δ ppm 7.60 (d, 1H) 7.28 (d, 1H) 6.76 (t, 1H), 3.94 (s, 3H), 2.71-2.75 (m, 1H) 1.94-2.01 (m, 1H) 1.78 - 1.92 (m, 1H) | |
| 1.036 | 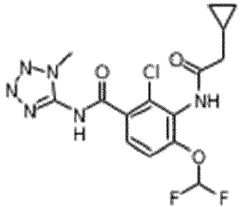 | 1HNMR(400MHz,METHANOL-d4): 0.31-0.36(m,2H), 0.60-0.65(m,2H), 1.18(brs,1H), 1.38(s,1H), 2.05(s,1H), 4.37(d,2H), 4.08(s,3H), 6.91(t,1H), 7.40(d,1H), 7.72(d,1H) | |
| 1.037 | 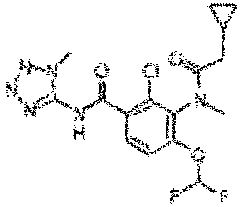 | 1H NMR (400 MHz, CHLOROFORM-d) δ ppm 0.01 (br d, 2 H) 0.49 (br d, 2 H) 0.97-1.05 (m, 1 H) 1.91 (br d, 2H) 3.19 (s, 3 H) 4.12 (s, 3 H) 6.69 (t,1H) 7.40 (d, 1 H) 7.82 (d,1 H) 11.18 (s, 1H) | |
| 1.038 | 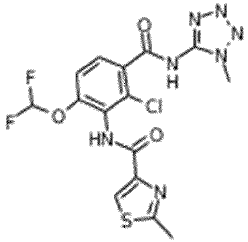 | 1H NMR (400 MHz, DMSO-d6) δ ppm 11.92 (br s, 1 H) 10.16 (s, 1 H) 8.30 (s, 1 H) 7.82 (d, 1 H) 7.44 (d, 1 H) 7.27 (t, 1 H) 4.00 (s, 3H) 2.78 (s, 3 H) | |
| 1.039 | 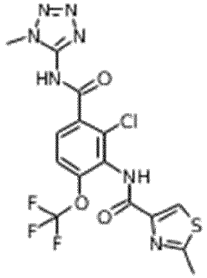 | 1HNMR: 1H NMR (400 MHz, DMSO-d6) δ ppm 2.78 (s, 3 H) 4.00 (s, 3 H) 7.08 (s, 1 H) 7.26 (s, 1 H) 7.38-7.47 (m, 1 H) 7.81 (d, J=8.56 Hz, 1 H) 8.29 (s, 1 H) 10.16 (s, 1 H) 11.92 (br s, 1 H) | |
| 1.040 | 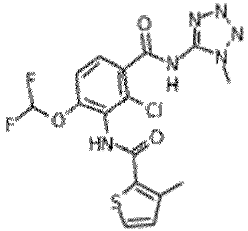 | 1H NMR(400MHz,CHLOROFORM-d): 2.56-2.62(s,3H), 4.06-4.17(s,3H), 6.63(t,1H), 7.00(d,1H), 7.29(s,1H), 7.43-7.49(d,2H), 7.73(d,1H), 10.42-10.80(s,1H) | |
| 1.041 | 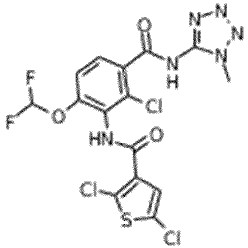 | 1H NMR (400 MHz, METHANOL-d4) δ ppm 7.67 (br d, 1 H) 7.34 (br d, 1 H) 7.20 (s, 1 H) 6.84 (t, 1 H) 4.00 (s, 3 H) | |
| 1.042 | 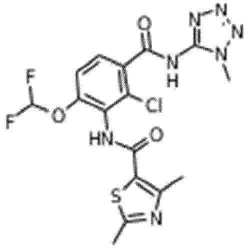 | 1H NMR(400MHz,DMSO-d6): 2.58(s,3H), 2.67(s,3H), 4.06(s,3H), 7.36(t,1H), 7.44(brd,1H), 7.82(brd,1H), 10.00(s,1H), 11.60-12.25(s,1H) | |
| 1.043 | 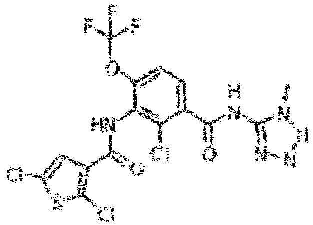 | 1H NMR (400 MHz, DMSO-d6) δ ppm 11.91 (br s, 1 H) 10.47 (s, 1 H) 7.90 (d, 1H) 7.71 (d, 1 H) 7.48 (s, 1 H) 4.01 (s, 3 H) | |
| 1.044 | 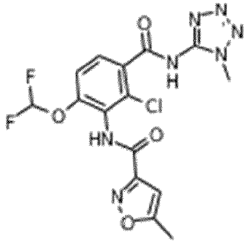 | 1H NMR (400 MHz, METHANOL-d4) δ ppm 7.67 (d, 1 H) 7.33 (d, 1 H) 6.82 (t, 1H) 6.46 (s, 1H) 3.96 (s, 3H) 2.43 (s, 3H) | |
| 1.045 | 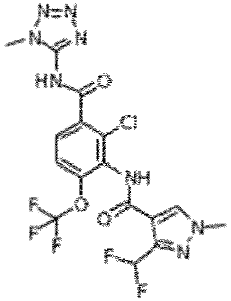 | 1H NMR (400 MHz, METHANOL-d4) δ ppm 8.21 (s, 1 H) 7.70 (d, 1 H) 7.48 (d, 1 H) 7.07 (t, J=54.10 Hz, 1 H) 3.97 (s, 3 H) 3.92 (s, 3 H) | |
| 1.046 | 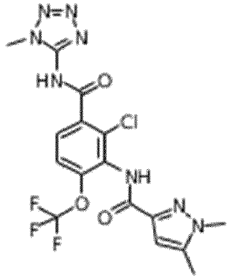 | 1H NMR (d6-DMSO): 11.96 (1H, s), 10.02 (1H,s), 7.84 (1H, d), 7.63 (1H, d), 6.56 (1H, s), 4.01 (3H, s), 3.86 (3H, s), 2.32 (3H, s) | |
| 1.047 | 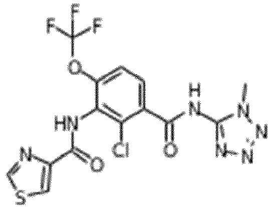 | 1H NMR(400MHz,DMSO-d6): 3.99(s,3H), 7.65(d,1H), 7.86(d,1H), 8.54(d,1H) 9.30(d,1H), 10.49(s,1H), 12.00(brs,1H) | |
| 1.048 | 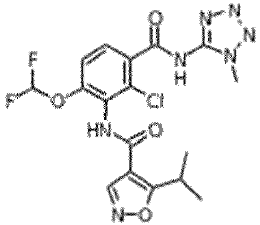 | 1HNMR(400MHz,DMSO-d6): 1.02-1.08(m,1H), 2.99(d,6H), 3.99(s,3H), 7.17(s,1H), 7.34(d,1H), 7.65(d,1H) 11.40(s,1H), 11.83(s,1H) | |
| 1.049 | 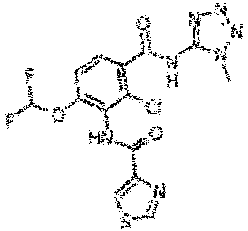 | 1H NMR(400MHz,DMSO-d6): 1.17(t,1H), 1.99(s,1H), 3.98(s,3H), 4.03(m,1H), 7.26 (1H, t), 7.44 (1H, d), 7.80(d,1H), 8.52(d,1H), 9.29(d,1H), 10.26(s,1H), 11.90 (1H, brs) | |
| 1.050 | 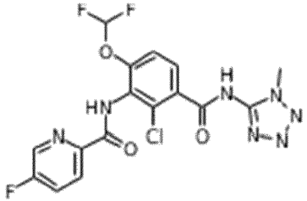 | 1H NMR (400 MHz, DMSO-d6) δ ppm 12.05 (br s, 1H) 10.55 (s, 1 H) 8.75 - 8.82 (m, 1 H) 8.21 (br dd, 1 H) 7.99 (td, 1 H) 7.83 (br d, 1 H) 7.44 (br d, 1 H) 7.09 (t, 1 H) 4.03 (s, 1 H) | |
| 1.051 | 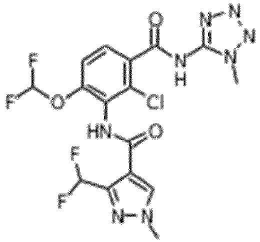 | 1H NMR (400 MHz, DMSO-d6) δ ppm 11.91 (s, 1 H) 10.09 (s, 1 H) 8.51 (s, 1 H) 7.81 (d, 1 H) 7.44 (d, 1 H) 7.29 (t 1 H) 7.28 (t, 1H) 4.01 (s, 3H) 4.00 (s, 3H) | |
| 1.052 | 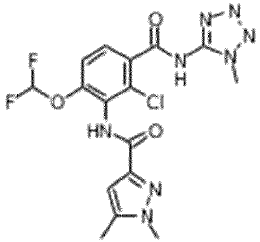 | 1H NMR (400 MHz, DMSO-d6) δ ppm 11.91 (br s, 1 H) 9.82 (s, 1 H) 7.79 (d, 1 H) 7.45 (d, 1 H) 7.23 (t, 1 H) 6.55 (s, 1 H) 4.00 (s, 3 H) 3.85 (s, 3 H) 2.32 (s, 3 H) | |
| 1.053 | 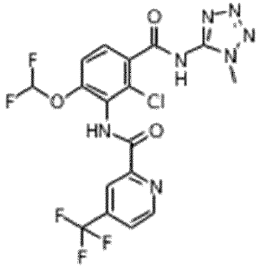 | 1H NMR (400 MHz, DMSO-d6): 11.93 (br s, 1 H) 10.80 (s, 1 H) 9.08 (d, 1 H) 8.32 (s, 1 H) 8.15 (br d, 1 H) 7.84 (d, 1 H) 7.46 (d, 1 H) 7.28 (t, 1 H) 3.98 (s, 3 H) | |
| 1.054 | 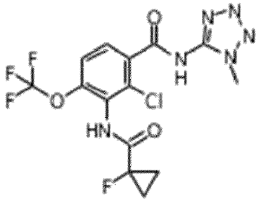 | 421.3 | 1.7 |
| 1.055 | 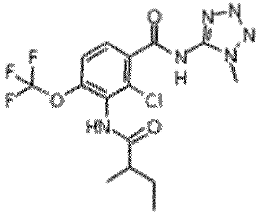 | 419.3 | 1.8 |
| 1.056 | 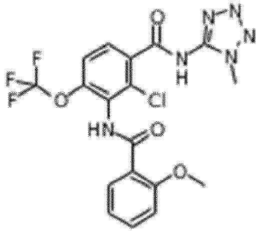 | 469.3 | 1.98 |
| 1.057 | 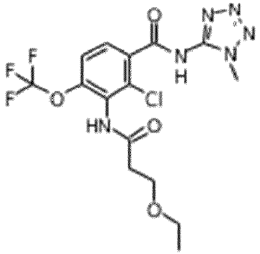 | 435.3 | 1.6 |
| 1.058 | 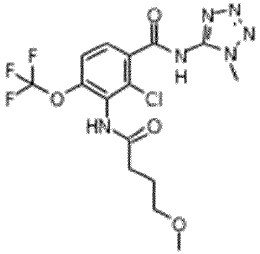 | 435.3 | 1.49 |
| 1.059 | 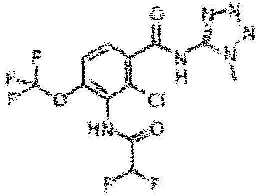 | 413.2 | 1.55 |
| 1.060 | 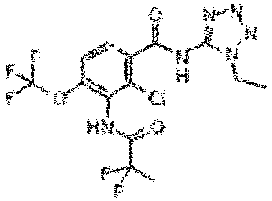 | 441.2 | 1.8 |
| 1.061 | 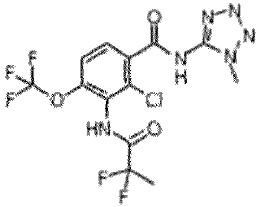 | 427.3 | 1.7 |
| 1.062 | 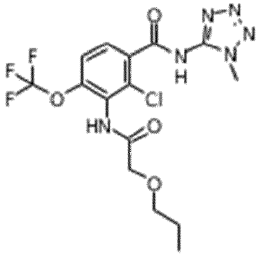 | 435.3 | 1.84 |
| 1.063 | 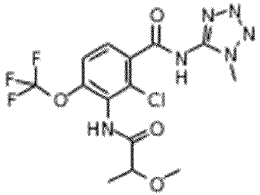 | 421.3 | 1.54 |
| 1.064 | 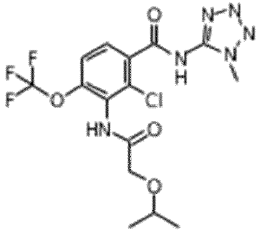 | 435.3 | 1.8 |
| 1.065 | 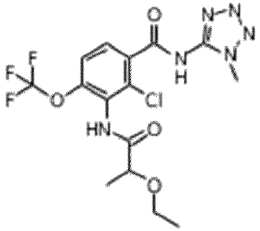 | 435.3 | 1.73 |
| 1.066 | 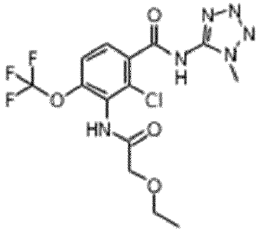 | 421.3 | 1.62 |
| 1.067 | 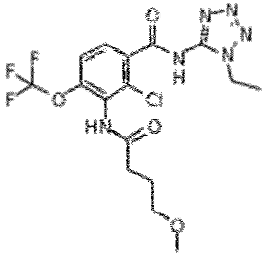 | 449.3 | 1.6 |
| 1.068 | 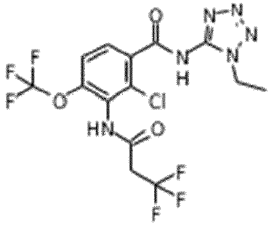 | 459.2 | 1.81 |
| 1.069 | 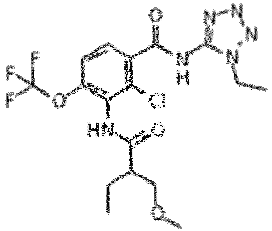 | 463.3 | 1.85 |
| 1.070 | 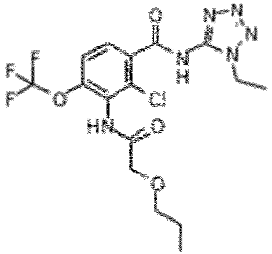 | 449.3 | 1.93 |
| 1.071 | 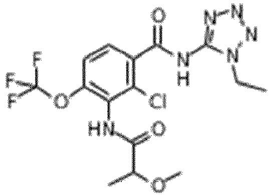 | 435.3 | 1.65 |
| 1.072 | 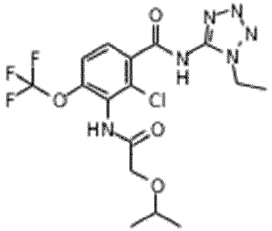 | 449.3 | 1.9 |
| 1.073 | 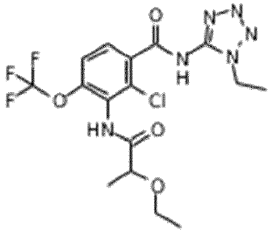 | 449.3 | 1.83 |
| 1.074 | 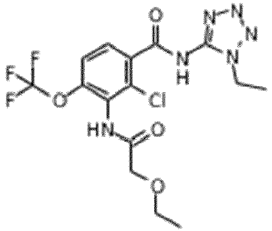 | 435.3 | 1.73 |
| 1.075 | 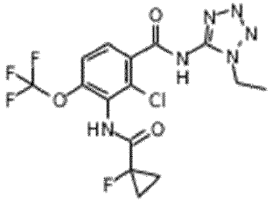 | 435.3 | 1.79 |
| 1.076 | 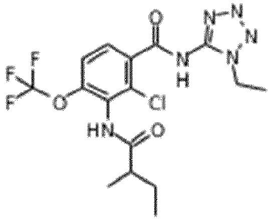 | 433.3 | 1.89 |
| 1.077 | 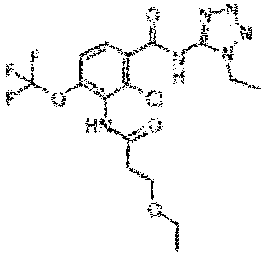 | 449.3 | 1.7 |
| 1.078 | 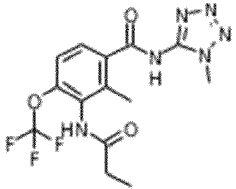 | 1H NMR (400MHz, methanol) δ ppm 7.70 (d, 1H) 7.35-7.43 (m, 1H) 4.05 (s, 3H) 2.48 (q, 2H) 2.38 (s, 3H) 1.26 (t, 3H) | |
| 1.079 | 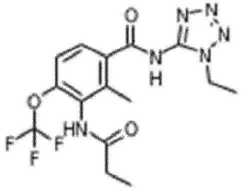 | 1H NMR (400MHz, methanol) δ ppm 7.69 (d,1H) 7.39 (dq, 1H) 4.41 (q,2H) 2.48 (q,2H) 2.38 (s, 3H) 1.58 (t, 3H) 1.26 (t, 3H) | |
| 1.080 | 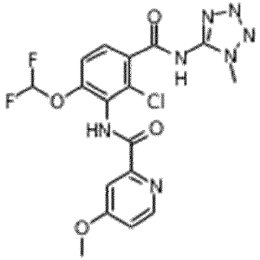 | 1H NMR (400 MHz, d6-DMSO): 11.92 (1H, brs, 10.51 (1H, s), 8.57 (1H, d), 7.81 (1H, d), 7.62 (1H, d), 7.46-7.06 (3H, m), 3.98 (3H, s), 3.94 (3H, s) | |
| 1.081 | 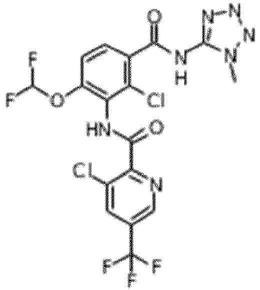 | 1H NMR (400 MHz, DMSO-d6) δ ppm 4.01 (s, 3 H) 7.37 (t, 1H), 7.49 (d, 1 H) 7.85 (d, 1 H) 8.71 (s, 1 H) 9.12 (s, 1 H) 10.74 (s, 1 H) 11.92 (br s, 1 H) | |
| 1.082 | 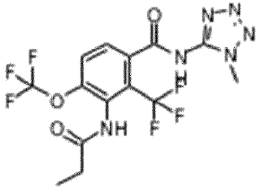 | 1H NMR (400MHz, methanol) δ ppm 7.83 (brs, 2H), 4.05 (s, 3H), 2.46 (q, 2H), 1.24 (t, 3H) | |
| 1.083 | 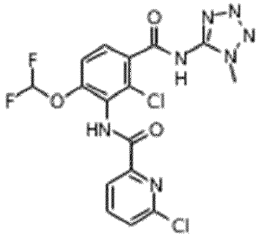 | 1H NMR (400 MHz, DMSO-d6) δ ppm 12.12 (br s, 1 H) 10.49 (s, 1 H) 8.08 - 8.16 (m, 2 H) 7.80 - 7.89 (m, 2 H) 7.46 (d, 1 H) 7.29 (t, 1 H) 3.99 (s, 3 H) | |
| 1.084 | 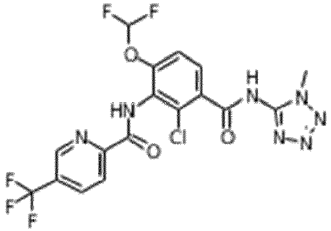 | 1H NMR (400 MHz, DMSO-d6) δ ppm 11.94 (br s, 1 H) 10.82 (s, 1 H) 9.19 (s, 1 H) 8.51 (dd, 1 H) 8.33 (d, 1 H) 7.86 (d, 1 H) 7.50 (d, 1 H) 7.23 (t, 1 H) 4.01 (s, 3 H) | |
| 1.085 | 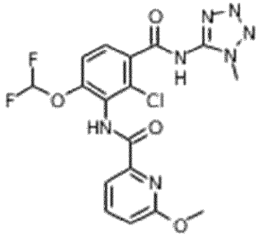 | 1H NMR (400 MHz, DMSO-d6) δ ppm 12.03 (br s, 1 H) 10.22 (s, 1 H) 7.95 (dd, 1 H) 7.85 (d, 1 H) 7.71 (d, 1 H) 7.49 (d, 1 H) 7.30 (t, 1 H) 7.14 (d, 1H) 4.01 (s, 3 H) 4.03 ( s, 3 H) | |
| 1.086 | 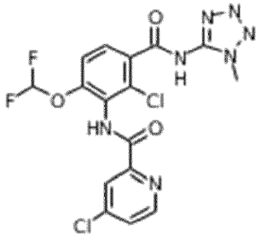 | 1H NMR (400 MHz, DMSO-d6) 11.92 (br s, 1 H) 10.66 (s, 1 H) 8.77 (d, 1 H) 8.13 (d, 1 H) 7.90 (dd, 1 H) 7.85 (d, 1 H) 7.46 (d, 1 H) 7.11 (t, 1 H) 4.01 (s, 3 H) | |
| 1.087 | 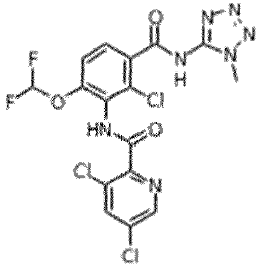 | 1H NMR (400 MHz, DMSO-d6) δ ppm 11.92 (s, 1 H) 10.62 (s, 1 H) 8.79 (d, 1 H) 8.46 (d, 1 H) 7.84 (d, 1 H) 7.46 (d, 1 H) 7.16 (t, 1 H) 4.01 (s, 3 H) | |
| 1.088 | 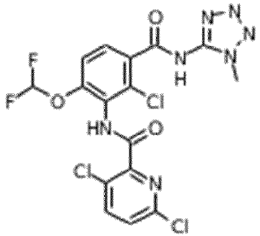 | 1H NMR (400 MHz, DMSO-d6) δ ppm 11.92 (br s, 1 H) 10.67 (s, 1 H) 8.21 (d, 1 H) 7.85 (br d, 1 H) 7.79 (d, 1 H) 7.47 (br d, 1 H) 7.36 (t, 1 H) 4.02 (s, 3 H) | |
| 1.089 | 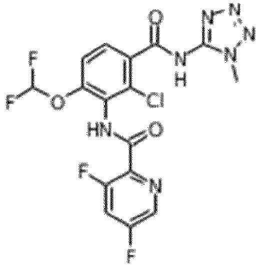 | 1H NMR (400 MHz, DMSO-d6) δ ppm 11.93 (br s, 1 H) 10.52 (s, 1 H) 8.71 (d, 1 H) 8.19 (brt, 1 H) 7.84 (d, 1 H) 7.42 (d, 1 H) 7.12 (t, 1 H) 4.01 (s, 3 H) | |
| 1.090 | 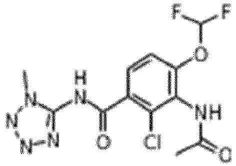 | 1H NMR (400 MHz, METHANOL-d4) δ ppm 7.59 (br d, 1 H) 7.26 (br d, 1 H) 6.78 (t, 1 H) 3.94 (s, 3 H) 2.09 (s, 3 H) | |
| 1.091 | 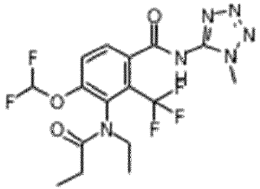 | 1H NMR (CHLOROFORM-d) δ ppm 7.72 (d, 1H) 7.60 (d, 1H) 6.78 (t, 1H) 4.25 (s, 3H) 4.14 - 4.18 (m, 1H) 3.96 - 4.40 (m, 1H) 2.71 (q, 2H) 1.27 (t, 3H) 1.16 (t, 3H) | |
| 1.092 | 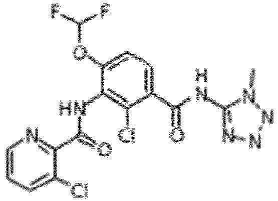 | 1H NMR (400 MHz, DMSO-d6) δ ppm 11.92 (s, 1 H) 10.57 (s, 1 H) 8.68 (d, 1 H) 8.13 (d, 1 H) 7.83 (br d, 1 H) 7.65 (dd, 1 H) 7.46 (br d, 1 H) 7.33 (t, 1 H) 4.01 (s, 3 H) | |
| 1.093 | 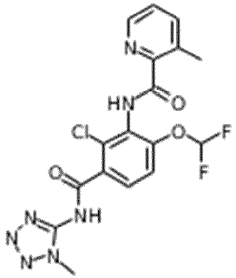 | 1H NMR (400 MHz, CHLOROFORM-d) δ ppm 11.00 (br s, 1 H) 10.00 (s, 1 H) 8.48 (br d, 1 H) 7.72 (d, 1 H) 7.64 (d, 1 H) 7.39 (m, 1 H) 7.25 (d, 1 H) 6.63 (t, 1 H) 4.01 (s, 3 H) 2.71 (s, 3 H) | |
| 1.094 | 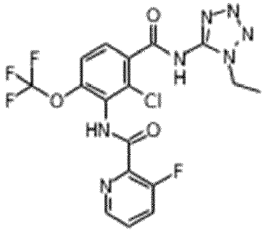 | 1HNMR(20-59780-11HNMR,400MHz,methanol)8.59(d,J=4.4Hz,1H),7.87-7.78(m,2H),7.76-7.70(m,1H), 7.63-7.58(m,1H),4.44(q,J=7.3Hz,2H),1.58(t,J=7.3Hz,3H) | |
| 1.095 | 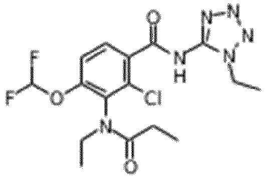 | 1H NMR (CHLOROFORM-d) δ ppm7.83 (d, 1H), 7.48 (d, 1H), 6.74 (t, 1H), 4.61 (q, 2H), 3.81- 3.88 (m, 2H), 2.07 - 2.13 (m, 2H), 1.70 (t, 3H), 1.31 (t, 3H), 1.08 (t, 3H) | |
| 1.096 | 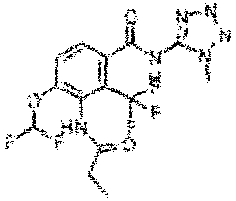 | 1H NMR (DMSO-d6) δ ppm 11.98 (br s, 1H), 9.70 (s, 1H), 7.85 (br d, 1H), 7.70 (br d, 1H), 7.23 (t, 1H), 3.99 (s,3H),2.35 (q, 2H), 1.11 (t, 3H) | |
| 1.097 | 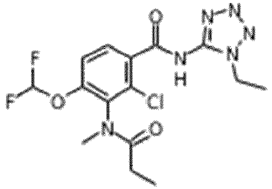 | 1H NMR (400 MHz, CHLOROFORM-d) δ ppm 1.08 (t, 3H) 1.63 (t, 3H) 1.96-2.01 (m, 2H) 3.19 (s, 3H) 4.49 (q, 2H) 6.68 (t, 1H) 7.40 (d, 1H), 7.82 (d, 1H), 11.13 (s, 1 H) | |
| 1.098 | 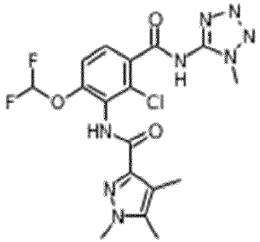 | 1H NMR (400 MHz, METHANOL-d4) δ ppm 2.22 (s, 3 H) 2.26 (s, 3 H) 3.87 (s, 3 H) 4.07 (s, 3 H) 6.90 (t, 1 H) 7.40 (d, 1 H) 7.72 (d, 1 H) | |
| 1.099 | 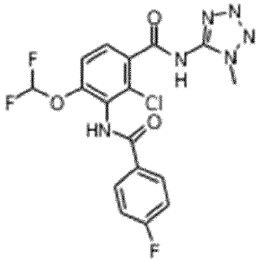 | 1H NMR (METHANOL-d4) δ ppm 8.06 (br dd, 2H), 7.76 (d, 1H), 7.42 (dd, 1H) 7.28 (br t, 2 H), 6.90 (t, 1H), 4.61 (s, 3H) | |
| 1.100 | 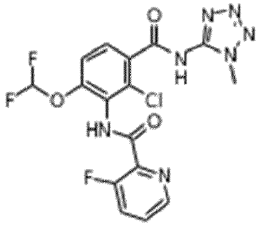 | 1H NMR (400 MHz, DMSO-d6) δ ppm 3.98 (s, 3H) 7.27 (t, 1H) 7.45 (d, 1H) 7.76 (m, 2H) 7.96 (t, 1H) 8.61 (d, 1H) 10.50 (s, 1H) 11.91 (s, 1 H) | |
| 1.101 | 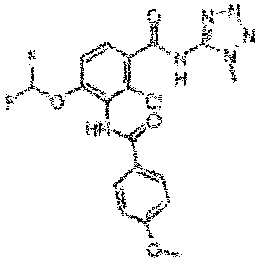 | 1H NMR (400 MHz, CHLOROFORM-d) δ ppm 3.91 (s, 3 H) 4.10 (s, 3 H) 6.61 (t, 1 H) 7.02 (d, 2 H) 7.28-7.36 (m, 1 H) 7.73 (br s, 2 H) 7.91 (d, 2 H) 10.40 (s, 1 H) | |
| 1.102 | 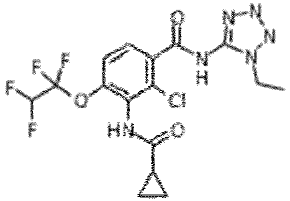 | 1H NMR (400MHz,methanol): 0.84-1.01(m,4H), 1.57(t,J=7.34Hz,3H), 1.82-1.94(m,1H), 4.37-4.49(m,2H), 6.17-6.51(m,1H), 7.50-7.57(m,1H), 7.67-7.74(m,1H) | |
| 1.103 | 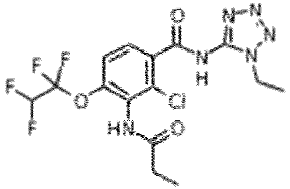 | 1H NMR(400MHz,methanol): 1.23(t,3H), 1.48-1.63(m,3H), 2.39-2.54(m,2H), 4.35-4.48(m,2H), 6.18-6.54(m,1H), 7.54(d, 1H), 7.67-7.76(m,1H) | |
| 1.104 | 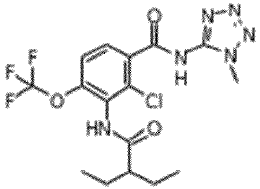 | 1H NMR (400MHz, DMSO-d6) 11.93 (s, 1H), 9.92 (s, 1H), 7.81 (d, 1H), 7.62 (d, 1H), 4.00 (s, 3H), 2.34 (m, 1H), 1.42-1.65 (m, 4H), 0.93 (t, 6H) | |
| 1.105 | 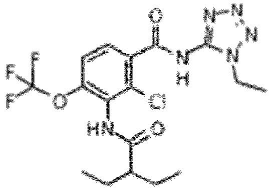 | 1H NMR (400 MHz, methanol) δ ppm 7.75 (d, 1 H), 7.56 (dq, 1 H), 4.43 (q, 2 H), 2.40 (tt, 1 H), 1.66- 1.77 (m, 2 H), 1.53 - 1.65 (m, 5 H), 1.04 (t, 6 H) | |
| 1.106 | 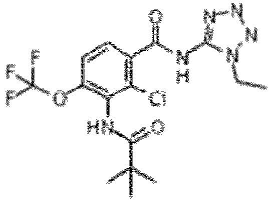 | 1H NMR (400MHz,chloroform) δ ppm 7.67(d, 1H), 7.31-7.35 (m, 2H), 4.44 (q, 2H), 1.60 (t, 3H), 1.35 (s, 9H) | |
| 1.107 | 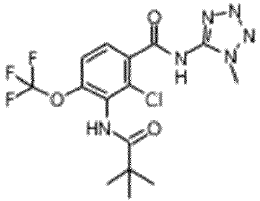 | 1H NMR (400MHz, chloroform) δ ppm 7.68 (d,1H), 7.31-7.37 (m, 2H), 4.08 (s, 3H), 1.35 (s, 9H) | |
| 1.108 | 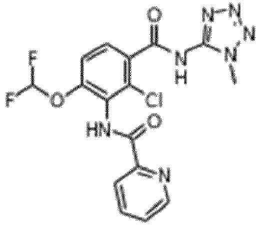 | 1H NMR (METHANOL-d4) δ ppm 8.74 (d, 1H) 8.30 (d, 1H) 8.20-8.25 (m, 1H) 7.78 (dd, 1H) 7.70 (d, 1H) 7.37 (d, 1H) 6.86 (t, 1H) 3.98 (s, 3H) | |
| 1.109 | 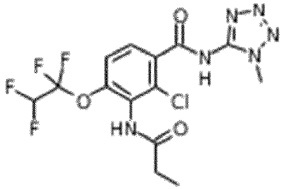 | 1H NMR(400MHz,methanol): 1.16-1.28(m,3H), 2.41-2.53(m,2H), 4.06(s,3H), 6.21-6.53(m,1H), 7.49-7.57(m,1H), 7.73(d,1H) | |
| 1.110 | 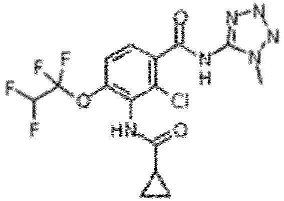 | 1H NMR(400MHz,methanol): 0.85-1.03(m,4H), 1.82-1.95(m,1H), 4.06(s,3H), 6.18-6.51(m,1H), 7.48-7.57(m,1H), 7.67-7.75(m,1H) | |
| 1.111 | 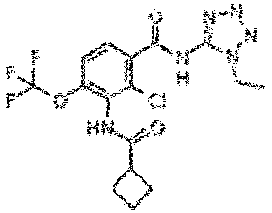 | 1H NMR (400MHz, methanol) δ ppm 7.74 (d, 1H), 7.54 (dq, 1H), 4.43 (q, 2H), 3.41 (quin, 1H), 2.33-2.46 (m, 2H), 2.23-2.33 (m, 2H), 2.01-2.15 (m, 1H), 1.89-2.00 (m,1H), 1.58 (t, 3H) | |
| 1.112 | 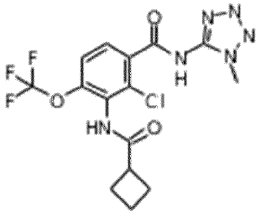 | 1H NMR (400MHz, methanol) δ ppm 7.75(d, 1H), 7.54(d, 1H), 4.07 (s, 3H), 3.41 (quin, 1H), 2.22-2.47 (m, 4H), 2.01-2.15 (m, 1H), 1.88-2.00 (m,1H) | |
| 1.113 | 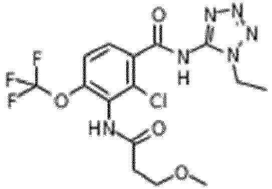 | 1H NMR (400MHz,methanol) 7.75 (d, 1H), 7.58-7.51 (m, 1H), 4.43 (q, 2H), 3.75 (t, 2H), 3.38 (s, 3H), 2.71 (t, 2H), 1.58 (t, 3H) | |
| 1.114 | 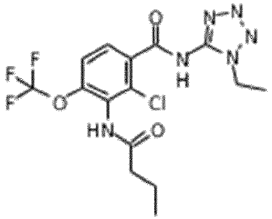 | 1H NMR (400MHz, methanol) 7.75 (d, 1H), 7.57-7.52 (m, 1H), 4.43 (q, 2H), 2.45 (t, 2H), 1.78 (sxt, 2H), 1.58 (t, 3H), 1.05 (t, 3H) | |
| 1.115 | 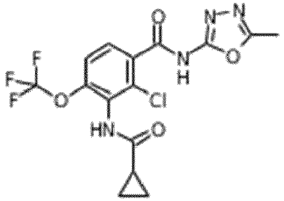 | 1H NMR (400 MHz, methanol) δ ppm 7.66 (d, 1 H), 7.50 (dq, 1 H), 2.51 (s, 3 H), 1.86- 1.95 (m, 1 H), 0.89 - 1.00 (m, 4H) | |
| 1.116 | 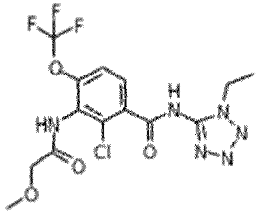 | 1H NMR (400MHz, methanol) 7.78 (d, 1H), 7.59-7.53 (m, 1H), 4.43 (q, 2H), 4.14 (s, 2H), 3.54 (s, 3H), 1.58 (t, 3H) | |
| 1.117 | 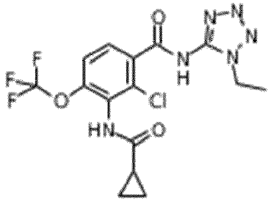 | 1H NMR (400MHz, methanol) δ ppm 7.73 (d, 1H), 7.53 (dq, 1H), 4.43 (q, 2H), 1.86-1.97 (m, 1H), 1.58 (t, 3H), 0.86-1.03 (m, 4H) | |
| 1.118 | 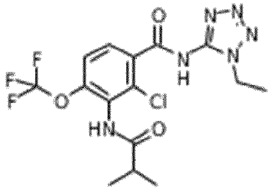 | 1H NMR(400MHz,methanol): 7.74(d,1H), 7.57-7.52(m,1H), 4.43(q,2H), 2.77(septet,1H), 1.58(t,3H), 1.26(d,6H) | |
| 1.119 | 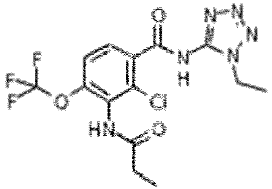 | 1H NMR (400MHz, methanol) 7.74 (d, 1H), 7.54 (m, 1H), 4.42 (q, 2H), 2.48 (q, 2H), 1.57 (t, 3H), 1.25 (t, 3H) | |
| 1.120 | 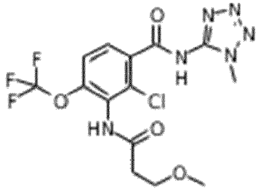 | 1H NMR (400MHz, methanol) 7.75 (d, 1H), 7.57-7.51 (m, 1H), 4.06 (s, 3H), 3.75 (t, 2H), 3.38 (s, 3H), 2.71 (t, 2H) | |
| 1.121 | 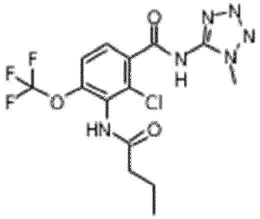 | 1H NMR (400MHz, methanol) 7.75 (d, 1H), 7.57-7.52 (m, 1H), 4.07 (s, 3H), 2.45 (t, 2H), 1.77 (sxt, 2H), 1.05 (t, 3H) | |
| 1.122 | 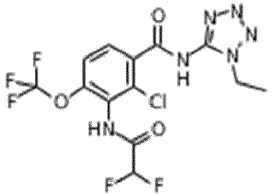 | 1H NMR(400MHz,methanol): 1.52-1.63(m,3H), 4.35-4.49(m,2H), 6.11-6.48(m,1H), 7.56-7.63(m,1H), 7.76-7.87(m,1H) | |
| 1.123 | 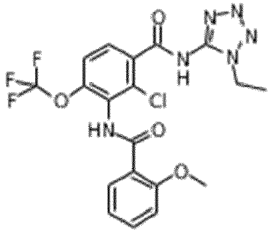 | 1H NMR(400MHz,methanol): 1.49-1.65(m,3H), 4.05(s,3H), 4.44(m,2H), 7.12(t,1H), 7.24(d, 1H), 7.58(d,2H), 7.78(d,1H), 7.93-8.02(m,1H) | |
| 1.124 | 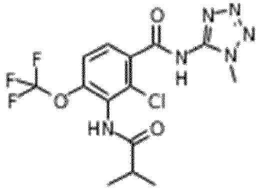 | 1H NMR (400MHz, methanol) 7.75 (d, 1H), 7.56-7.51 (m, 1H), 4.06 (s, 3H), 2.77 (spt, 1H), 1.25 (d, 6H) | |
| 1.125 | 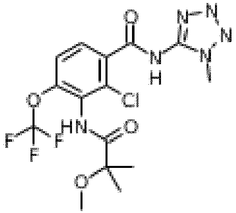 | 1H NMR (400 MHz, methanol) 7.79 (d, 1H), 7.57 (m, 1H), 4.08 (s, 3H), 3.45 (s, 3H), 1.50 (s, 6H) | |
| 1.126 | 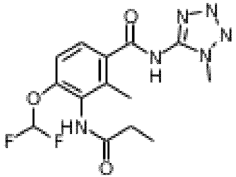 | 1H NMR (400 MHz, DMSO-d6) δ ppm 11.61 (br s, 1 H) 9.45 (s, 1 H) 7.65 (br d, 1 H) 7.21 (br d, 1 H) 7.13 (t, 1 H) 3.97 (s, 3 H) 2.35 (q, 2 H) 2.26 (s, 3 H) 1.12 (t, 3 H) | |
| 1.127 | 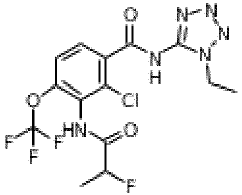 | 1H NMR (400 MHz, acetonitrile) 8.54 (br s, 1H), 7.78 (d, 1H), 7.56 (m, 1H), 5.26 (m, 1H), 4.37 (m, 2H), 1.65 (m, 3H), 1.56 (m, 3H) | |
| 1.128 | 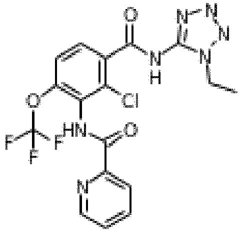 | 1H NMR (400 MHz, methanol) 8.78 (m, 1H), 8.22 (m, 1H), 8.07 (m, 1H), 7.83 (d, 1H), 7.68 (m, 1H), 7.63 (m, 1H), 4.46 (m, 2H), 1.60 (m, 3H) | |
| 1.129 | 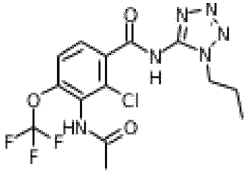 | 1H NMR (400 MHz, methanol) 7.73 (d, 1H), 7.54 (m, 1H), 4.36 (m, 2H), 2.20 (s, 3H), 1.99 (m, 2H), 0.97 (m, 3H) | |
| 1.130 | 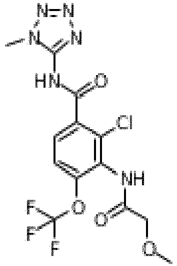 | 1H NMR (400MHz, methanol) 7.78 (d, 1H), 7.59-7.53 (m, 1H), 4.14 (s, 2H), 4.06 (s, 3H), 3.54 (s, 3H) | |
| 1.131 | 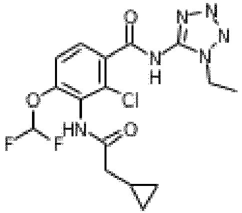 | 1H NMR(Methanol): 7.69(d,1H), 7.38(d,1H), 6.89(m,1H), 4.42(m,2H), 2.35(d,2H), 1.57(m,3H), 1.21-1.09(m,1H), 0.65-0.56(m,2H), 0.34-0.27(m,2H) | |
| 1.132 | 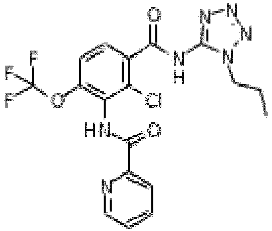 | 1H NMR(400MHz,methanol): 8.76(dd,1H), 8.20(d,1H), 8.05(dt,1H), 7.80(d,1H), 7.66(ddd,1H), 7.64-7.59(m,1H), 4.38(t,2H),2.06-1.95(m,2H), 0.98(t,3H) | |
| 1.133 | 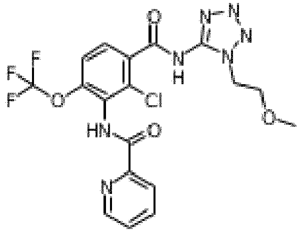 | 1H NMR (400MHz,acetonitrile): 9.80(brs,1H),8.71(d,1H), 8.18(d,1H), 8.03(dt,1H), 7.75(d,1H), 7.65(ddd,1H), 7.60-7.54(m,1H), 4.55(t,2H), 3.83-3.75(m,2H), 3.29(s,3H) | |
| 1.134 | 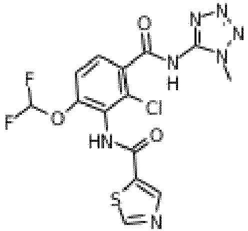 | 1H NMR (400 MHz, METHANOL-d4) δ ppm 9.56 (s, 1 H) 9.39 (dd, 1 H) 8.10 (br d, 1 H) 7.71 (d, 1 H) 7.37 (d, 1 H) 6.87 (t, 1 H) 3.97 (s, 3 H) | |
| 1.135 | 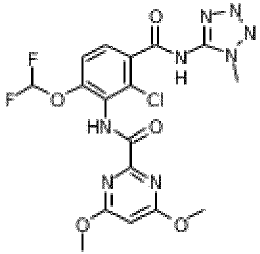 | 1H NMR (400 MHz, METHANOL-d4): 7.68 (d, 1 H) 7.35 (d, 1 H) 6.86 (t, 1 H) 6.26 (s, 1 H) 3.98 (s, 3H) 3.97 (s, 6 H) | |
| 1.136 | 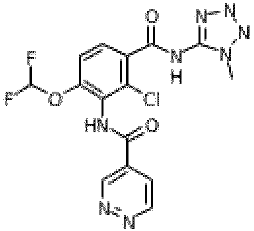 | 1H NMR (400 MHz, DMSO-d6) δ ppm 9.56 (s, 1 H) 9.39 (dd, 1H) 8.10 (dd, 1 H) 7.71 (d, 1 H) 7.37 (d, 1 H) 6.87 (t, 1 H) 3.97 (s, 3 H) | |
| 1.137 | 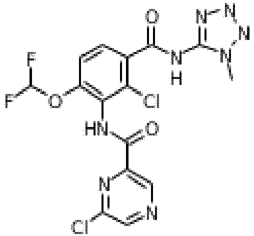 | 1HNMR(400MHz,DMSO-d6): 3.98(s,3H), 7.30(d,1H), 7.46(t,1H), 7.85(d,1H), 9.11(s,1H), 9.23(d,1H), 10.74(s,1H), 11.92(s,1H) | |
| 1.138 | 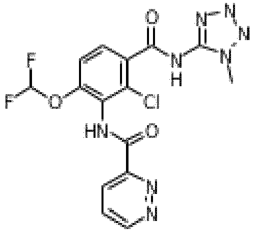 | 1H NMR (400 MHz, DMSO-d6) δ ppm 11.98 (br s, 1 H) 11.14 (s, 1 H) 9.53 (dd, 1 H) 8.32 (dd, 1 H) 8.01 (dd, 1 H) 7.86 (d, 1 H) 7.49 (d, 1H) 7.31 (t, 1H) 4.01 (s, 3 H) | |
| 1.139 | 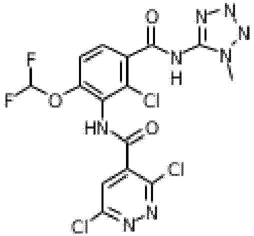 | 1H NMR (400 MHz, DMSO-d6) δ ppm 11.94 (br s, 1H) 10.94 (br s, 1 H) 8.24 (s, 1H), 7.88 (d, 1H) 7.51 (d, 1H) 7.41 (t, 1H) 4.02 (s, 3H) | |
| 1.140 | 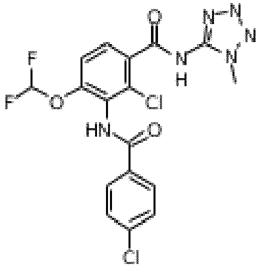 | 455.2 | 1.92 |
| 1.141 | 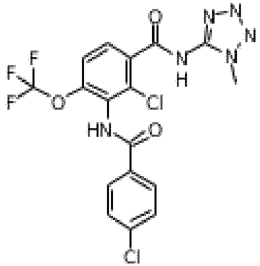 | 473.2 | 2.04 |
| 1.142 | 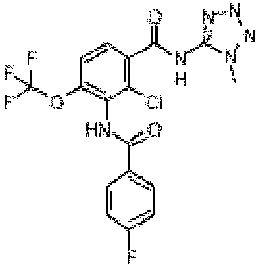 | 457.2 | 1.88 |
| 1.143 | 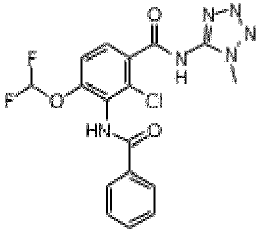 | 421.2 | 1.69 |
| 1.144 | 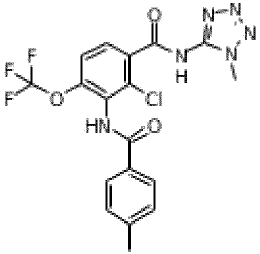 | 453.2 | 1.96 |
| 1.145 | 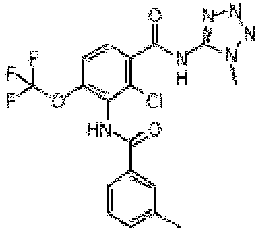 | 453.2 | 1.97 |
| 1.146 | 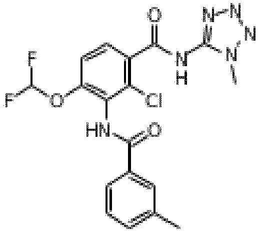 | 435.3 | 1.85 |
| 1.147 | 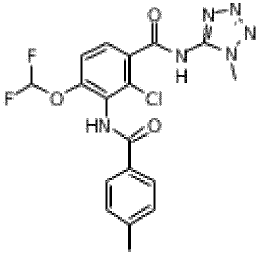 | 435.3 | 1.84 |
| 1.148 | 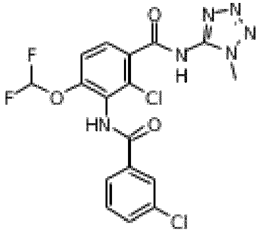 | 455.2 | 1.91 |
| 1.149 | 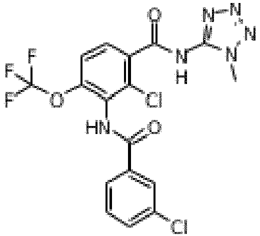 | 473.2 | 2.04 |
| 1.150 | 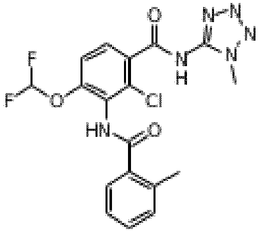 | 435.3 | 1.79 |
| 1.151 | 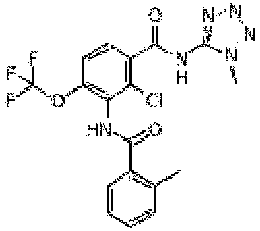 | 453.2 | 1.91 |
| 1.152 | 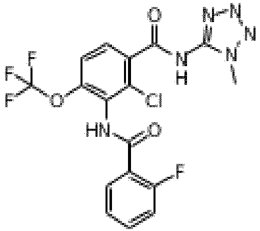 | 457.2 | 1.86 |
| 1.153 | 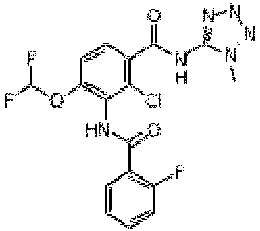 | 439.2 | 1.73 |
| 1.154 | 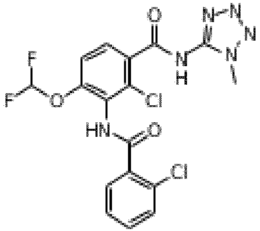 | 455.2 | 1.75 |
| 1.155 | 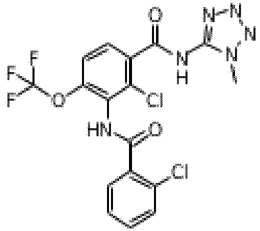 | 473.2 | 1.88 |
| 1.156 | 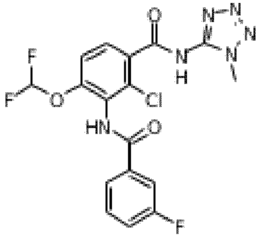 | 439.2 | 1.77 |
| 1.157 | 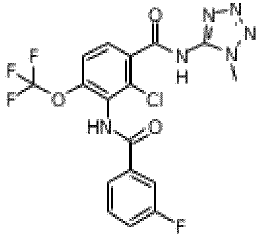 | 457.2 | 1.9 |
| 1.158 | 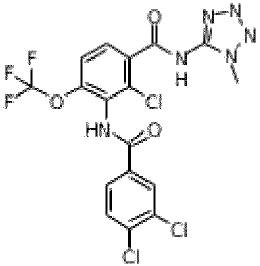 | 507.1 | 2.23 |
| 1.159 | 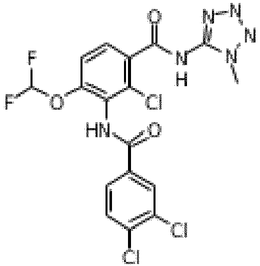 | 489.2 | 2.1 |
| 1.160 | 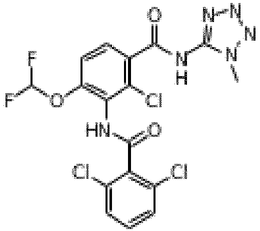 | 489.1 | 1.81 |
| 1.161 | 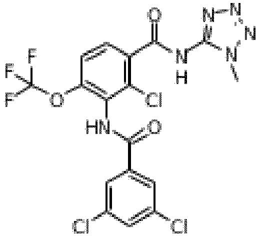 | 507.1 | 2.28 |
| 1.162 | 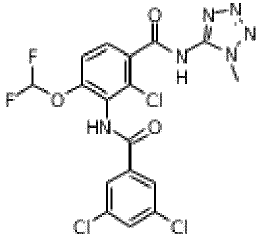 | 489.1 | 2.15 |
| 1.163 | 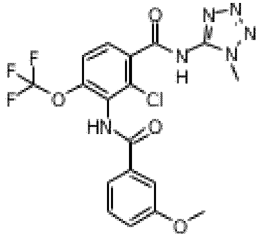 | 469.2 | 1.87 |
| 1.164 | 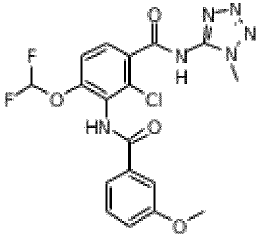 | 451.2 | 1.75 |
| 1.165 | 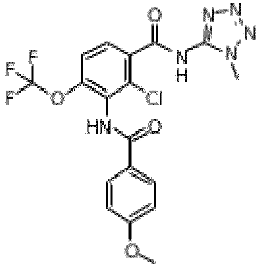 | 469.2 | 1.84 |
| 1.166 | 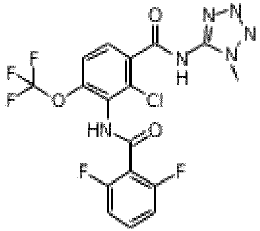 | 475.2 | 1.81 |
| 1.167 | 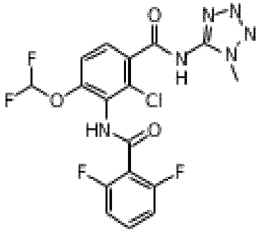 | 457.2 | 1.67 |
| 1.168 | 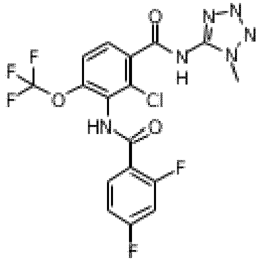 | 475.2 | 1.92 |
| 1.169 | 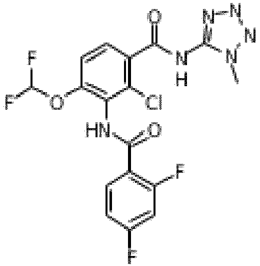 | 457.2 | 1.8 |
| 1.170 | 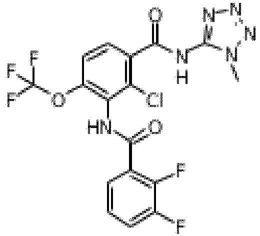 | 475.2 | 1.92 |
| 1.171 | 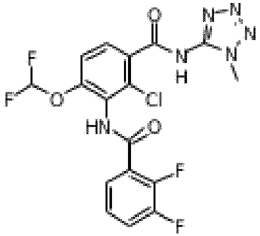 | 457.2 | 1.78 |
| 1.172 | 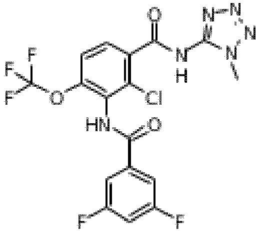 | 475.2 | 1.99 |
| 1.173 | 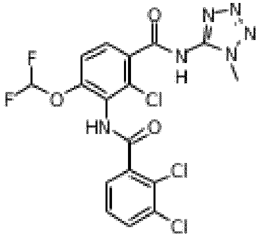 | 489.2 | 1.93 |
| 1.174 | 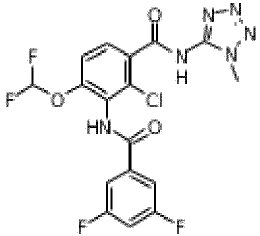 | 457.2 | 1.86 |
| 1.175 | 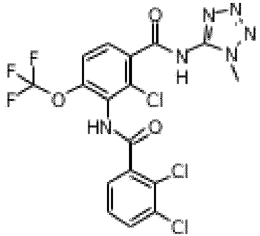 | 507.1 | 2.05 |
| 1.176 | 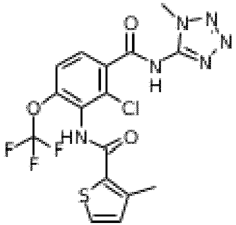 | 1H NMR (400 MHz, DMSO-d6) δ ppm 7.66 (d, 2 H) 11.99 (br s, 1 H) 10.04 (s, 1H) 7.88 (d, 1 H) 4.01 (s, 3 H) 2.47 (s, 3 H) | |
| 1.177 | 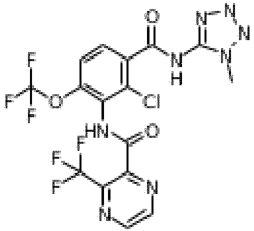 | 1HNMR (400MHz,DMSO-d6): 4.01(s,3H), 7.68-7.72(d,1H), 7.92(d,1H), 9.08(d,1H), 9.16(d,1H), 11.06(s,1H), 12.01(brs,1H) | |
| 1.178 | 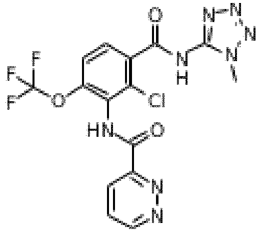 | 1H NMR (400 MHz, DMSO-d6) δ ppm 12.05 (br s, 1H) 11.33 (br s, 1H) 9.54 (dd,1 H) 8.33 (dd, 1 H) 8.01 (dd, 1H) 7.89 (d, 1 H) 7.68 (br d, 1 H) 3.99 (s, 3 H) | |
| 1.179 | 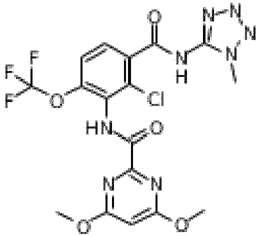 | 1H NMR (400 MHz, DMSO-d6): 12.03 (br s, 1H) 10.59 (s, 1 H) 7.90 (br d, 1 H), 7.69 (br d, 1 H), 6.52 (s, 1 H), 4.06 (s, 3H), 4.04 (s, 6H) | |
| 1.180 | 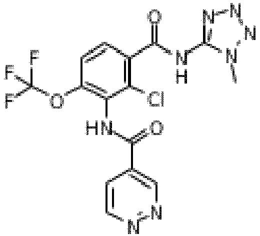 | 1H NMR (400 MHz, DMSO-d6): 3.93 (s, 3 H) 7.48 - 7.76 (m, 1 H) 7.68 (br s, 1 H) 7.86 (br d, 1 H) 8.16 (br s, 1 H) 9.57 (br d, 1 H) 9.67 (br s, 1 H) 10.63 - 11.20 (m | |
| 1.181 | 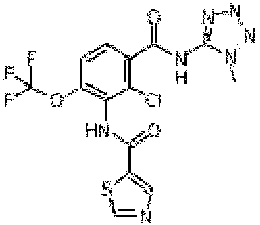 | 1H NMR (400 MHz, DMSO-d6, 80 °C): 10.35 (br s, 1 H) 9.30 (s, 1 H) 8.67 (s, 1 H) 7.82 (br d, 1 H) 7.52 (d, 1 H) 3.87 (s, 3H) | |
| 1.182 | 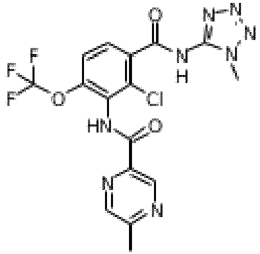 | 1HNMR(400MHz,methanol): 9.18(d,1H), 8.70(d,1H),7.83(d,H), 7.61(qd,1H), 4.07(s,3H), 2.70(s,3H) | |
| 1.183 | 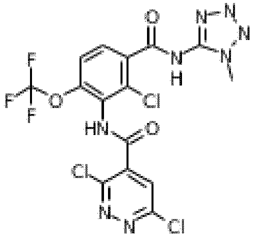 | 1H NMR (400 MHz, DMSO-d6) δ ppm 12.01 (br s, 1 H) 11.15 (br s, 1 H) 8.27 (s, 1 H) 7.95 (d, 1 H) 7.75 (d, 1H) 4.03 (s, 3 H) | |
| 1.184 | 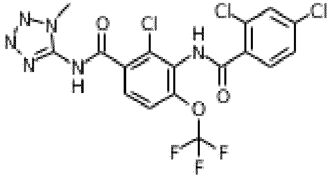 | 507.1 | 2.1 |
| 1.185 | 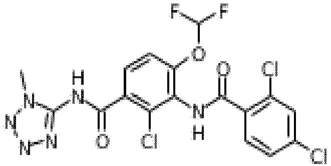 | 489.1 | 1.98 |
| 1.186 | 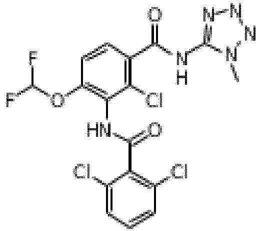 | 489.1 | 1.81 |
| 1.187 | 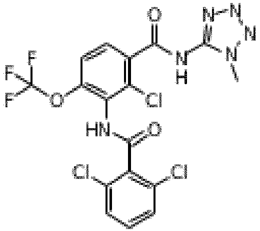 | 507.1 | 1.94 |
| 1.188 | 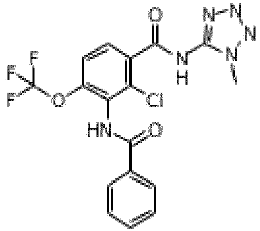 | 1H NMR (400 MHz, methanol) 8.03 - 7.95 (m, 2H), 7.81 (d, 1H), 7.68 - 7.62 (m, 1H), 7.62 - 7.52 (m, 3H), 4.07 (s, 3H) | |
| 1.189 | 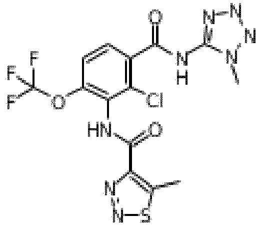 | 461.1 | 1.81 |
| 1.190 | 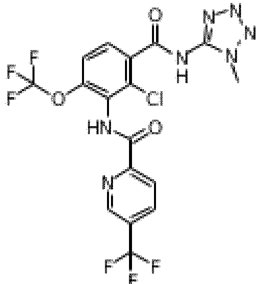 | 508.6 | 2.15 |
| 1.191 | 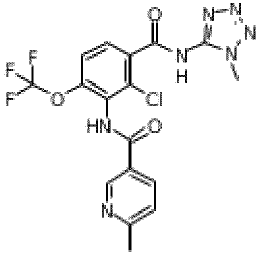 | 454.2 | 1.22 |
| 1.192 | 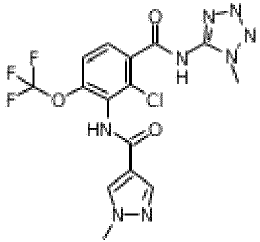 | 443.2 | 1.38 |
| 1.193 | 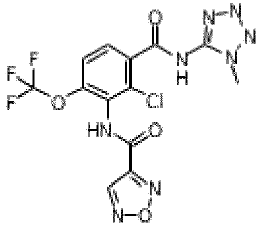 | 431.2 | 1.73 |
| 1.194 | 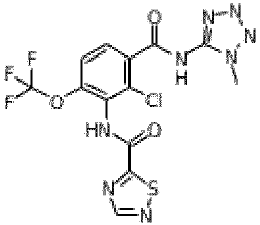 | 447.1 | 1.71 |
| 1.195 | 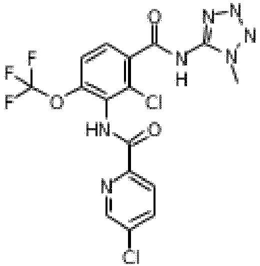 | 474.1 | 2.05 |
| 1.196 | 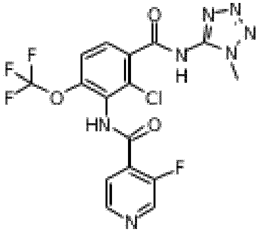 | 458.1 | 1.54 |
| 1.197 | 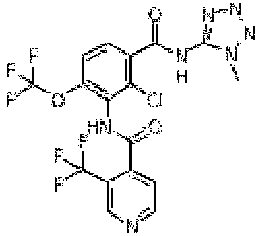 | 509.2 | 1.72 |
| 1.198 | 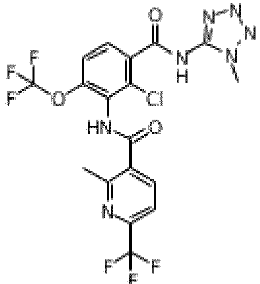 | 522.1 | 2.01 |
| 1.199 | 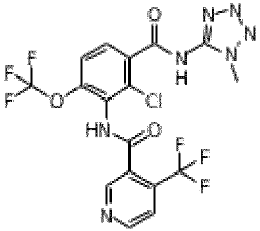 | 508 | 1.73 |
| 1.200 | 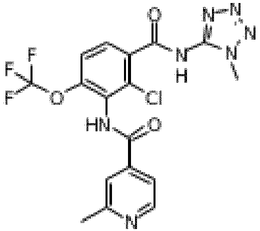 | 454.2 | 1.19 |
| 1.201 | 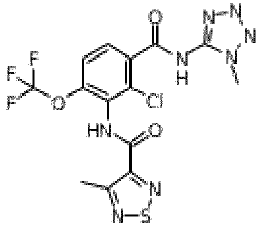 | 461.1 | 1.88 |
| 1.202 | 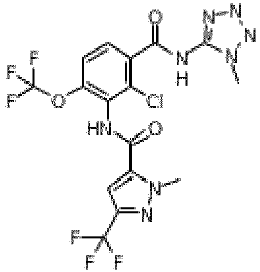 | 511.1 | 2.07 |
| 1.203 | 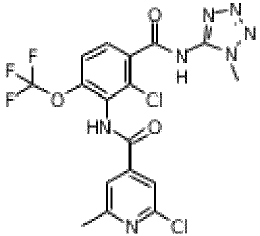 | 488.1 | 1.88 |
| 1.204 | 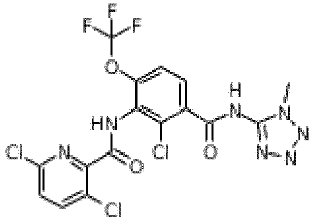 | 510.1 | 2 |
| 1.205 | 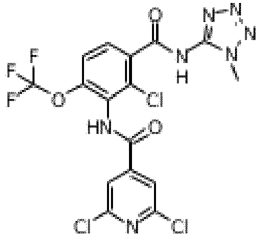 | 510.0 | 2.09 |
| 1.206 | 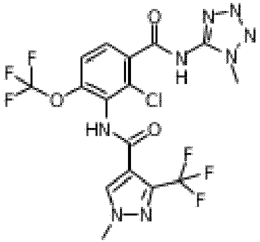 | 513.1 | 1.78 |
| 1.207 | 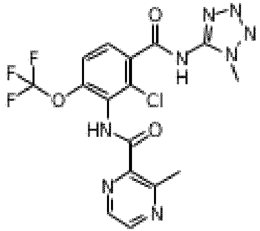 | 455.2 | 1.67 |
| 1.208 | 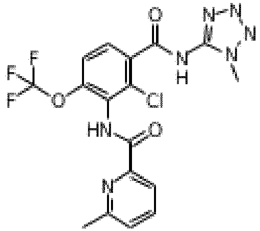 | 456.1 | 1.96 |
| 1.209 | 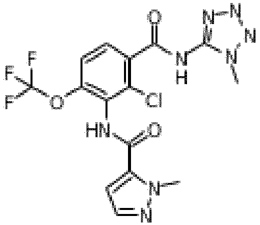 | 443.2 | 1.56 |
| 1.210 | 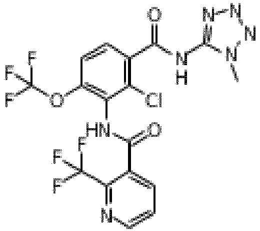 | 508.0 | 1.74 |
| 1.211 | 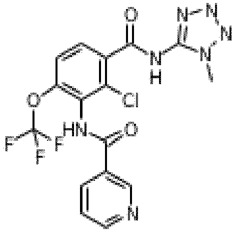 | 441.8 | 1.25 |
| 1.212 | 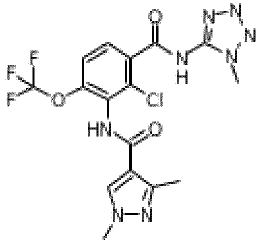 | 459.1 | 1.45 |
| 1.213 | 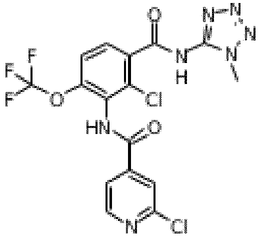 | 474.0 | 1.78 |
| 1.214 | 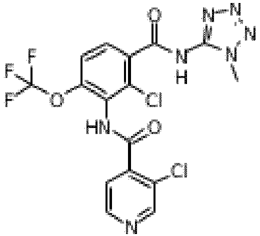 | 474.0 | 1.58 |
| 1.215 | 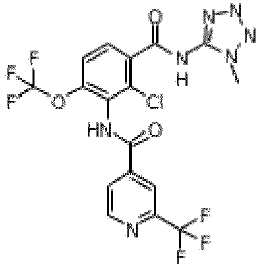 | 508.0 | 1.94 |
| 1.216 | 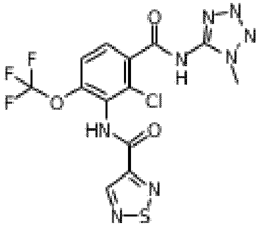 | 447.1 | 1.68 |
| 1.217 | 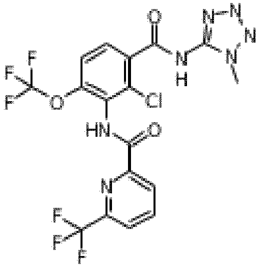 | 510.1 | 2.09 |
| 1.218 | 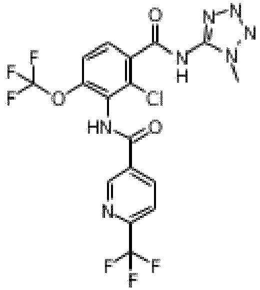 | 508.0 | 1.94 |
| 1.219 | 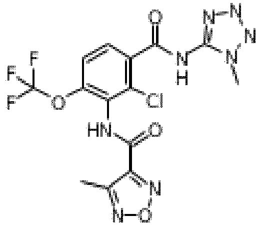 | 445.1 | 1.85 |
| 1.220 | 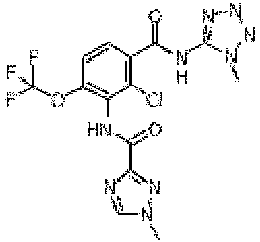 | 444.2 | 1.32 |
| 1.221 | 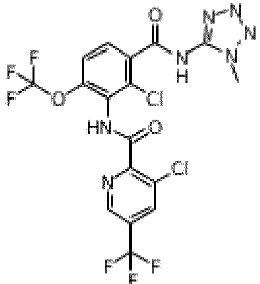 | 542.0 | 2.13 |
| 1.222 | 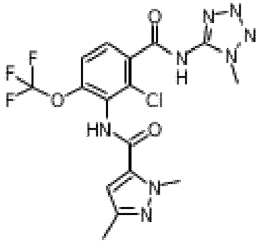 | 457.1 | 1.64 |
| 1.223 | 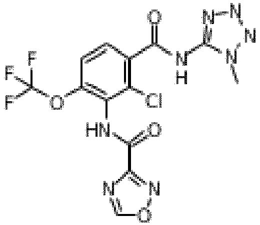 | 431.1 | 1.44 |
| 1.224 | 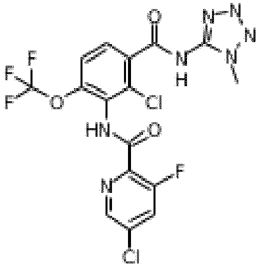 | 492.1 | 1.96 |
| 1.225 | 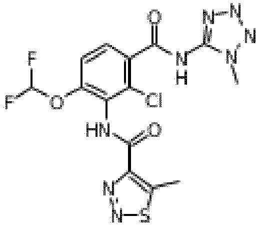 | 443.1 | 1.65 |
| 1.226 | 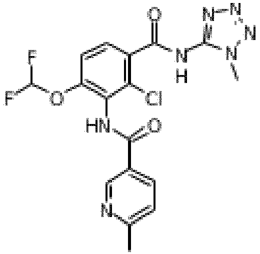 | 436.1 | 1.08 |
| 1.227 | 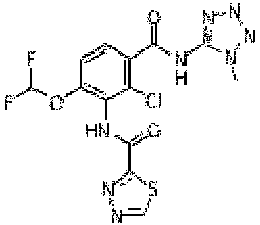 | 429.1 | 1.35 |
| 1.228 | 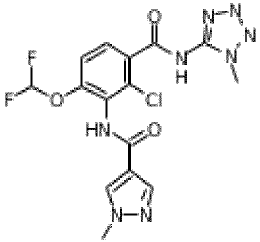 | 425.2 | 1.24 |
| 1.229 | 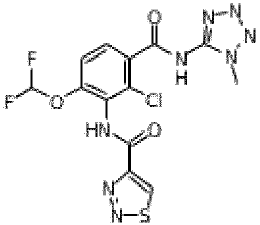 | 429.1 | 1.46 |
| 1.230 | 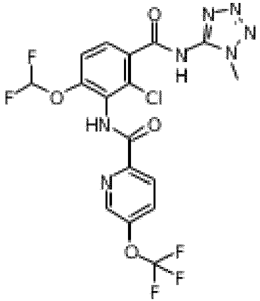 | 507.8 | 2.06 |
| 1.231 | 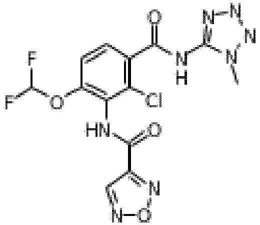 | 413.1 | 1.56 |
| 1.232 | 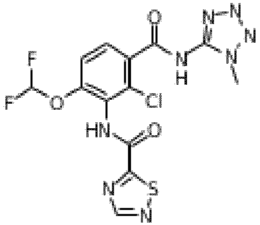 | 429.1 | 1.55 |
| 1.233 | 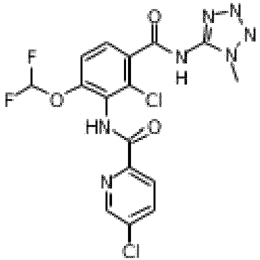 | 456.1 | 1.91 |
| 1.234 | 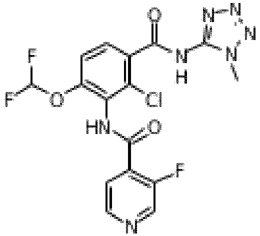 | 440.2 | 1.39 |
| 1.235 | 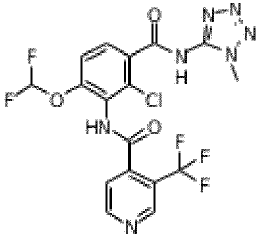 | 492.1 | 1.58 |
| 1.236 | 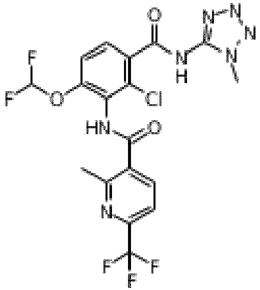 | 505.6 | 1.88 |
| 1.237 | 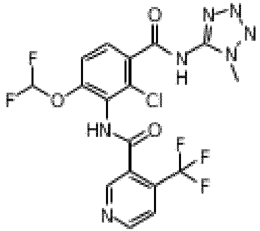 | 491.9 | 1.59 |
| 1.238 | 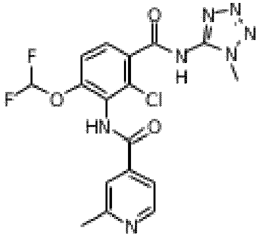 | 436.2 | 1.07 |
| 1.239 | 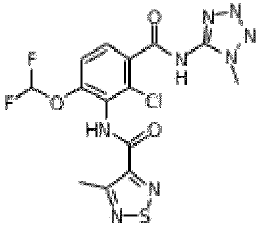 | 443.1 | 1.72 |
| 1.240 | 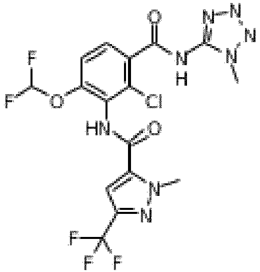 | 495.2 | 1.94 |
| 1.241 | 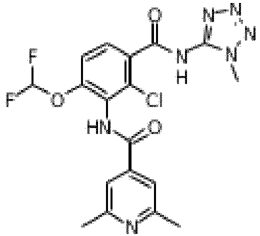 | 450.2 | 1.1 |
| 1.242 | 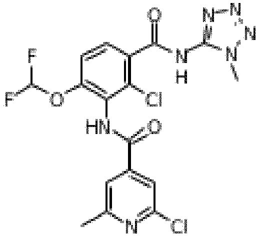 | 470.1 | 1.74 |
| 1.243 | 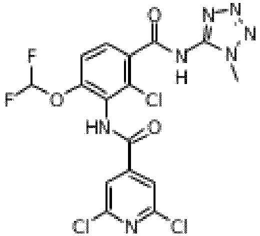 | 490.0 | 1.96 |
| 1.244 | 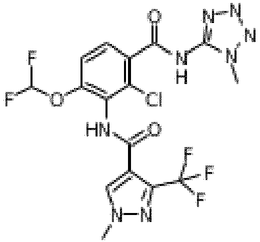 | 493.1 | 1.66 |
| 1.245 | 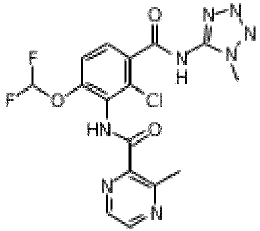 | 438.2 | 1.8 |
| 1.246 | 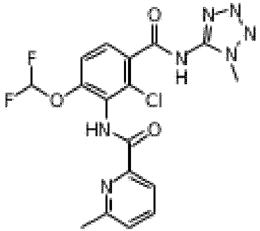 | 437.2 | 1.52 |
| 1.247 | 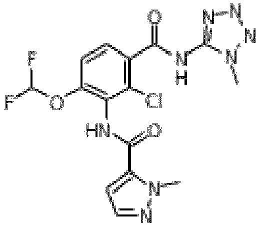 | 425.2 | 1.42 |
| 1.248 | 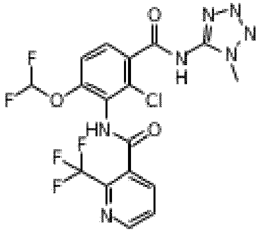 | 491.8 | 1.6 |
| 1.249 | 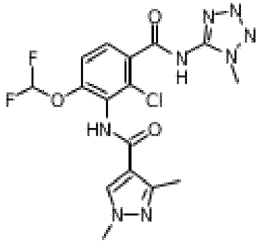 | 439.2 | 1.33 |
| 1.250 | 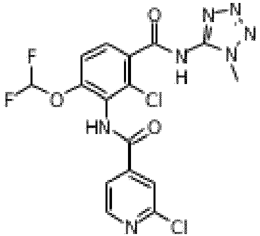 | 456.1 | 1.64 |
| 1.251 | 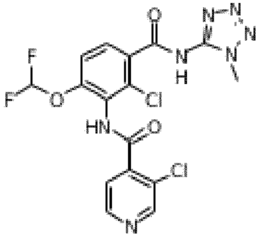 | 456.1 | 1.44 |
| 1.252 | 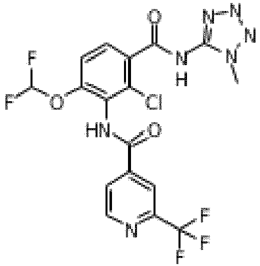 | 490.1 | 1.82 |
| 1.253 | 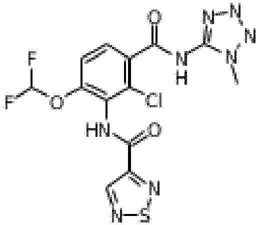 | 429.1 | 1.54 |
| 1.254 | 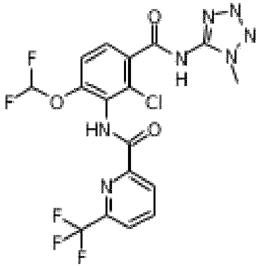 | 492. | 1.97 |
| 1.255 | 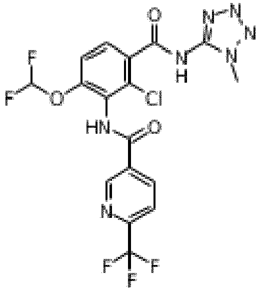 | 492.01 | 1.83 |
| 1.256 | 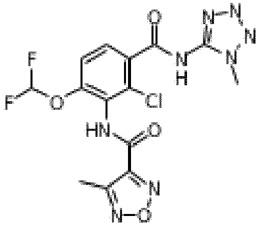 | 427.2 | 1.72 |
| 1.257 | 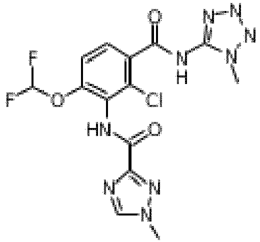 | 426.2 | 1.2 |
| 1.258 | 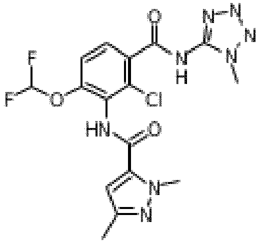 | 439.2 | 1.51 |
| 1.259 | 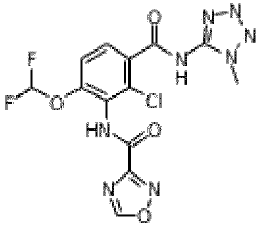 | 413.1 | 1.28 |
| 1.260 | 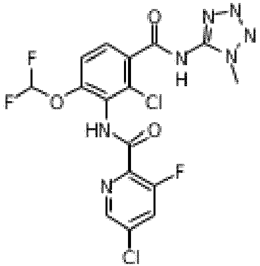 | 474.1 | 1.83 |
| 1.261 | 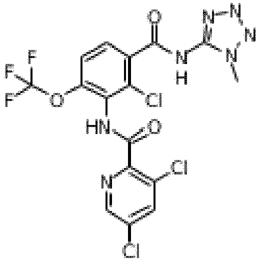 | | |
| 1.262 | 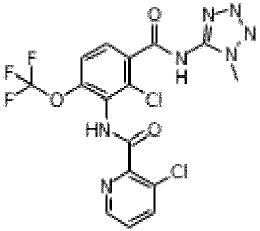 | | |
| 1.263 | 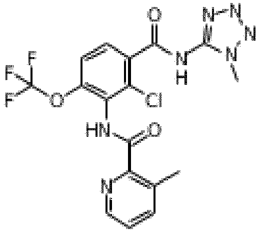 | | |
| 1.264 | 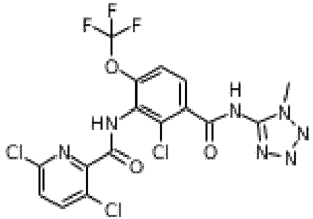 | | |
| 1.265 | 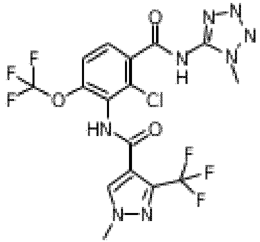 | | |
| 1.266 | 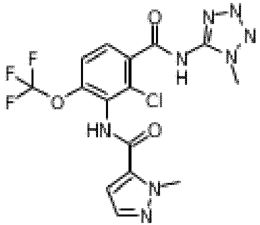 | | |
| 1.267 | 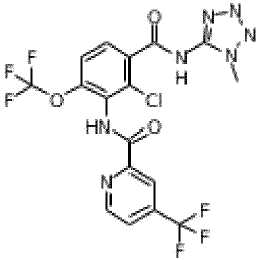 | | |
| 1.268 | 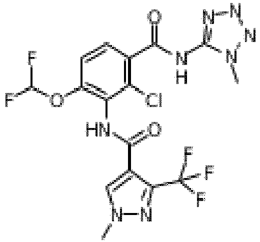 | | |
| 1.269 | 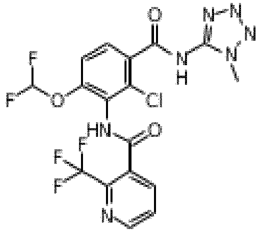 | | |
| 1.270 | 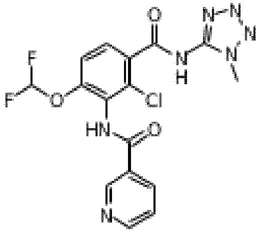 | | |
| 1.271 | 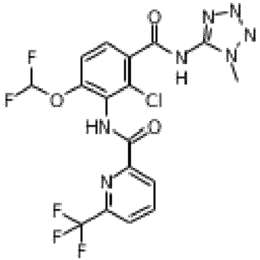 | | |
| 1.272 | 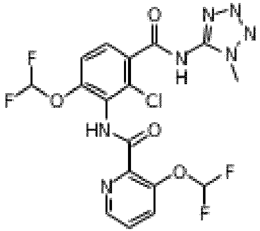 | | |
| 1.273 | 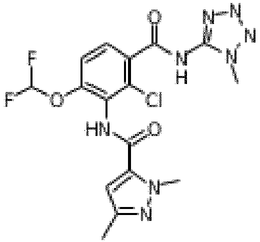 | | |
| 1.274 | 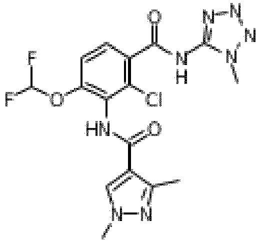 | | |
| 1.275 | 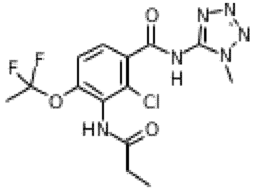 | | |
| 1.276 | 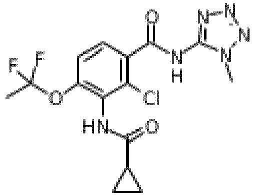 | | |
| 1.277 | 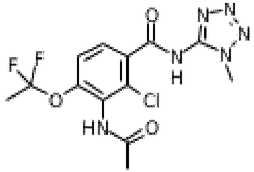 | | |

**TABLE 2 - Examples of herbicidal compounds of the present invention.**

| **Compound Number** | **Structure** | **¹H-NMR** |
|---|---|---|
| 2.001 | 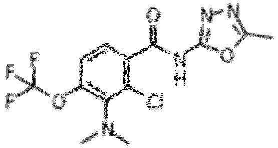 | 1H NMR (Methanol): 7.37-7.34 (m, 2H), 2.90 (s, 6H), 2.51 (s, 3H) |
| 2.002 | 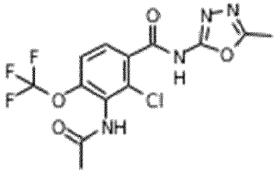 | 1H NMR (Methanol): 7.67 (d, 1H), 7.51 (m, 1H), 2.51 (s, 3H), 2.19 (s, 3H) |
| 2.003 | 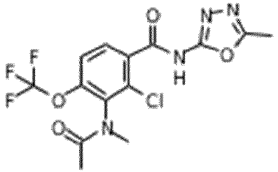 | 1H NMR (Methanol, rotameric): 7.79 (d, 0.8H), 7.72 (d, 0.2H), 7.61 (m, 0.8H), 7.54 (m, 0.2H), 3.35 (s, 0.6H), 3.15 (s, 2.4H), 2.58-2.45 (m, 3H), 2.31 (s, 0.6H), 1.83 (s, 2.4H) |
| 2.004 | 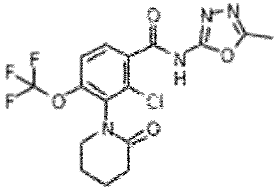 | 1H NMR (Methanol): 7.74 (d, 1H), 7.55 (m, 1H), 3.68-3.59 (m, 1H), 3.57-3.49 (m, 1H), 2.57 (m, 2H), 2.51 (s, 3H), 2.08-1.93 (m, 3H) |
| 2.005 | 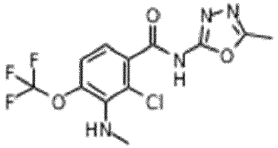 | |
| 2.006 | 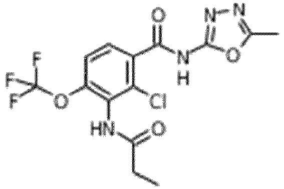 | 1H NMR (400 MHz, methanol) δ ppm 7.67 (d, 1 H), 7.51 (dq, 1 H), 2.51 (s, 3 H), 2.43 - 2.50 (m, 2 H), 1.25 (t, 3 H) |

### Biological Examples

Seeds of a variety of test species are sown in standard soil in pots (*Lolium perenne* (LOLPE), *Amaranthus retoflexus* (AMARE), *Abutilon theophrasti* (ABUTH), *Setaria faberi* (SETFA), *Echinochloa crus-galli* (ECHCG), *Ipomoea hederacea* (IPOHE)). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 500 g/h unless otherwise indicated. The test plants are then grown in a glasshouse under controlled conditions in a glasshouse (at 24/16°C, day/night; 14 hours light; 65 % humidity) and watered twice daily. After 13 days for pre and post-emergence, the test is evaluated for the percentage damage caused to the plant. The biological activities are shown in the following table on a five-point scale (*5* = 80-100%; *4* = 60-79%; *3*=40-59%; *2*=20-39%; *1*=0-19%).

**TABLE B1**

| Compound | POST Application | | | | | PRE Application | | | | |
|---|---|---|---|---|---|---|---|---|---|---|
| | AMARE | ABUTH | SETFA | ECHCG | IPOHE | AMARE | ABUTH | SETFA | ECHCG | IPOHE |
| 1.001 | 5 | 5 | 5 | 5 | 5 | 5 | 5 | 5 | 5 | 5 |
| 1.002 | 5 | 5 | 5 | 5 | 5 | 5 | 5 | 2 | 5 | 1 |
| 1.003 | 5 | 5 | - | 5 | 5 | 5 | 5 | 5 | 5 | 1 |
| 1.004 | 5 | 5 | 5 | 5 | 5 | 5 | 5 | 4 | 3 | 1 |
| 1.005 | 5 | 5 | 5 | 5 | 5 | 5 | 5 | 5 | 5 | 5 |
| 1.006 | 5 | 5 | 5 | 5 | 5 | 5 | 5 | 5 | 5 | 2 |
| 1.007* | 5 | - | 5 | 5 | 4 | 5 | 5 | 5 | 5 | 5 |
| 1.008 | 5 | 5 | 5 | 5 | 5 | 5 | 5 | 4 | 5 | 3 |
| 1.009 | 5 | 5 | 5 | 5 | 5 | 5 | 5 | 5 | 1 | 4 |
| 1.010 | - | 5 | 5 | 5 | 5 | 5 | 5 | 2 | 1 | 2 |
| 1.011 | 5 | 5 | 4 | 5 | 5 | 5 | 4 | 1 | 1 | 4 |
| 1.012* | 4 | 4 | 4 | 3 | 4 | 5 | 5 | 5 | 5 | 4 |
| 1.014* | 4 | 5 | 4 | 4 | 4 | 5 | 5 | 5 | 4 | 5 |
| 1.015 | 5 | 5 | 5 | 5 | 5 | 5 | 5 | 2 | 4 | 1 |
| 1.016 | 5 | 5 | 5 | 5 | 5 | 5 | 5 | 5 | 5 | 5 |
| 1.017 | 5 | 4 | 5 | 5 | 5 | 5 | 5 | 5 | 5 | 5 |
| 1.018 | 5 | 5 | 5 | 5 | 4 | 5 | 4 | 3 | 3 | 3 |
| 1.019* | 5 | 5 | 5 | 5 | 5 | 5 | 5 | 5 | 5 | 5 |
| 1.020* | 5 | 5 | 5 | 5 | 5 | 5 | 5 | 5 | 5 | 5 |
| 1.021* | 3 | 4 | 4 | 4 | 3 | 3 | 5 | 3 | 3 | 3 |
| 1.022* | 5 | 5 | 5 | 5 | 4 | 5 | 5 | 5 | 5 | 4 |
| 1.023* | 5 | 5 | 5 | 5 | 5 | 5 | 5 | 5 | 5 | 2 |
| 1.024* | 3 | 4 | 4 | 4 | 4 | 5 | 5 | 4 | 4 | 4 |
| 1.025* | 5 | 4 | 4 | 5 | 2 | 5 | 5 | 3 | 3 | 4 |
| 1.026* | 4 | 4 | 4 | 5 | 4 | 5 | 5 | 5 | 5 | 4 |
| 1.028 | 5 | 5 | 4 | 3 | 4 | 5 | 5 | 3 | 4 | 3 |
| 1.029 | 5 | 5 | 5 | 5 | 5 | 5 | 5 | 5 | 5 | 4 |
| 1.030 | 5 | 5 | 5 | 5 | 4 | 5 | 5 | 3 | 3 | 3 |
| 1.031* | 4 | 4 | 4 | 4 | 4 | 5 | 4 | 3 | 4 | 3 |
| 1.032 | 5 | 5 | 5 | 5 | 5 | 5 | 5 | 5 | 5 | 3 |
| 1.033 | 5 | 5 | 5 | 5 | 5 | 5 | 5 | 5 | 5 | 5 |
| 1.034 | 5 | 5 | 5 | 5 | 5 | 5 | 5 | 5 | 5 | 4 |
| 1.035* | 4 | 4 | 4 | 4 | 4 | 5 | 5 | 4 | 3 | 3 |
| 1.036* | 5 | 5 | 5 | 5 | 4 | 5 | 5 | 5 | 5 | 4 |
| 1.037* | 5 | 5 | 5 | 5 | 4 | 5 | 5 | 4 | 5 | 4 |
| 1.038* | 4 | - | 4 | - | 4 | 5 | 5 | 4 | 5 | 5 |
| 1.041* | 3 | - | 3 | - | 3 | 2 | 5 | 2 | 3 | 2 |
| 1.042* | 5 | - | 5 | 5 | 5 | 5 | 4 | 5 | 5 | 5 |
| 1.044* | 5 | 3 | 3 | 2 | 4 | 4 | 4 | 2 | 2 | 1 |
| 1.045* | 5 | - | 5 | 5 | 4 | 5 | 5 | 5 | 5 | 4 |
| 1.046* | 5 | - | 5 | 5 | 5 | 5 | 5 | 5 | 5 | 5 |
| 1.052* | 5 | - | 5 | 4 | 4 | 5 | 5 | 5 | 5 | 4 |
| 1.053* | 5 | - | 5 | 3 | 4 | 5 | 5 | 4 | 4 | 4 |
| 1.054* | 5 | 5 | 5 | - | 5 | 5 | 5 | 5 | 5 | 4 |
| 1.055* | 5 | 5 | 5 | 5 | 4 | 5 | 5 | - | 5 | 4 |
| 1.056* | 4 | - | 4 | 4 | 4 | 3 | 5 | 3 | 1 | - |
| 1.057* | 5 | - | 5 | 5 | 4 | 5 | 5 | 4 | 5 | 5 |
| 1.058* | 4 | - | 4 | 5 | 4 | 5 | 5 | 3 | 5 | 5 |
| 1.059* | 5 | - | 4 | 5 | 3 | 5 | 5 | 1 | 4 | 3 |
| 1.060* | 5 | - | 5 | 4 | 4 | 5 | 5 | 4 | 4 | 3 |
| 1.061* | 5 | - | 5 | 4 | 4 | 5 | - | 3 | 3 | 3 |
| 1.062* | 5 | - | 5 | 4 | 4 | 5 | - | 3 | 2 | 4 |
| 1.063* | 5 | 5 | 5 | 5 | 4 | 5 | 5 | 5 | 5 | 5 |
| 1.064* | 4 | - | 4 | - | 4 | 5 | 5 | 4 | 3 | 4 |
| 1.065* | 5 | 5 | 5 | 5 | 5 | 5 | 5 | 5 | 5 | 5 |
| 1.066* | 4 | - | 4 | - | 4 | 5 | 5 | 4 | 5 | 5 |
| 1.067* | 5 | - | 5 | 5 | 4 | 5 | 5 | 5 | 5 | 5 |
| 1.068* | 4 | - | 5 | - | 4 | 5 | 5 | 5 | 5 | 3 |
| 1.069* | 4 | - | 5 | 5 | 4 | 5 | 5 | 5 | 5 | 4 |
| 1.070* | 4 | - | 4 | 4 | 4 | 5 | 5 | 4 | 4 | 4 |
| 1.071* | 4 | - | 5 | 5 | 4 | 5 | 5 | 5 | 5 | 5 |
| 1.072* | 4 | - | 5 | 5 | 4 | 5 | 5 | 4 | 5 | 5 |
| 1.073* | 5 | - | 5 | 5 | 4 | 5 | 5 | 5 | 5 | 5 |
| 1.074* | 5 | - | 5 | 5 | 5 | 5 | 5 | 4 | 4 | 5 |
| 1.075* | 4 | - | 5 | 4 | 4 | 5 | 5 | 5 | 5 | 5 |
| 1.076* | 4 | - | 4 | 5 | 4 | 5 | 5 | 5 | 5 | 5 |
| 1.077* | 5 | - | 5 | 5 | 4 | 5 | 5 | 3 | 5 | 5 |
| 1.078* | 5 | - | 5 | 5 | 4 | 5 | 5 | 4 | 5 | 4 |
| 1.079* | 4 | - | 4 | 5 | 4 | 5 | 5 | 4 | 5 | 4 |
| 1.080* | 4 | - | 4 | - | 4 | 4 | 1 | 1 | 1 | 2 |
| 1.081* | 4 | - | 3 | 3 | 4 | 5 | 5 | 1 | 1 | 1 |
| 1.082* | 4 | 4 | 4 | 3 | 4 | 5 | 5 | 4 | 5 | 3 |
| 1.083* | 4 | 4 | 4 | 2 | 4 | 5 | 5 | 4 | - | 3 |
| 1.084* | 4 | 5 | 3 | 3 | 3 | 5 | 4 | 1 | 1 | 1 |
| 1.085* | 4 | 4 | 4 | 4 | 4 | 4 | 5 | 3 | - | 3 |
| 1.086* | 5 | 4 | 4 | 4 | 5 | 5 | 5 | 4 | | 4 |
| 1.087* | 4 | 4 | 4 | - | 4 | 5 | 4 | 4 | 1 | 4 |
| 1.088* | 4 | 4 | 4 | 3 | 4 | 5 | 4 | 5 | - | 4 |
| 1.089* | 4 | 3 | 4 | 2 | 3 | 5 | 2 | 3 | - | 3 |
| 1.090* | 4 | 4 | 4 | 4 | 4 | 5 | 5 | 4 | 5 | 4 |
| 1.091* | 4 | - | 4 | 5 | 3 | 5 | 5 | 4 | 5 | 3 |
| 1.092* | 3 | - | 4 | - | 4 | 5 | 5 | 5 | - | 3 |
| 1.093* | 4 | 4 | 4 | 5 | 4 | 5 | 5 | 4 | 5 | 4 |
| 1.094 | 5 | - | 5 | 5 | 5 | 5 | 1 | 1 | 1 | 1 |
| 1.095* | 5 | - | 5 | 5 | 4 | 5 | 5 | 4 | 5 | 4 |
| 1.096* | 4 | - | 3 | 4 | 3 | 4 | 3 | 1 | 1 | 3 |
| 1.097* | 4 | - | 5 | 4 | 4 | 5 | 5 | 4 | 5 | 5 |
| 1.098* | 4 | 4 | 4 | 4 | 4 | 3 | 4 | 4 | 1 | 4 |
| 1.099* | 4 | 4 | 3 | 4 | 4 | 5 | 4 | 1 | 1 | 3 |
| 1.100* | 4 | 4 | 4 | 4 | 4 | 5 | 5 | 3 | 1 | 2 |
| 1.102 | 5 | 5 | 5 | 5 | 5 | 5 | 4 | 4 | 5 | 1 |
| 1.101 * | 4 | 4 | 4 | 5 | 4 | 4 | 4 | 4 | 1 | 2 |
| 1.103 | 5 | 5 | 5 | 5 | 5 | 5 | 4 | 1 | 3 | 1 |
| 1.104 | 5 | 5 | 5 | 5 | 5 | 5 | 4 | 3 | 5 | 2 |
| 1.105 | 5 | 5 | 5 | 5 | 5 | 5 | 5 | 3 | 5 | 2 |
| 1.106 | 5 | 5 | 5 | 5 | 5 | 5 | 5 | 5 | 5 | - |
| 1.107 | 5 | 5 | 5 | 5 | 5 | 5 | 5 | 5 | 5 | 4 |
| 1.108* | 5 | 4 | 4 | 5 | 4 | 5 | 4 | 3 | 2 | 2 |
| 1.109 | 5 | - | 5 | - | 5 | 5 | 5 | 4 | 4 | 5 |
| 1.110 | 5 | - | 5 | 5 | 5 | 5 | 3 | 5 | 5 | 1 |
| 1.111 | 5 | 5 | 1 | 5 | 5 | 5 | 4 | 1 | 4 | 1 |
| 1.112 | 5 | 5 | 5 | 5 | 5 | 5 | 4 | 1 | 5 | 1 |
| 1.113 | 5 | 5 | 5 | 5 | 5 | 5 | 5 | 2 | 5 | 1 |
| 1.114 | 5 | 5 | 5 | 5 | 5 | 5 | 5 | 2 | 5 | 3 |
| 1.115* | 3 | 4 | 4 | 4 | 4 | 4 | 4 | 4 | 5 | 2 |
| 1.116 | 5 | 5 | 5 | 5 | 5 | 5 | 5 | 1 | 3 | 1 |
| 1.117 | 5 | 5 | 5 | 5 | 5 | 5 | 5 | 4 | 5 | 4 |
| 1.118* | 4 | 4 | 4 | 4 | 4 | 4 | 4 | 4 | 5 | 2 |
| 1.119 | 5 | 5 | 5 | 5 | 5 | 5 | 5 | 2 | 5 | 4 |
| 1.120 | 5 | 5 | 5 | 5 | 5 | 5 | 4 | 1 | 5 | 1 |
| 1.121 | 5 | 5 | 5 | 5 | 5 | 5 | 4 | 1 | 5 | 2 |
| 1.122* | 5 | 4 | 3 | 4 | 2 | 5 | 3 | 3 | 4 | 1 |
| 1.123* | 4 | 4 | 4 | 4 | 5 | 3 | 3 | 4 | 3 | 2 |
| 1.124 | 5 | 5 | 5 | 5 | 5 | 5 | 4 | 4 | 5 | 1 |
| 1.125 | 5 | 5 | 5 | 5 | 5 | 5 | 5 | 1 | 5 | 4 |
| 1.126* | 5 | 5 | 5 | 5 | 4 | 5 | 5 | 2 | 5 | 3 |
| 1.127 | 5 | 5 | 5 | 5 | 5 | 5 | 5 | 3 | 5 | 2 |
| 1.128 | 5 | 5 | 5 | 5 | 5 | 5 | 4 | 1 | 3 | 1 |
| 1.129 | 5 | 5 | 5 | 5 | 5 | 5 | 5 | 1 | 4 | 2 |
| 1.130 | 5 | 5 | 5 | 5 | 4 | 5 | 4 | 1 | 2 | 1 |
| 1.131 | 5 | 5 | 4 | 5 | 5 | 5 | 5 | 1 | 3 | 1 |
| 1.132 | 5 | 5 | 5 | 5 | 5 | 5 | 4 | 1 | 3 | 4 |
| 2.001 | 5 | 4 | 5 | 5 | 5 | 5 | 4 | 5 | 5 | 3 |
| 2.002 | 5 | 5 | 5 | 5 | 5 | 1 | 3 | 1 | 1 | 1 |
| 2.003 | 5 | 5 | 5 | 5 | 5 | 5 | 5 | 5 | 5 | 4 |
| 2.004 | 5 | 5 | 5 | 5 | 5 | 5 | 4 | 5 | 5 | 1 |

| | | | | | | | | | | |
|---|---|---|---|---|---|---|---|---|---|---|
| *Applied at 125g/ha | | | | | | | | | | |

### TABLE B2 - COMPARATIVE TEST

Seeds of test species were sown in standard soil in pots. After cultivation for one day under controlled conditions in a glasshouse (at 24/16°C, day/night; 14 hours light; 65 % humidity), the plants were sprayed with an aqueous spray solution derived from the formulation of the technical active ingredient in 0.6 ml acetone and 45 ml formulation solution containing 10.6% Emulsogen EL (Registry number 61791-12-6), 42.2% N-methyl pyrrolidone, 42.2% dipropylene glycol monomethyl ether (CAS RN 34590-94-8) and 0.2 % X-77 (CAS RN 11097-66-8).

The test plants were then grown in a glasshouse under controlled conditions in a glasshouse (at 24/16°C, day/night; 14 hours light; 65 % humidity) and watered twice daily. After 14 days, the test was evaluated (100 = total damage to plant; 0 = no damage to plant).

Test species: ABUTH (*Abutilon theophrasti);* BROTE (*Bromus tectorum*); ECHCG (*Echinochloa crus-galli*); SINAR (*Sinapis arvensis*)*.*

| **Compound** | PRE Application | | | | |
|---|---|---|---|---|---|
| C1 | 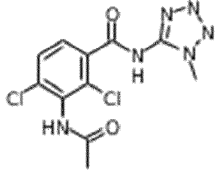 | | | | |
| | Rate g/ha | ABUTH | BROTE | ECHCG | SINAR |
| | 250 | 0 | 0 | 20 | 30 |
| | 130 | 0 | 0 | 30 | 0 |
| | 30 | 0 | 0 | 10 | 0 |
| | | | | | |
| 1.002 | 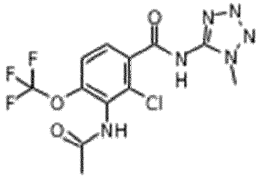 | | | | |
| | Rate g/ha | ABUTH | BROTE | ECHCG | SINAR |
| | 250 | 80 | 70 | 80 | 80 |
| | 130 | 10 | 10 | 30 | 30 |
| | 30 | 0 | 0 | 20 | 10 |
| | | | | | |

| **Compound** | PRE Application | | | | |
|---|---|---|---|---|---|
| 1.007 | 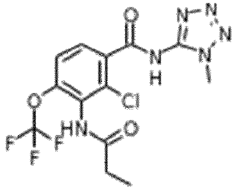 | | | | |
| | Rate g/ha | ABUTH | BROTE | ECHCG | SINAR |
| | 250 | 90 | 20 | 90 | 100 |
| | 130 | 60 | 0 | 70 | 60 |
| | 30 | 0 | 0 | 20 | 10 |
| | | | | | |
| 1.012 | 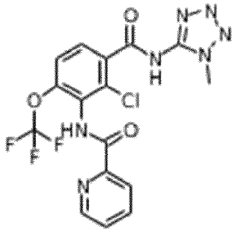 | | | | |
| | Rate g/ha | ABUTH | BROTE | ECHCG | SINAR |
| | 250 | 10 | 70 | 30 | 80 |
| | 130 | 0 | 0 | 30 | 70 |
| | 30 | 0 | 0 | 10 | 0 |
| | | | | | |
| 1.014 | 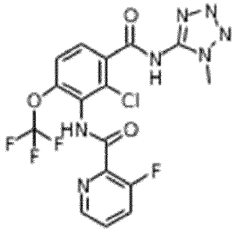 | | | | |
| | Rate g/ha | ABUTH | BROTE | ECHCG | SINAR |
| | 250 | 60 | 30 | 30 | 80 |
| | 130 | 0 | 10 | 40 | 80 |
| | 30 | 0 | 0 | 20 | 20 |

C1 is compound 4-659 disclosed in WO2012/028579. As can be seen, the replacement of the 4-chloro substituent on the phenyl ring with the haloalkoxy group of the present invention provides an unexpected improvement in the weed control observed.

## Claims

1. A compound of Formula (I): 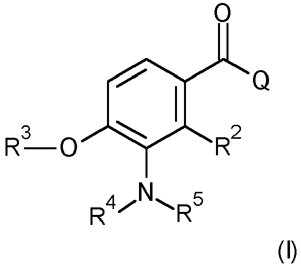
or an agronomically acceptable salt thereof,
wherein:-
Q is Q¹ or Q²; 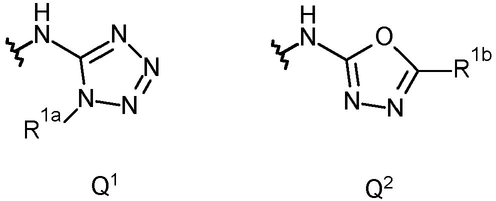
R^{1a} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R^{1b} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is C₁-C₆haloalkyl or C₁-C₆alkyl;
R⁴ is selected from the group consisting of C₁-C₆alkyl-, C₁-C₆ haloalkyl-, C₁-C₆alkyl-C(O)-, C₁-C₆haloalkyl-C(O)-, C₃-C₆cycloalkyl-, C₃-C₆cycloalkyl-C₁-C₃alkyl-, C₃-C₆cycloalkyl-C(O)-, C₁-C₃alkoxy-C₁-C₃alkyl-, C₁-C₃alkoxy-C₁-C₃alkyl-C(O)-, -C(O)-phenyl and -C(O)-heteroaryl wherein the phenyl, heteroaryl or C₃-C₆cycloalkyl is optionally substituted by 1, 2 or 3 substituents selected from the group consisting of halogen, C₁-C₆ alkyl, C₁-C₆ haloalkyl and C₁-C₆ alkoxy;
R⁵ is selected from the group consisting of hydrogen, C₁-C₆alkyl-, C₁-C₆haloalkyl and C₁-C₆cycloalkyl; or
R⁴ and R⁵ together with the nitrogen atom to which they are attached form a 5- or 6-membered saturated heterocycle which is optionally oxo substituted; and
p is 0, 1 or 2.

2. A compound according to claim 1, wherein R^{1a} or R^{1b} are selected from the group consisting of methyl, ethyl and *n*-propyl.

3. A compound according to any one of the previous claims, wherein Q is Q¹ and R^{1a} is methyl.

4. A compound according to claim 1 or claim 2, wherein Q is Q² and R^{1b} is methyl.

5. A compound according to any one of the previous claims, wherein R² is selected from the group consisting of methyl, Cl, -CF₃ and -SO₂methyl.

6. A compound according to claim 5, wherein R² is Cl.

7. A compound according to any one of the previous claims, wherein R³ is -CF₃ or -CHF₂.

8. A compound according to any one of the previous claims, wherein R⁴ is selected from the group consisting of C₁-C₆alkyl-, C₁-C₆alkyl-C(O)- and C₃-C₆cycloalkyl-.

9. A compound according to any one of claims 1 to 7, wherein R⁴ is -C(O)-heteroaryl wherein the heteroaryl is optionally substituted by 1, 2 or 3 substituents selected from the group consisting of halogen, C₁-C₆ alkyl, C₁-C₆ haloalkyl and C₁-C₆ alkoxy.

10. A compound according to any one of the previous claims, wherein R⁵ is hydrogen or C₁-C₆alkyl-.

11. A herbicidal composition comprising a compound according to any one of the previous claims and an agriculturally acceptable formulation adjuvant.

12. A herbicidal composition according to claim 11, further comprising at least one additional pesticide.

13. A herbicidal composition according to claim 12, wherein the additional pesticide is a herbicide or herbicide safener.

14. A method of controlling weeds at a locus comprising application to the locus of a weed controlling amount of a composition according to any one of claims 11 to 13.

15. Use of a compound of Formula (I) as defined in claim 1 as a herbicide.

16. A compound of Formula (II) 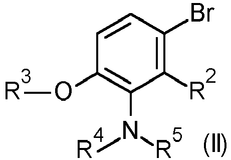 wherein R², R⁴ and R⁵ are as defined in the compound of Formula (I) in any one of claims 1 to 10 above and R³ is C₁-C₆haloalkyl.

17. A compound of Formula (V) 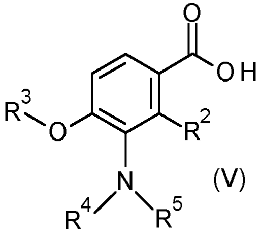 wherein R², R⁴ and R⁵ are as defined in the compound of Formula (I) in any one of claims 1 to 10 above and R³ is C₁-C₆haloalkyl.

18. A compound of Formula (VIa) 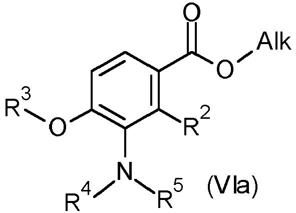 wherein "Alk" is C₁-C₆ alkyl and R², R³, R⁴ and R⁵ are as defined in the compound of Formula (I) in any one of claims 1 to 10 above.

## Patentansprüche

1. Verbindung der Formel (I): 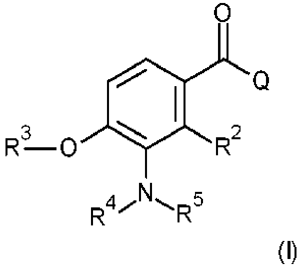
oder ein agronomisch unbedenkliches Salz davon, wobei
Q für Q¹ oder Q² steht; 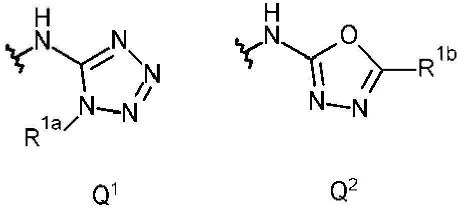
R^{1a} aus der Gruppe bestehend aus C₁-C₄-Alkyl-, C₁-C₄-Halogenalkyl-, C₁-C₄-Alkoxy-C₁-C₄-alkyl- und C₁-C₄-Halogenalkoxy-C₁-C₄-alkyl- ausgewählt ist;
R^{1b} aus der Gruppe bestehend aus C₁-C₄-Alkyl-, C₁-C₄-Halogenalkyl-, C₁-C₄-Alkoxy-C₁-C₄-alkyl- und C₁-C₄-Halogenalkoxy-C₁-C₄-alkyl- ausgewählt ist;
R² aus der Gruppe bestehend aus Halogen, C₁-C₆-Alkyl-, C₁-C₃-Alkoxy-, C₁-C₆-Halogenalkyl-, C₁-C₃-Halogenalkoxy- und -S(O)ₚ-C₁-C₆-Alkyl ausgewählt ist;
R³ für C₁-C₆-Halogenalkyl oder C₁-C₆-Alkyl steht;
R⁴ aus der Gruppe bestehend aus C₁-C₆-Alkyl-, C₁-C₆-Halogenalkyl-, C₁-C₆-Alkyl-C(O)-, C₁-C₆-Halogenalkyl-C(O)-, C₃-C₆-Cycloalkyl-, C₃-C₆-Cycloalkyl-C₁-C₃-alkyl-, C₃-C₆-Cycloalkyl-C(O)-, C₁-C₃-Alkoxy-C₁-C₃-alkyl-, C₁-C₃-Alkoxy-C₁-C₃-alkyl-C (O)-, -C(O)-Phenyl und -C(O)-Heteroaryl ausgewählt ist, wobei das Phenyl, Heteroaryl oder C₃-C₆-Cycloalkyl gegebenenfalls durch 1, 2 oder 3 Substituenten, die aus der Gruppe bestehend aus Halogen, C₁-C₆-Alkyl, C₁-C₆-Halogenalkyl und C₁-C₆-Alkoxy ausgewählt sind, substituiert ist;
R⁵ aus der Gruppe bestehend aus Wasserstoff, C₁-C₆-Alkyl-, C₁-C₆-Halogenalkyl und C₁-C₆-Cycloalkyl ausgewählt ist; oder
R⁴ und R⁵ zusammen mit dem Stickstoffatom, an das sie gebunden sind, einen 5- oder 6-gliedrigen gesättigten Heterocyclus bilden, der gegebenenfalls oxosubstituiert ist; und
p für 0, 1 oder 2 steht.

2. Verbindung nach Anspruch 1, wobei R^{1a} oder R^{1b} aus der Gruppe bestehend aus Methyl, Ethyl und n-Propyl ausgewählt ist.

3. Verbindung nach einem der vorhergehenden Ansprüche, wobei Q für Q¹ steht und R^{1a} für Methyl steht.

4. Verbindung nach Anspruch 1 oder Anspruch 2, wobei Q für Q² steht und R^{1b} für Methyl steht.

5. Verbindung nach einem der vorhergehenden Ansprüche, wobei R² aus der Gruppe bestehend aus Methyl, Cl, -CF₃ und -SO₂-Methyl ausgewählt ist.

6. Verbindung nach Anspruch 5, wobei R² für Cl steht.

7. Verbindung nach einem der vorhergehenden Ansprüche, wobei R³ für -CF₃ oder -CHF₂ steht.

8. Verbindung nach einem der vorhergehenden Ansprüche, wobei R⁴ aus der Gruppe bestehend aus C₁-C₆-Alkyl-, C₁-C₆-Alkyl-C(O)- und C₃-C₆-Cycloalkyl- ausgewählt ist.

9. Verbindung nach einem der Ansprüche 1 bis 7, wobei R⁴ für -C(O)-Heteroaryl steht, wobei das Heteroaryl gegebenenfalls durch 1, 2 oder 3 Substituenten, die aus der Gruppe bestehend aus Halogen, C₁-C₆-Alkyl, C₁-C₆-Halogenalkyl und C₁-C₆-Alkoxy ausgewählt sind, substituiert ist.

10. Verbindung nach einem der vorhergehenden Ansprüche, wobei R⁵ für Wasserstoff oder C₁-C₆-Alkyl- steht.

11. Herbizide Zusammensetzung, umfassend eine Verbindung nach einem der vorhergehenden Ansprüche und ein landwirtschaftlich unbedenkliches Formulierungshilfsmittel.

12. Herbizide Zusammensetzung nach Anspruch 11, ferner umfassend mindestens ein zusätzliches Pestizid.

13. Herbizide Zusammensetzung nach Anspruch 12, wobei es sich bei dem zusätzlichen Pestizid um ein Herbizid oder einen Herbizid-Safener handelt.

14. Verfahren zur Bekämpfung von Unkräutern an einem Standort, bei dem man eine unkrautbekämpfende Menge einer Zusammensetzung nach einem der Ansprüche 11 bis 13 auf den Standort ausbringt.

15. Verwendung einer Verbindung der Formel (I) gemäß Anspruch 1 als ein Herbizid.

16. Verbindung der Formel (II) 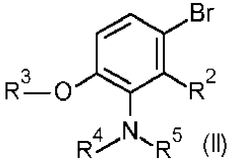 wobei R², R⁴ und R⁵ wie in der Verbindung der Formel (I) in einem der Ansprüche 1 bis 10 oben definiert ist und R³ für C₁-C₆-Halogenalkyl steht.

17. Verbindung der Formel (V) 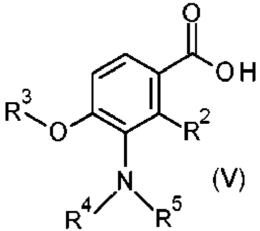 wobei R², R⁴ und R⁵ wie in der Verbindung der Formel (I) in einem der Ansprüche 1 bis 10 oben definiert ist und R³ für C₁-C₆-Halogenalkyl steht.

18. Verbindung der Formel (VIa) 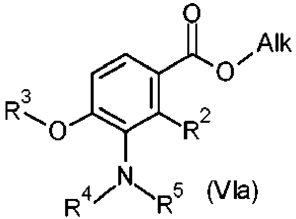 wobei "Alk" für C₁-C₆-Alkyl steht und R², R³, R⁴ und R⁵ wie in der Verbindung der Formel (I) in einem der Ansprüche 1 bis 10 oben definiert sind.

## Revendications

1. Composé de Formule (I) : 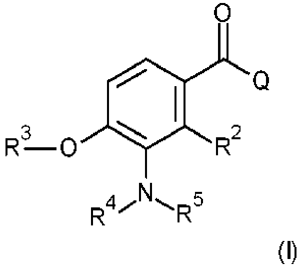
ou sel acceptable sur le plan agronomique de celui-ci,
Q étant Q¹ ou Q² ; 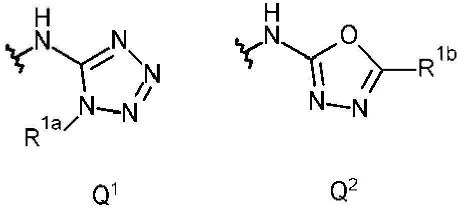
R^{1a} étant choisi dans le groupe constitué par C₁-C₄alkyle-, C₁-C₄halogénoalkyle-, C₁-C₄alcoxy-C₁-C₄alkyle- et C₁-C₄halogénoalcoxy-C₁-C₄alkyle- ;
R^{1b} étant choisi dans le groupe constitué par C₁-C₄alkyle-, C₁-C₄halogénoalkyle-, C₁-C₄alcoxy-C₁-C₄alkyle- et C₁-C₄halogénoalcoxy-C₁-C₄alkyle- ;
R² étant choisi dans le groupe constitué par halogène, C₁-C₆alkyle-, C₁-C₃alcoxy-, C₁-C₆ halogénoalkyle-, C₁-C₃halogénoalcoxy- et -S(O)ₚC₁-C₆alkyle ;
R³ étant C₁-C₆halogénoalkyle ou C₁-C₆alkyle ;
R⁴ étant choisi dans le groupe constitué par C₁-C₆alkyle-, C₁-C₆halogénoalkyle-, C₁-C₆alkyl-C(O)-, C₁-C₆halogénoalkyl-C(O)-, C₃-C₆cycloalkyle-, C₃-C₆cycloalkyl-C₁-C₃alkyle-, C₃-C₆cycloalkyl-C(O)-, C₁-C₃alcoxy-C₁-C₃alkyle-, C₁-C₃alcoxy-C₁-C₃alkyl-C(O)-, - C(O)-phényle et -C(O)-hétéroaryle dans lequel le phényle, hétéroaryle ou C₃-C₆cycloalkyle est éventuellement substitué par 1, 2 ou 3 substituants choisis dans le groupe constitué par halogène, C₁-C₆ alkyle, C₁-C₆ halogénoalkyle et C₁-C₆ alcoxy ;
R⁵ étant choisi dans le groupe constitué par hydrogène, C₁-C₆alkyle-, C₁-C₆halogénoalkyle et C₁-C₆cycloalkyle ; ou R⁴ et R⁵, conjointement avec l'atome d'azote auquel ils sont fixés, formant un hétérocycle saturé à 5 ou 6 chaînons qui est éventuellement substitué par oxo ; et
p étant 0, 1 ou 2.

2. Composé selon la revendication 1, R^{1a} ou R^{1b} étant choisi dans le groupe constitué par méthyle, éthyle et n-propyle.

3. Composé selon l'une quelconque des revendications précédentes, Q étant Q¹ et R^{1a} étant méthyle.

4. Composé selon la revendication 1 ou la revendication 2, Q étant Q² et R^{1b} étant méthyle.

5. Composé selon l'une quelconque des revendications précédentes, R² étant choisi dans le groupe constitué par méthyle, Cl, CF₃ et -SO₂méthyle.

6. Composé selon la revendication 5, R² étant Cl.

7. Composé selon l'une quelconque des revendications précédentes, R³ étant -CF₃ ou -CHF₂.

8. Composé selon l'une quelconque des revendications précédentes, R⁴ étant choisi dans le groupe constitué par C₁-C₆alkyle-, C₁-C₆alkyl-C(O)- et C₃-C₆cycloalkyle-.

9. Composé selon l'une quelconque des revendications 1 à 7, R⁴ étant -C(O)-hétéroaryle, l'hétéroaryle étant éventuellement substitué par 1, 2 ou 3 substituants choisis dans le groupe constitué par halogène, C₁-C₆alkyle, C₁-C₆halogénoalkyle et C₁-C₆alcoxy.

10. Composé selon l'une quelconque des revendications précédentes, R⁵ étant hydrogène ou C₁-C₆alkyle-.

11. Composition herbicide comprenant un composé selon l'une quelconque des revendications précédentes et un adjuvant de formulation acceptable sur le plan agricole.

12. Composition herbicide selon la revendication 11, comprenant en outre au moins un pesticide supplémentaire.

13. Composition herbicide selon la revendication 12, le pesticide supplémentaire étant un herbicide ou un phytoprotecteur herbicide.

14. Procédé de lutte contre des adventices au niveau d'un lieu comprenant une application sur le lieu d'une quantité de lutte contre des adventices d'une composition selon l'une quelconque des revendications 11 à 13.

15. Utilisation d'un composé de Formule (I) tel que défini selon la revendication 1, comme herbicide.

16. Composé de formule (II) 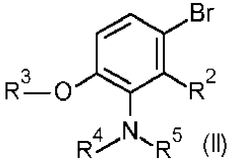 R², R⁴ et R⁵ étant tels que définis dans le composé de formule (I) dans l'une des revendications 1 à 10 ci-dessus et R³ étant C₁-C₆halogénoalkyle.

17. Composé de formule (V) 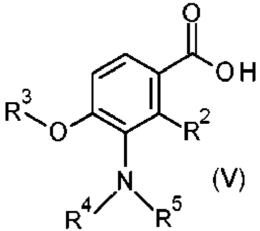 R², R⁴ et R⁵ étant tels que définis dans le composé de formule (I) dans l'une des revendications 1 à 10 ci-dessus et R³ étant C₁-C₆halogénoalkyle.

18. Composé de formule (VIa) 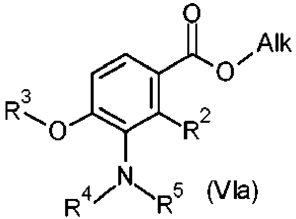 « Alk » étant C₁-C₆ alkyle et R², R³, R⁴ et R⁵ étant tels que définis dans le composé de Formule (I) dans l'une des revendications 1 à 10 ci-dessus.
